# EUROPEAN PATENT APPLICATION

(11) **EP 4 625 427 A1**
(43) Date of publication of application: **01.10.2025**
(21) Application number: 25166222.7
(22) Date of filing: 26.03.2025
(51) Int. Cl.: G16H 10/60, G06F 21/60, G06Q 10/10, G16H 40/20, G16H 40/67

(54) **DATA SHARING SYSTEM AND DATA SHARING METHOD**

(30) Priority: 29.03.2024 JP 2024056657
(71) Applicant: SYSMEX CORPORATION, Kobe-shi Hyogo 651-0073 (JP)
(72) Inventor: MURAKAMI, Kohei, Kobe-shi, Hyogo, 651-0073 (JP)
(74) Representative: Hoffmann Eitle

(57) **Abstract**

Disclosed is a data sharing system configured to share health data of a patient between (i) a plurality of types of applications configured to acquire the health data and (ii) a terminal of a medical institution, the data sharing system comprising: a data storage configured to store, in association with at least one of the applications, a linkage between a first account ID which allows the patient to access the data sharing system and a second account ID which allows a user of the terminal of the medical institution to access the data sharing system; and a controller programmed to identify, in response to a request from the terminal of the medical institution, the health data acquired by the at least one application associated with the linkage between the first account ID and the second account ID.

## Description

### CROSS REFERENCE TO RELATED APPLICATIONS

This application claims priority from prior Japanese Patent Application No. 2024-056657, filed on March 29, 2024, entitled "DATA SHARING SYSTEM AND DATA SHARING METHOD", the entire content of which is incorporated herein by reference.

### FIELD OF THE INVENTION

The present invention relates to a data sharing system and a data sharing method.

### BACKGROUND OF THE INVENTION

Systems that connect medical institutions and patients, allowing the sharing of patient information via a server, have been proposed in recent years. For example, Japanese Laid-Open Patent Publication No. 2019-191765 discloses a patient information sharing server and a patient information sharing method for sharing patient information between medical institutions and patients. The disclosed server assigns a patient identifier to each of the patients and associates the patient identifier with a medical institution that is specified for the patient. The patient uses a terminal to input some information in response to a notification given from the medical institution to the patient. The server receives the information inputted by the patient, and stores the input as patient information in association with the patient identifier.

The present invention has been made in view of the above technical background, and one object of the present invention is to provide a data sharing method that makes it possible to share, with a medical institution, various types of health information acquired from a plurality of types of applications.

### SUMMARY OF THE INVENTION

A first aspect of the present invention is directed to a data sharing system configured to share health data of a patient between (i) a plurality of types of applications configured to acquire the health data and (ii) a terminal of a medical institution, the data sharing system including: a data storage configured to store, in association with at least one of the applications, a linkage between a first account ID which allows the patient to access the data sharing system and a second account ID which allows a user of the terminal of the medical institution to access the data sharing system; and a controller programmed to identify, in response to a request from the terminal of the medical institution, the health data acquired by the at least one application associated with the linkage between the first account ID and the second account ID.

A second aspect of the present invention is directed to a method for sharing data in a data sharing system configured to share health data of a patient between (i) a plurality of types of applications configured to acquire the health data and (ii) a terminal of a medical institution, the data sharing method including: managing, in association with at least one of the applications, a linkage between a first account ID which allows the patient to access the data sharing system and a second account ID which allows a user of the terminal of the medical institution to access the data sharing system; and identifying, in response to a request from the terminal of the medical institution, the health data acquired by the at least one application associated with the linkage between the first account ID and the second account ID.

According to the present invention, health data of a patient can be shared between (i) a plurality of types of applications configured to acquire the health data of the patient and (ii) a terminal of a medical institution. In addition, a linkage between a first account ID which allows the patient to access the data sharing system and a second account ID which allows a user of the terminal of the medical institution to access the data sharing system is managed in association with at least one of the applications. Consequently, the health data related to the specific application can be identified in response to a request from the medical institution based on the managed linkage between the account IDs. In addition, through this health data identification, the health data can be provided to the medical institution.

### BRIEF DESCRIPTION OF THE DRAWINGS

FIG. 1 is a flowchart showing an example of the flow of a data sharing method according to an embodiment;
FIG. 2 shows an example of a system configuration of an information system according to a first implementation example;
FIG. 3 shows an example of a function realized by a controller of a management server according to the first implementation example;
FIG. 4 shows an example of information stored in a data storage of the management server according to the first implementation example;
FIG. 5 shows an example of PHR user account registered data according to the first implementation example;
FIG. 6 shows an example of PHR medical institution account registered data according to the first implementation example;
FIG. 7 shows an example of PHR application registered data according to the first implementation example;
FIG. 8 shows an example of account ID-PHR application linkage management data according to the first implementation example;
FIG. 9 shows an example of PHR application data management data according to the first implementation example;
FIG. 10 shows an example of a function realized by a controller of a patient terminal according to the first implementation example;
FIG. 11 shows an example of information stored in a data storage of the patient terminal according to the first implementation example;
FIG. 12 shows an example of screens displayed on a display of one of terminals according to the first implementation example;
FIG. 13 shows an example of screens displayed on the display of the terminal according to the first implementation example;
FIG. 14 shows an example of screens displayed on the display of the terminal according to the first implementation example;
FIG. 15 shows an example of screens displayed on a display of one of the terminals according to the first implementation example;
FIG. 16 shows an example of screens displayed on the display of the terminal according to the first implementation example;
FIG. 17 shows an example of a screen displayed on a display of a medical institution terminal according to the first implementation example;
FIG. 18 shows another example of the screen displayed on the display of the medical institution terminal according to the first implementation example;
FIG. 19 is a flowchart showing an example of the flow of processes to be executed by respective devices according to the first implementation example;
FIG. 20 is a flowchart showing an example of the flow of processes to be executed by respective devices according to the first implementation example;
FIG. 21 is a diagram for explaining a data sharing method according to a modification of the first implementation example;
FIG. 22 is a diagram for explaining a data sharing method according to the modification of the first implementation example;
FIG. 23 shows an example of screens displayed on the displays of the respective terminals according to another modification of the first implementation example;
FIG. 24 shows an example of screens displayed on the display of one of the terminals according to the modification of the first implementation example;
FIG. 25 is a flowchart showing an example of the flow of processes to be executed by the respective devices according to the modification of the first implementation example;
FIG. 26 shows an example of screens displayed on the display of one of the terminals according to still another modification of the first implementation example;
FIG. 27 shows an example of screens displayed on the display of the terminal according to the modification of the first implementation example;
FIG. 28 is a flowchart showing an example of the flow of processes to be executed by the respective devices according to the modification of the first implementation example;
FIG. 29 shows an example of information stored in a data storage of the medical institution terminal according to still another modification of the first implementation example;
FIG. 30 shows an example of PHR application patient identification data according to the modification of the first implementation example;
FIG. 31 shows an example of a system configuration of an information system according to a second implementation example;
FIG. 32 shows an example of functions realized by a controller of a medical institution server according to the second implementation example;
FIG. 33 shows an example of information stored in a data storage of the medical institution server according to the second implementation example;
FIG. 34 shows an example of EMR account registered data according to the second implementation example;
FIG. 35 shows an example of EMR patient information management data according to the second implementation example;
FIG. 36 shows an example of PHR-EMR association registered data according to the second implementation example;
FIG. 37 shows an example of screens displayed on the displays of the respective terminals according to the second implementation example;
FIG. 38 shows an example of screens displayed on the displays of the respective terminals according to the second implementation example;
FIG. 39 shows an example of a screen displayed on the display of the medical institution terminal according to the second implementation example;
FIG. 40 is a flowchart showing an example of the flow of processes to be executed by respective devices according to the second implementation example;
FIG. 41 is a flowchart showing an example of the flow of processes to be executed by the respective devices according to the second implementation example;
FIG. 42 shows an example of screens displayed on the displays of the respective terminals according to a modification of the second implementation example;
FIG. 43 shows an example of screens displayed on the displays of the respective terminals according to the modification of the second implementation example;
FIG. 44 is a flowchart showing an example of the flow of processes to be executed by the respective devices according to the modification of the second implementation example;
FIG. 45 shows an example of screens displayed on the display of one of the terminals according to another modification of the second implementation example;
FIG. 46 is a flowchart showing an example of the flow of processes to be executed by the respective devices according to the modification of the second implementation example;
FIG. 47 shows another example of the EMR patient information management data according to still another modification of the second implementation example;
FIG. 48 shows an example of screens displayed on the displays of the respective terminals according to a third implementation example;
FIG. 49 shows an example of screens displayed on the displays of the respective terminals according to the third implementation example;
FIG. 50 is a flowchart showing an example of the flow of processes to be executed by the respective devices according to the third implementation example;
FIG. 51 shows an example of screens displayed on the display of one of the terminals according to a fourth implementation example;
FIG. 52 shows an example of screens displayed on the display of one of the terminals according to the fourth implementation example;
FIG. 53 is a flowchart showing an example of the flow of processes to be executed by the respective devices according to the fourth implementation example;
FIG. 54 shows an example of PHR-EMR association registered data according to a fifth implementation example;
FIG. 55 is a flowchart showing an example of the flow of processes to be executed by the respective devices according to the fifth implementation example; and
FIG. 56 is a flowchart showing an example of the flow of processes to be executed by the respective devices according to the fifth implementation example.

### DETAILED DESCRIPTION

A data sharing system and a data sharing method according to the present disclosure will be described with reference to the drawings. In description based on the drawings, the same elements are denoted by the same reference characters, and repetitive explanations thereof are sometimes omitted. Constituents described in the present embodiment are merely examples and are not intended to limit the scope of the present disclosure thereto.

An information system 1 according to the present embodiment is, for example, an information system designed to share health data from various applications used by patients between multiple patients and multiple medical institutions (health professionals). The information system 1 may be referred to as, for example, a data sharing system.

Hereinafter, an application (or an application program) for, for example, collecting, recording, checking, and sharing pieces of the health data is referred to as a personal health record (PHR) application. The health data is referred to as PHR data. The PHR data may include, for example: various types of health information (e.g., a drug ingestion record, a behavior record, and the like); lifelogs such as a blood pressure, a body weight, step count, sleep pattern, and diet. Additionally, the PHR data may include data generated by medical institutions, for which a consent has been given to allow use by other organizations or for non-medical purposes. The PHR data includes data in any data format such as text, a numerical value, a movie, or a still image.

Hereinafter, a patient using the PHR application may simply be referred to as "user". Meanwhile, enabling a user (e.g., a health professional) in a medical institution to refer to (and optionally edit) the PHR data of the user by using the PHR application or the like is sometimes referred to as "PHR data linkage", "PHR application linkage", or "PHR data sharing".

A platform that integrally manages accounts and PHR data in various types of PHR applications may be referred to as a PHR platform.

Users who are allowed to use the PHR platform are not limited to the patients and the medical institutions. For example, the patients and personnels handling the PHR data of the patients (e.g., caregivers, teachers for a special needs class, or trainers in a training gym) may be allowed to use the PHR platform.

Hereinafter, for example, an application intended primarily for use within a medical institution by its users, such as an electronic medical record application, is referred to as an EMR (Electronic Medical Record) application. In the present specification, the EMR application includes, in addition to diagnosis-related information included in the electronic medical record, test information (a blood collection result, a test image, or the like), background/basic information about the patient (a past medical history, a blood pressure, a body weight, or the like), and information about medical practitioners' receipts for health insurance claims (medical practice, a drug, or the like). The EMR application may encompass a concept of an electric health record (EHR) application intended for an information linkage with an external medical institution.

Hereinafter, an application for managing data mainly acquired in a medical institution may be referred to as EMR application, and an application for managing data mainly acquired by a patient may be referred to as a PHR application, in order to distinguish these applications.

In the present disclosure, the information system 1 may be configured to have a plurality of devices, for example. The information system 1 may be configured such that, for example, the plurality of devices perform a certain process in cooperation.

The information system 1 for a client (client device) and a server may be, as an example, a system including at least one terminal and at least one server. The system in this example may be considered as a client server system.

The server may be a single device or a combination (server system) of a plurality of devices. A function of the server may be provided in the form of PaaS, IaaS, or SaaS in cloud computing.

The information system 1 may be a system composed of a plurality of terminals. This system may be configured as follows for example:
· a system (distributed system) in which the terminals have functions of the server, the system being able to be implemented by, for example, utilizing a block chain technology; or
· a system in which the terminals perform wireless communication with one another, the system being able to be implemented by, for example, performing communication through a peer-to-peer (P2P) method or the like by utilizing a short-range wireless communication technology according to Bluetooth (registered trademark) or the like.

PHR data collected from various PHR applications enable the medical institutions and the patients to ascertain comprehensive medical information. However, management of the PHR applications and PHR data is complicated for both the medical institutions and the patients. For example, in diagnosis and therapy of ASD (Autism Spectrum Disorder), a patient inputs several kinds of PHR data such as a behavior record, a drug ingestion record, a body weight, and a body height through a patient terminal by using applications each specialized in recording specific kind of PHR data. The medical institution utilizes the various kinds of PHR data such as the behavior record, the drug ingestion record, the body weight, and the body height of the patient as reference information for diagnosis and determining therapeutic strategy for ASD. With such a comprehensive information, the medical institution can make an appropriate diagnosis and also can select an appropriate therapy method. However, without a centralized means to manage and share these PHR applications and various kinds of PHR data, it becomes difficult to collectively analyze the PHR data or compare one kind of PHR data with another, whereby the efficiency and the accuracy of diagnoses and therapies might decrease. Therefore, an efficient data sharing method for a plurality of such applications and pieces of such PHR data has been desired.

Each of the pieces of PHR data may not only be manually inputted by a patient but may also be automatically recorded or provided from another person, to be registered to the corresponding PHR application. For example, a patient terminal may constantly measure the number of steps taken while the patient is walking, and may automatically record the number in the corresponding PHR application. Also, the patient terminal may receive prescription information for the patient from a doctor or a pharmacist and may automatically record the prescription information in the corresponding PHR application.

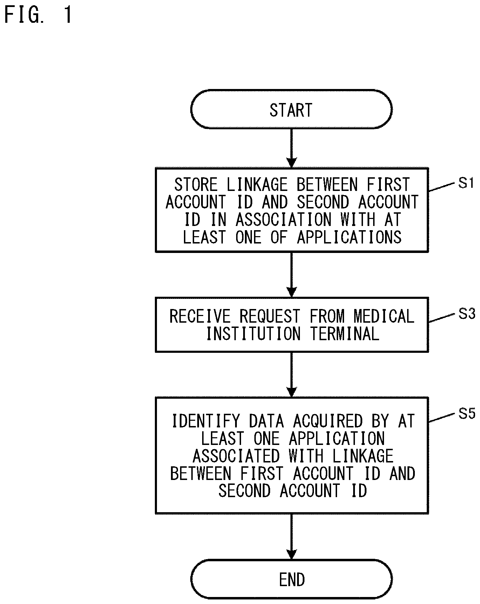

Firstly, a data sharing method according to the present embodiment will be described with reference to FIG. 1. In the present embodiment, health data of a patient is shared between (i) a plurality of types of applications which acquire the health data of the patient and (ii) a medical institution terminal. The data sharing method may be a method to be performed by the data sharing system (hereinafter, sometimes referred to as "sharing system").

A linkage between a first account ID which allows the patient to access the sharing system and a second account ID which allows a user of the medical institution terminal to access the sharing system is stored in association with at least one of the applications (S1).

A request from the medical institution terminal is received (S3). For example, a request (e.g., a data provision request) for health data associated with the linkage between the first account ID and the second account ID is received.

The health data acquired by the at least one application associated with the linkage between the first account ID and the second account ID is identified in response to the above request from the medical institution terminal (S5). Through this health data identification, the health data can be provided to the medical institution terminal. As described later in detail, the sharing system may play a main role in providing the health data, or an entity other than the sharing system may play a main role in providing the health data.

Between steps S1 and S3, step S2 (e.g., a step of registering the health data of the patient to the sharing system) may be performed as a step of registering the health data of the patient in association with the first account ID and the application having acquired this health data. In this case, either of steps S1 and S2 may be performed first, or steps S1 and S2 may be simultaneously performed.

The sharing system may be a system composed of, for example, one or more management devices (e.g., one or more server devices). The sharing system may be considered as a system that performs management regarding sharing of the health data of the patient.

Hereinafter, implementation examples for implementing the above data sharing method will be specifically described.

### <First Implementation Example>

A first implementation example is an implementation example of PHR platform that allows a medical institution terminal of a medical institution to access various kinds of PHR data. The various kinds of PHR data is recorded by a plurality of types of PHR applications running on a terminal that the patient can operate. The features described in the first implementation example are applicable to the other implementation examples and modifications in the same manner.

### <System Configuration>

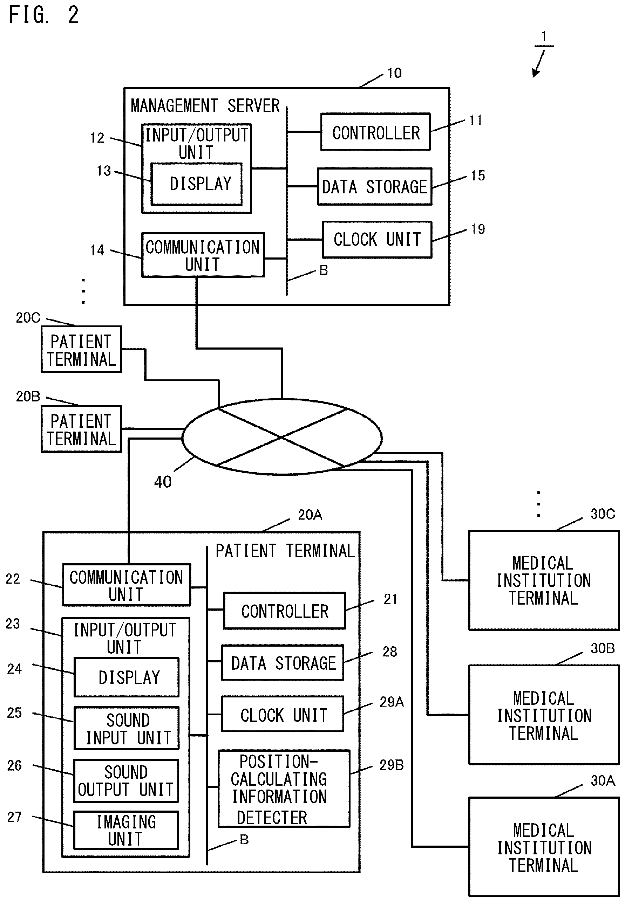

FIG. 2 shows an example of a system configuration of an information system 1 according to the present implementation example. In the information system 1, a management server 10 of the PHR platform, one or more patient terminals 20 (20A, 20B, 20C, ··· ), and one or more medical institution terminals 30 (30A, 30B, 30C, ··· ) are connected to one another via a network 40, for example.

In the following description, a user of the patient terminal 20A in communication with the management server 10 will be referred to as "user U.A". A user of the patient terminal 20B in communication with the management server 10 will be referred to as "user U.B". A user of the patient terminal 20C in communication with the management server 10 will be referred to as "user U.C". In addition, in the following description, a user of the medical institution terminal 30A in communication with the management server 10 will be referred to as "medical institution H.A". A user of the medical institution terminal 30B in communication with the management server 10 will be referred to as "medical institution H.B". A user of the medical institution terminal 30C in communication with the management server 10 will be referred to as "medical institution H.C".

The management server 10 is, for example, an information processing device or a computer of a business entity running the PHR platform. The management server 10 has a function of, for example, collecting and recording PHR data acquired by the multiple PHR applications from the patient terminals 20 and the like via the network 40. The management server 10 also has a function of linking or sharing the pieces of PHR data via the network 40. The management server 10 may be considered as an example of the sharing system. The management server 10 may be a single server device or a plurality of server devices as described above. The business entity running the PHR platform may be a developer that has developed the PHR application or a provider providing services of PHR application. The business entity may alternatively be an independent business entity cooperating with the developer or provider of the PHR application.

Each of the patient terminals 20 may be, for example, a smartphone, a tablet terminal, a personal computer, a smartwatch, or the like. The configurations of the patient terminal 20A, the patient terminal 20B, and the patient terminal 20C may be, for example, identical to one another. Hereinafter, each of the terminals that is used by a user X may be expressed as a patient terminal 20X, as necessary.

The same may apply to the medical institution terminals 30.

The network 40 serves to provide a connection path such that: these devices composing the information system 1 are connected to one another; and data can be transmitted and received therebetween. The network 40 may be, for example, a wide area network such as the Internet implemented by a wired network or a wireless network.

### [Hardware Configuration of Each Device]

### (1) Hardware configuration of each terminal

FIG. 2 shows an example of a hardware configuration of each of the patient terminals 20. The patient terminal 20 includes, for example: a controller 21 including a CPU and the like; a data storage 28 including a RAM, a ROM, and the like; a communication unit 22 having a wired or wireless communication function; an input/output unit 23 including a device for inputting various operations to the patient terminal 20 and a device for outputting a processing result acquired through processing on the patient terminal 20; a clock unit 29A configured to have a clock in which a quartz crystal oscillator is used, a clock for which the network identity and time zone (NITZ) standard is applied; and a position-calculating information detector 29B including a sensor or a unit for calculating the position of the patient terminal 20 by utilizing a satellite positioning system such as the global navigation satellite system (GNSS). These constituents of the hardware of the patient terminal 20 are connected to one another via, for example, a bus B. The patient terminal 20 may have a configuration in which an individual constituent or a plurality of constituents are detachable.

The input/output unit 23 includes, for example: a display 24 implemented by a liquid crystal display or an OELD; a sound input unit 25 implemented by a microphone; a sound output unit 26 implemented by a speaker; and an imaging unit 27 implemented by a camera or the like.

The same may apply to each of the medical institution terminals 30.

### (2) Hardware configuration of server

FIG. 2 shows an example of a hardware configuration of the management server 10. The management server 10 includes, for example: a controller 11 including a CPU and the like; a data storage 15 including a RAM, a ROM, a magnetic disk, and a semiconductor drive; a communication unit 14 having a wired or wireless communication function; an input/output unit 12 implemented by a keyboard and the like; a display 13 such as a liquid crystal display; and a clock unit 19 configured to have a clock in which a quartz crystal oscillator is used, an NITZ clock, or the like. These components of the hardware of the management server 10 are connected to one another via, for example, a bus B. The hardware of the management server 10 may have a configuration in which an individual constituent or a plurality of constituents are detachable.

### (3) Others

A process in the management server 10, the patient terminal 20, and/or the medical institution terminal 30 may be at least partially performed through cloud computing implemented by one or more computers. A configuration may be employed in which the process in the patient terminal 20 and/or the medical institution terminal 30 is at least partially or entirely performed by the management server 10. In contrast, a configuration may be employed in which the process in the management server 10 is at least partially or entirely performed by the patient terminal 20 and/or the medical institution terminal 30. The configuration for determination in the embodiment of the present disclosure is not indispensable unless otherwise noted expressly, and a predetermined process may be performed when a determination condition is satisfied, or a predetermined process may be performed when a determination condition is not satisfied.

### [Functional Configuration of Each Device]

### (1) Functional configuration of management server

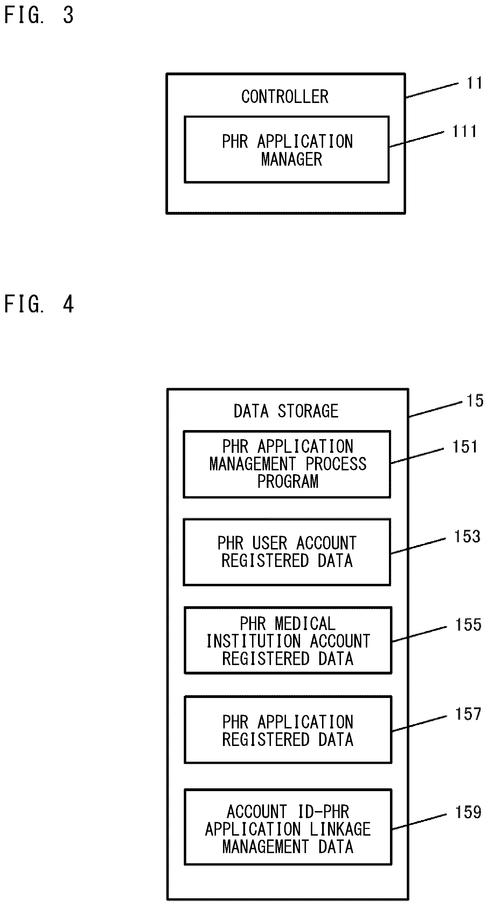

FIG. 3 shows a functional block diagram of a function realized by the controller 11 of the management server 10 according to the present implementation example. The controller 11 includes, as a functional unit, a PHR application manager 111 which executes a PHR application management process (which may be rephrased as a PHR platform management process) according to a PHR application management process program 151 stored in the data storage 15, for example.

FIG. 4 shows an example of information stored in the data storage 15 of the management server 10 according to the present implementation example. The data storage 15 stores therein, for example, the PHR application management process program 151, PHR user account registered data 153, PHR medical institution account registered data 155, PHR application registered data 157, and account ID-PHR application linkage management data 159.

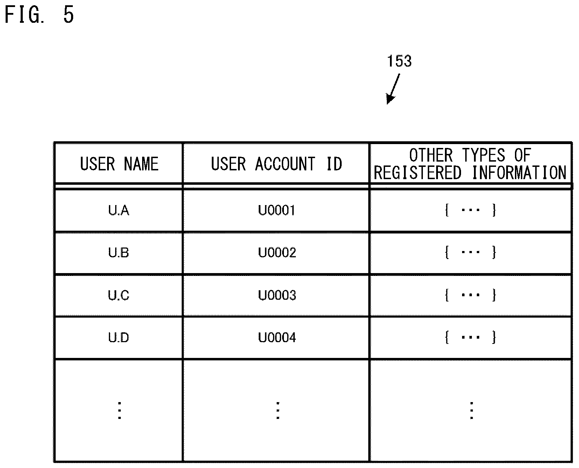

The PHR user account registered data 153 is data related to accounts of patients registered in the PHR platform. An example of the data configuration is shown in FIG. 5. In the PHR user account registered data 153, user names, user account IDs, and other types of registered information are stored in association with one another, for example.

Each of the user names is account name of the corresponding patient terminal 20 utilizing the PHR platform. For example, a name registered by the user of the patient terminal 20 to utilize the PHR platform is stored.

Each of the user account IDs is information to be used for identifying the account of the corresponding patient in the PHR platform. Each of the user account IDs is preferably a numerical value that is unique to the corresponding account, and, for example, a unique value (distinct value) is set and stored for each of the accounts by the management server 10. The user account ID is information associated with the patient terminal 20 or the user of the patient terminal 20. The user account ID may be regarded as an example of information about the terminal or information about the user of the terminal. Also, the user account ID may be defined as a first account ID. The first account ID may be considered as an example of an ID which allows the patient (or the patient terminal 20) to access the sharing system. In a case where the user account ID and identification information for identifying the patient terminal 20 are uniquely associated with each other, the user account ID may be considered as identification information for the patient terminal 20.

In the other types of registered information, various types of information may be stored, for example. The various types of information include: pieces of user information (addresses, birthdays, sexes, and the like); pieces of identification information for identifying the respective patient terminals 20 (international mobile equipment identities (IMEIs) and the like); telephone numbers (terminal telephone numbers) of the respective patient terminals 20; e-mail addresses (terminal e-mail addresses) of the respective patient terminals 20; and pieces of authentication information such as passwords (login passwords, authentication passwords, and the like) to be used for various authentications in the PHR platform or an application.

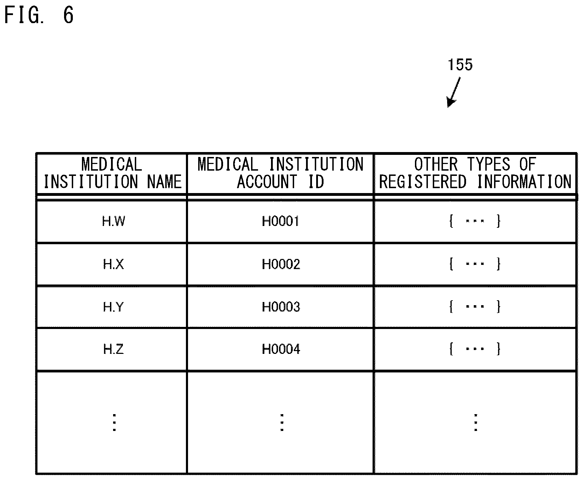

The PHR medical institution account registered data 155 is registered data related to accounts of medical institutions participating in the PHR platform. An example of a data configuration of the data 155 is shown in FIG. 6. In the PHR medical institution account registered data 155, medical institution names, medical institution account IDs, and other types of registered information are stored in association with one another, for example.

Each of the medical institution names is the name of an account of the corresponding medical institution terminal 30 utilizing the PHR platform. For example, a name registered by the medical institution that uses the medical institution terminal 30 to utilize the PHR platform is stored.

Each of the medical institution account IDs is information to be used for identifying the account of the corresponding medical institution in the PHR platform. Each of the medical institution account IDs is preferably a value that is unique to the corresponding account, and, for example, a unique value (distinct value) is set and stored for each of the accounts by the management server 10. The medical institution account ID is information associated with the medical institution terminal 30 or the medical institution corresponding to the medical institution terminal 30. The medical institution account ID may be considered as an example of information about the terminal or information about the medical institution. The medical institution account ID may be defined as a second account ID. The second account ID may be considered as an example of an ID which allows a user of the medical institution terminal 30 (or the medical institution terminal 30) to access the sharing system.

In the other types of registered information, various types of information may be stored, for example. The various types of information include: pieces of medical institution information (addresses, representative contact information, positional information, medical specialties, and the like); pieces of identification information (international mobile equipment identities (IMEIs) and the like) for identifying the respective medical institution terminals 30; telephone numbers (terminal telephone numbers) of the respective medical institution terminals 30; e-mail addresses (terminal e-mail addresses) of the respective medical institution terminals 30; and pieces of authentication information such as passwords (login passwords, authentication passwords, and the like) to be used for various authentications in the PHR platform or an application.

In a same medical institution, a plurality of the medical institution account IDs may be allowed to be registered for respective medical specialties or health professionals.

In addition, the medical institution account ID may be allowed to be acquired by personnel who handles PHR data in the PHR platform. Such personnel may be a caregiver, a teacher for a special needs class, a trainer in a training gym, or the like, for example.

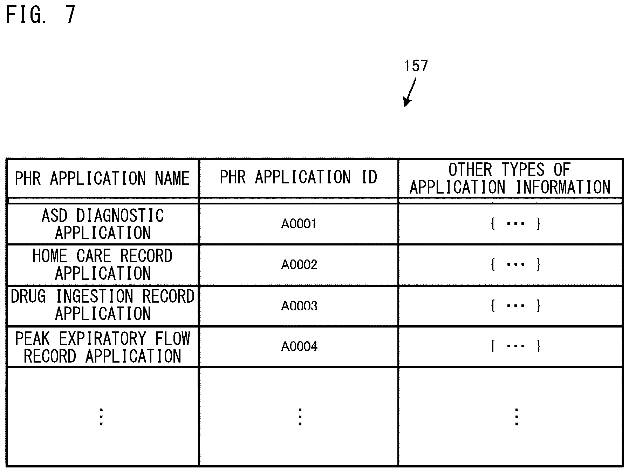

The PHR application registered data 157 is registered data related to applications linked with the PHR platform. An example of a data configuration of the data 157 is shown in FIG. 7. In the PHR application registered data 157, PHR application names, PHR application IDs, and other types of application information are stored in association with one another, for example.

Each of the PHR application names is the name of the corresponding PHR application. For example, a name set registered by the developer of the PHR application onto the PHR platform is stored.

Each of the PHR application IDs is information to be used for identifying the corresponding PHR application. Each of the PHR application IDs is preferably a value that is unique to the corresponding PHR application. For example, a unique value (distinct value) is set and stored for each of the PHR applications by the management server 10.

As the other types of application information, uniform resource identifiers (URIs) for downloading the respective PHR applications, descriptions of the respective PHR applications, and the like may be stored, for example.

The PHR applications that can be run on the PHR platform may be various types of applications. Examples of the PHR applications are: (i) an autism spectrum disorder (ASD) diagnostic application programmed to record an image and/or a movie of a patient and/or a related person of the patient and to refer to the recorded image and/or movie; (ii) a home care record application programmed to create records of the homecare given by a home caregiver to the patient; (iii) a drug ingestion record application programmed to record intake records of prescription drugs; and (iv) a peak expiratory flow record application programmed to record a peak expiratory flow related to asthma. The PHR applications may further include: (v) an application programmed to monitor records related to a predetermined health condition such as hay fever; (vi) an application used to handle information acquired through communication with a device monitoring a health condition by the patient terminal 20 such as a blood sugar level monitoring device or a smartwatch.

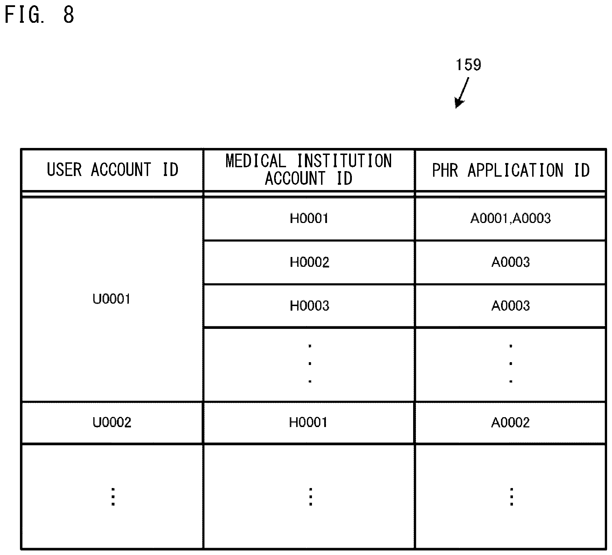

The account ID-PHR application linkage management data 159 is management data for a linkage among each of the user account IDs, the corresponding medical institution account ID, and the corresponding PHR application ID, and an example of a data configuration thereof is shown in FIG. 8. In the account ID-PHR application linkage management data 159, the aforementioned user account IDs, the aforementioned medical institution account IDs, and the aforementioned PHR application IDs are stored in association with one another, for example.

The account ID-PHR application linkage management data 159 may be considered as an example of data for managing, in association with at least one of the applications, a linkage between a first account ID which allows the patient to access the sharing system and a second account ID which allows a user of the medical institution terminal to access the sharing system.

As a method for providing PHR data requested from any of the medical institutions (medical institution terminals 30), various types of methods are conceivable.

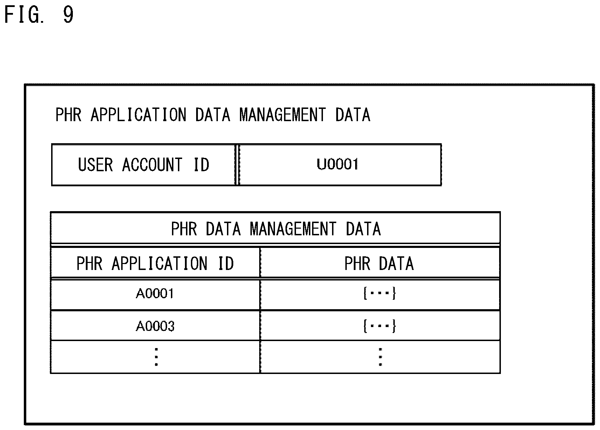

The following case will be presented as an example of first implementation example. That is, PHR data is registered to the management server 10. The management server 10 provides, based on a request from any of the medical institution terminals 30, the PHR data stored in the data storage 15 to the medical institution terminal 30. In this case, pieces of PHR application data management data shown in FIG. 9 may be stored in the data storage 15 of the management server 10 as pieces of management data for the respective user account IDs, for example. In FIG. 9, a piece of PHR application data management data with a user account ID of "U0001" is shown as an example.

In each of the pieces of PHR application data management data, a user account ID and PHR data management data may be stored, for example. The PHR application data management data may be considered as an example of data for managing an account corresponding to the patient based on the user account ID shared by the plurality of types of PHR applications. The PHR data management data may be data in which a plurality of pieces of PHR data corresponding to respective PHR applications can be registered, and, for example, each of the PHR application IDs and the corresponding piece of PHR data may be stored in association with each other.

### (2) Function configuration of patient terminal

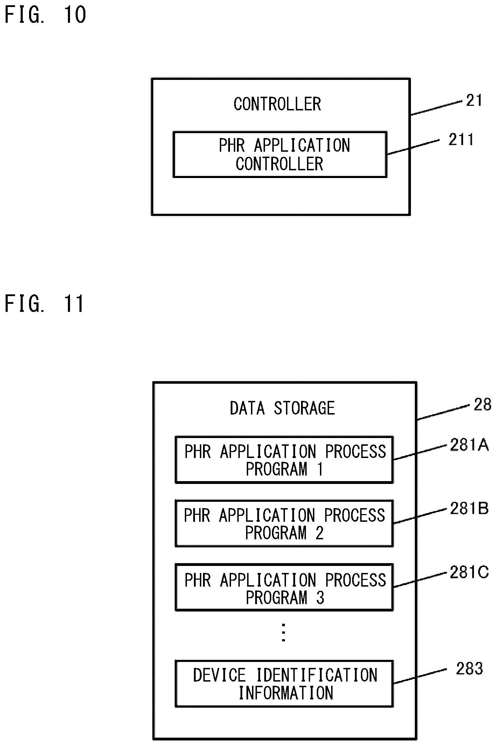

FIG. 10 shows an example of a function realized by the controller 21 of each of the patient terminals 20 according to the present implementation example. The controller 21 includes, as a functional unit, a PHR application controller 211 which executes a PHR application process according to a PHR application process program 281 stored in the data storage 28, for example.

FIG. 11 shows an example of data stored in the data storage 28 of the patient terminal 20, and the like according to the present implementation example. The data storage 28 stores therein, for example, various types of PHR application process programs 281A, 281B, 281C, ··· to be executed as PHR application processes and device identification information 283 (e.g., a telephone number, an IMEI, or the like) for identifying this patient terminal 20 or the account of the user of the patient terminal 20. In each of the PHR application process programs 281, a PHR application ID assigned thereto may be stored. In addition, in the device identification information 283, the user account ID of this patient terminal 20 may be stored.

### (3) Function configuration of medical institution terminal

A function configuration of each of the medical institution terminals 30 may be, for example, the same as that of the patient terminal 20. In each of PHR application process programs 381, a PHR application ID assigned thereto may be stored. In addition, in device identification information 383, the medical institution account ID of this medical institution terminal 30 may be stored.

### < Displayed Screen>

Hereinafter, examples of displayed screens will be described. Transition between the displayed screens described below is merely an example of transition between displayed screens for implementing the technique of the present disclosure. Regarding the transition between the displayed screens presented below as an example, some of the displayed screens do not have to be displayed, or another displayed screen may be added.

Hereinafter, a case where, for example, a patient terminal 20 and a medical institution terminal 30 are smartphones respectively including the display 24 and a display 34 as vertically long displays will be presented as an example. For some of the displayed screens, a case where the medical institution terminal 30 is a PC including a horizontally long display 34 will be sometimes presented as an example.

The flow of transition between screens will be sometimes described by showing, within one of the drawings or over a plurality of the drawings, predetermined different screens displayed on the patient terminal 20 and/or the medical institution terminal 30.

Hereinafter, a case where the patient terminal 20A of the user U.A is used as an example of the patient terminal 20 that records PHR data in the PHR platform will be presented as an example. An account of the patient terminal 20A or an account of the user U.A may be considered as an example of a first account.

Also, hereinafter, a case where the medical institution terminal 30A in the medical institution H.A is used as an example of the medical institution terminal 30 that refers to PHR data based on a linkage from the user in the PHR platform will be presented as an example. An account of the medical institution terminal 30A or an account of the medical institution H.A may be considered as an example of a second account.

In this example, ASD diagnostic applications are presented as exemplary PHR applications, and the name written as "PHR for ASD User App" is presented as an example of the name of a PHR application that is used by a patient. In addition, the name written as "PHR for ASD Institute App" is presented as an example of the name of a PHR application that is used in the medical institution.

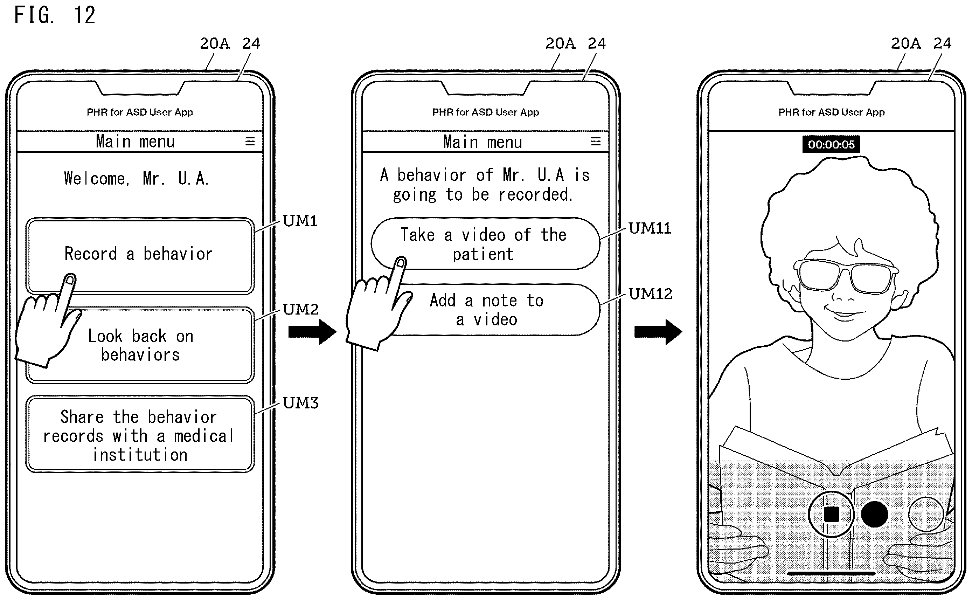

FIG. 12 to FIG. 16 show examples of screens displayed on the display 24 of the patient terminal 20A and the display 34 of the medical institution terminal 30A according to the present implementation example.

On the left side of FIG. 12, an example of a main menu screen of the ASD diagnostic application displayed on the patient terminal 20A is shown. This main menu screen is configured such that a function button UM1 for recording a behavior of the patient, a function button UM2 for referring to recorded behaviors of the patient, and a function button UM3 for sharing the recorded behavior records (an example of the PHR data) with a medical institution are displayed as a selection menu intended for the patient beneath the name ("U.A") of the user using the patient terminal 20A.

When the user taps the function button UM1 in the screen on the left side of FIG. 12, the screen displayed on the patient terminal 20A is changed to a behavior recording screen at the center of FIG. 12. The user can record the behavior of the patient in a video by using the camera of the patient terminal 20A. In the behavior recording screen in this example, a message reporting that the behavior of "U.A" is to be recorded (behavior recording is to be started) is displayed, and a "Take a video of the patient" button UM11 for recording a video (moving image) of the patient by the imaging unit 27 and an "Add a note to a video" button UM12 for adding a note to a recorded video are displayed.

In response to tapping of the "Take a video of the patient" button, the imaging unit 27 (which may be an app camera) is activated, and, on the right side of FIG. 12, a state where a moving image of the user "U.A" is being taken by the inward-facing camera (frontward-facing camera) as a result of the activation is shown.

Meanwhile, in response to tapping of the "Add a note to a video" button, a list of taken videos may be displayed, and the user may be able to, after selecting any of the videos, input a note by using a keyboard or the like, for example. Then, data of the inputted note may be stored in association with data of the video.

After the behavior of the patient is recorded in a video, the displayed screen is returned to the main menu screen. The user taps the function button UM2, then the screen displayed on the patient terminal 20A is changed to a screen to display PHR data, whereby the user can look back on recorded videos, for example.

As described above, the PHR data may include data in any data format such as text, a numerical value, a moving image (video), or a still image. Thus, for example, a "Manually input a behavior" button for allowing the user to input his/her own behavior by means of text or the like may be displayed in the screen at the center of FIG. 12, and information (e.g., text information) about the behavior inputted by using a keyboard or the like may be included in the PHR data and recorded.

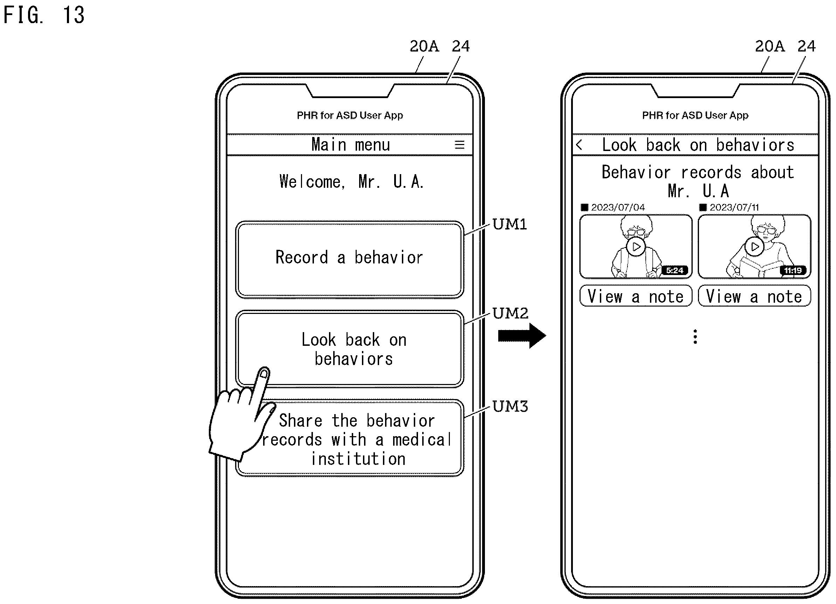

On the left side of FIG. 13, the same main menu screen as that on the left side of FIG. 12 is shown. In response to the user tapping the function button UM2, the screen displayed on the patient terminal 20A is changed to a behavior review screen on the right side of FIG. 13. The behavior review screen is configured such that, for example, PHR data (in this example, the dates on which videos have been recorded, the videos, and notes added to the videos) based on the above behavior records are displayed as behavior records about the user "U.A".

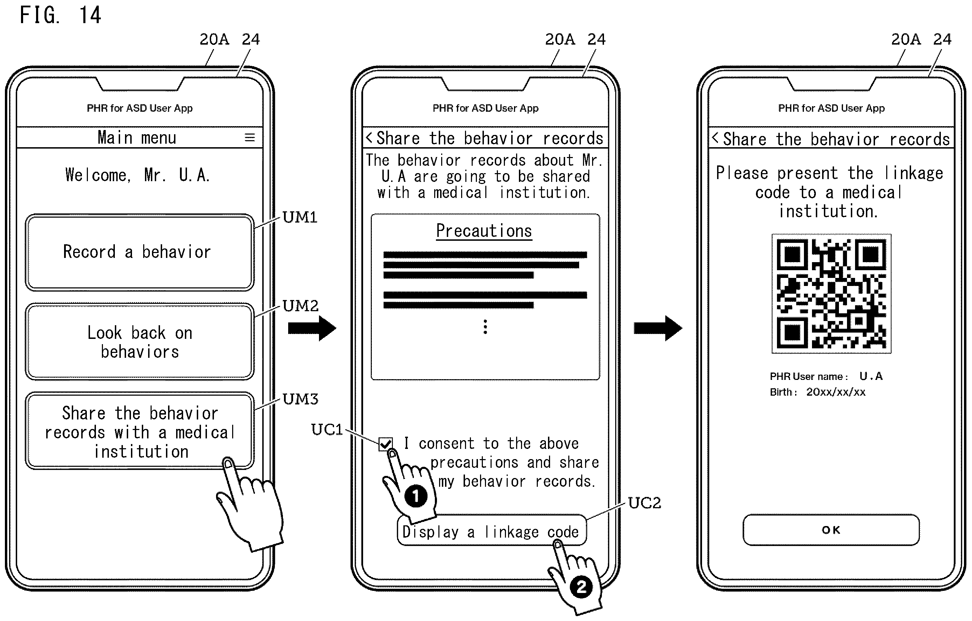

On the left side of FIG. 14, the same main menu screen as that on the left side of FIG. 12 is shown. In response to the user tapping the function button UM3, the screen displayed on the patient terminal 20A is changed to a PHR data sharing confirmation screen at the center of FIG. 14. This screen is configured to display precautions on sharing of the PHR data (which may be rephrased as provision or linkage of PHR data) with a medical institution. In addition, this screen is configured such that a checkbox UC1 for consenting to sharing of the PHR data based on the precautions and a function button UC2 for displaying PHR application linkage code information to be read by the medical institution terminal 30 are displayed beneath the precautions.

For example, tapping the checkbox UC1 by the user changes the display mode of the function button UC2 from a grayed-out display mode to an ordinary display mode so that operation is enabled. Then, the user taps the function button UC2, whereby the screen displayed on the patient terminal 20A is changed to a screen to display PHR application linkage code information on the right side of FIG. 14. The screen is configured such that, for example, PHR application linkage code information expressed by a two-dimensional code, the user name of the patient terminal 20A in the PHR application, and a birth date as an example of user information are displayed. Also, information such as the sex of the user and the name of the PHR application to be linked may be displayed. Consequently, the user of the medical institution terminal 30 can prevent errors such as misidentifying patient whose PHR data is linked.

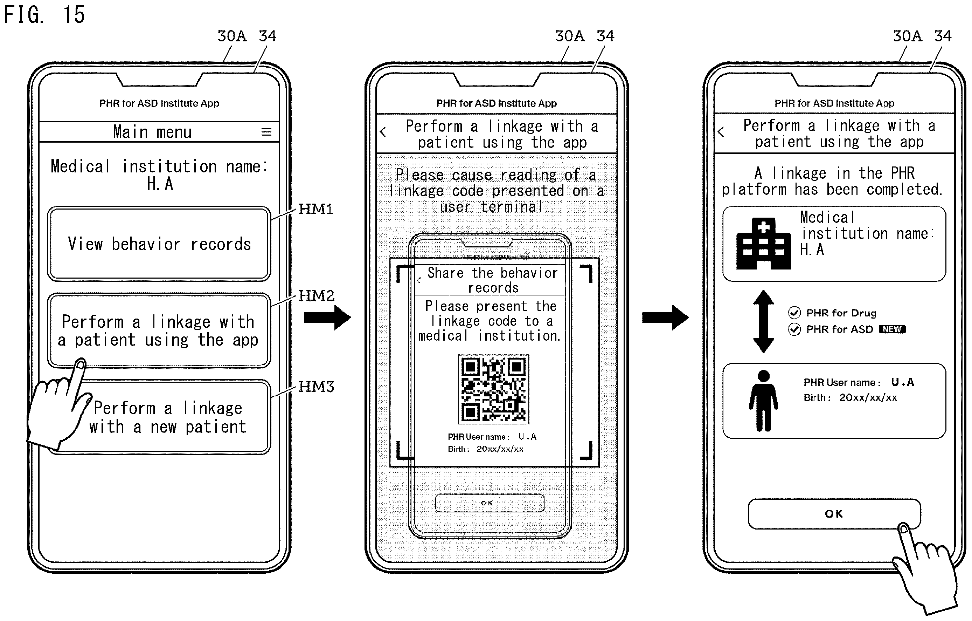

On the left side of FIG. 15, a main menu screen of the ASD diagnostic application displayed on the medical institution terminal 30A is shown. This main menu screen is configured to display, as a selection menu intended for the medical institution: (i) a function button HM1 to allow viewing of behaviors of a patient whose PHR data is shared through PHR application linkage, (ii) a function button HM2 to perform a PHR application linkage with a patient who is already using the ASD diagnostic application (who has created an account for the PHR platform), and (iii) a function button HM3 to instruct a patient who is not using the ASD diagnostic application to install the application such that PHR application linkage can be performed (referred to as "app prescription"), beneath the name ("H.A") of the medical institution.

For example, by the user tapping the function button HM2, the screen displayed on the medical institution terminal 30A is changed to a PHR application linkage code information reading screen at the center of FIG. 15. The PHR application linkage code information reading screen is configured such that, for example, a preview screen for an image to be acquired by an imaging unit 37 and guidance information for prompting placement, within the preview screen, of the PHR application linkage code information displayed on the patient terminal 20 are displayed.

For example, by the imaging unit 37 reading the PHR application linkage code information and the management server 10 executes a PHR application account linkage process described later, the screen displayed on the medical institution terminal 30A is changed to a screen to display PHR linkage notification information on the right side of FIG. 15. This screen is configured to display, as PHR linkage notification information, (i) information about the medical institution which participates the PHR data sharing (e.g., name of the medical institution); and (ii) information about the patient (e.g., a user name, a birth date, and the like) whose PHR data is shared, in a comparable manner. In addition, this screen is configured to display, between the information about the medical institution and the information about the patient, the names of PHR applications whose PHR data is shared. In addition, an icon indicating that the ASD diagnostic application has been newly linked is displayed in this screen.

The PHR linkage notification information may be displayed on the patient terminal 20A when the PHR application account linkage process is executed.

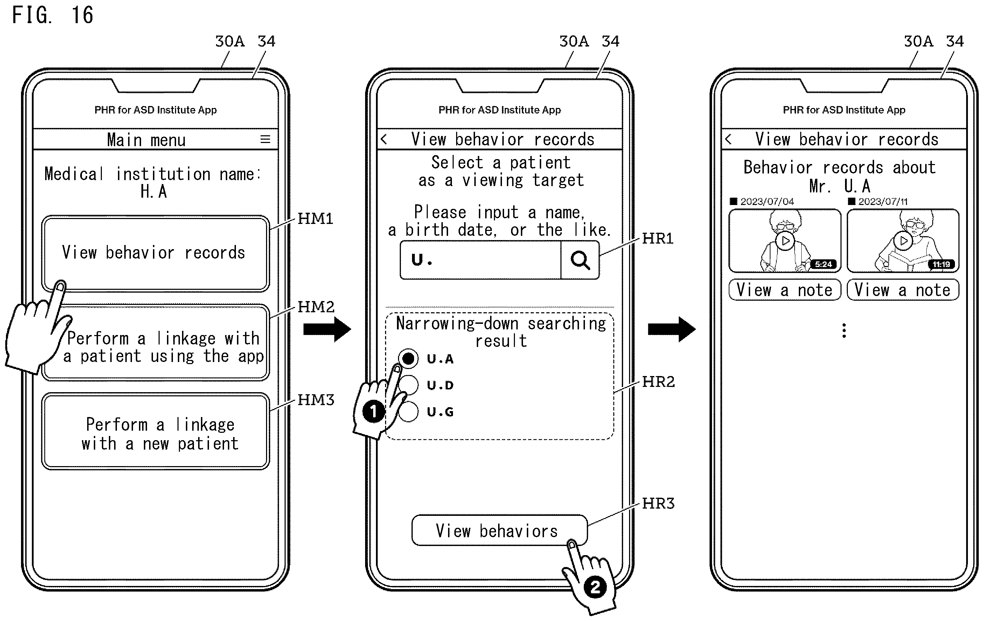

For example, in response to the function button HM1 being tapped in the main menu screen on the left side of FIG. 16 on the medical institution terminal 30A after a linkage regarding the ASD diagnostic application with the user U.A is performed, the screen displayed on the medical institution terminal 30A is changed to a PHR data reference request screen at the center of FIG. 16. This screen is configured to display a user information input region HR1 for narrowing down users whose PHR data is to be viewed. In addition, this screen is configured to display, beneath the user information input region HR1, a PHR data reference user selection region HR2 for displaying a list of users obtained by the narrowing-down through the input to the region HR1.

For example, when U.A is selected from among the users displayed in the PHR data reference user selection region HR2, and a PHR data reference button HR3 is tapped, the screen displayed on the medical institution terminal 30A is changed to a screen to display PHR data as illustrated on the right side of FIG. 16. The screen displays video data and note data as the PHR data beneath the name of the patient selected. The video data and the note data are the same as the aforementioned pieces of data on the right side of FIG. 13, while the displayed data does not necessarily have to be the same as that on the right side of FIG. 13. The displayed data may differ between the patient terminal 20 and the medical institution terminal 30.

The same screen to display PHR data as that on the right side of FIG. 16 may be displayed when the function button UM2 is tapped in the main menu screen of the ASD diagnostic application on the left side of FIG. 14.

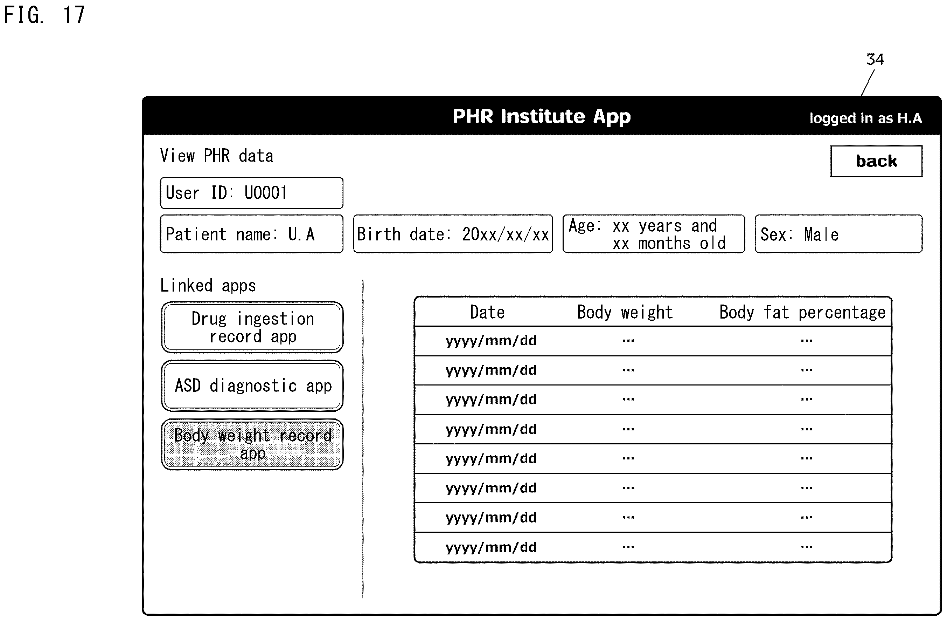

FIG. 17 shows another example of the screen to display PHR data displayed on the display 34 of the medical institution terminal 30. The screen in this example is displayed in a PHR integration application ("PHR Institute App") that makes it possible to perform a PHR data linkage in a user-selected PHR application or collectively refer to pieces of PHR data shared in the PHR platform. This screen is configured to display information about a user (e.g., a user account ID, a user name, and the like) of the PHR data to view at an upper part of the display 34, for example. In addition, this screen is configured to display, beneath the information about the user, information about a list of linked applications in a left pane. In addition, this screen is configured to display, in a right pane, the PHR data of the selected application among the list of the linked applications. In the illustrated screen, a body weight record app is selected as an example of the selected application and a body weight and a body fat percentage on each date is displayed as PHR data. When a drug ingestion record app is selected from the left pane, the date and the time of drug ingestion, the type and the amount of the drug, and the like may be displayed as PHR data, for example. When an ASD diagnosis app is selected, dates on which recording has been performed and content of behavior records may be displayed as PHR data, for example.

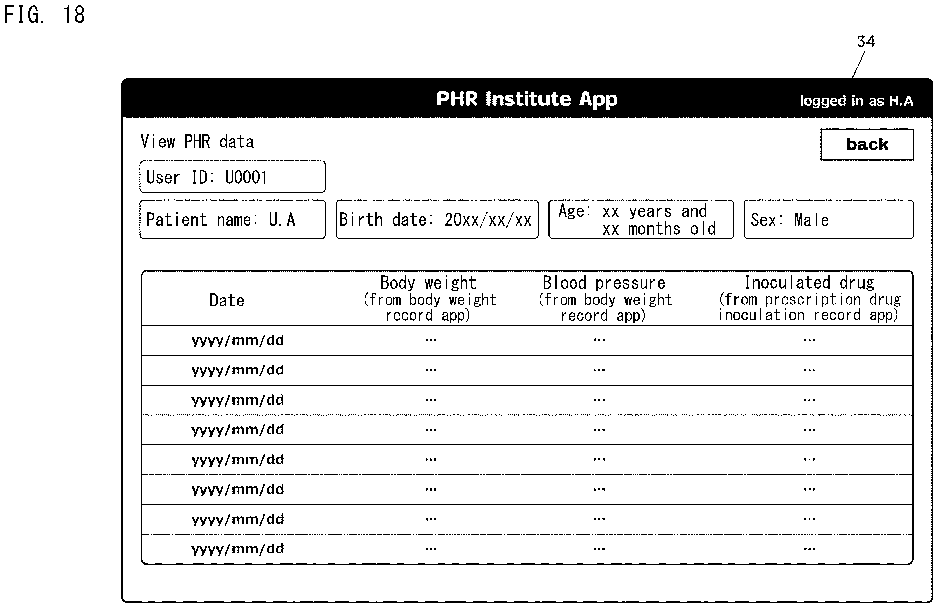

Alternatively, in the screen of the PHR integration application, all kinds of PHR data in the linked applications may be listed without need of selecting any PHR application, as shown in FIG. 18. Consequently, the user of the PHR integration application can directly check the various kinds of PHR data without selecting any PHR application.

The PHR integration application may be provided as, for example, software as a service (SaaS). For example, the management server 10 may be configured to enable usage of the PHR integration application as a web application. Also, the PHR integration application may be set to be usable on the patient terminal 20, for example.

The PHR integration application may be configured such that: a user account ID can be identified by inputting information (e.g., a user name, a birth date, and the like) about a patient in the same manner as in FIG. 16; and the corresponding linked PHR data can be viewed across different applications.

### <Processes>

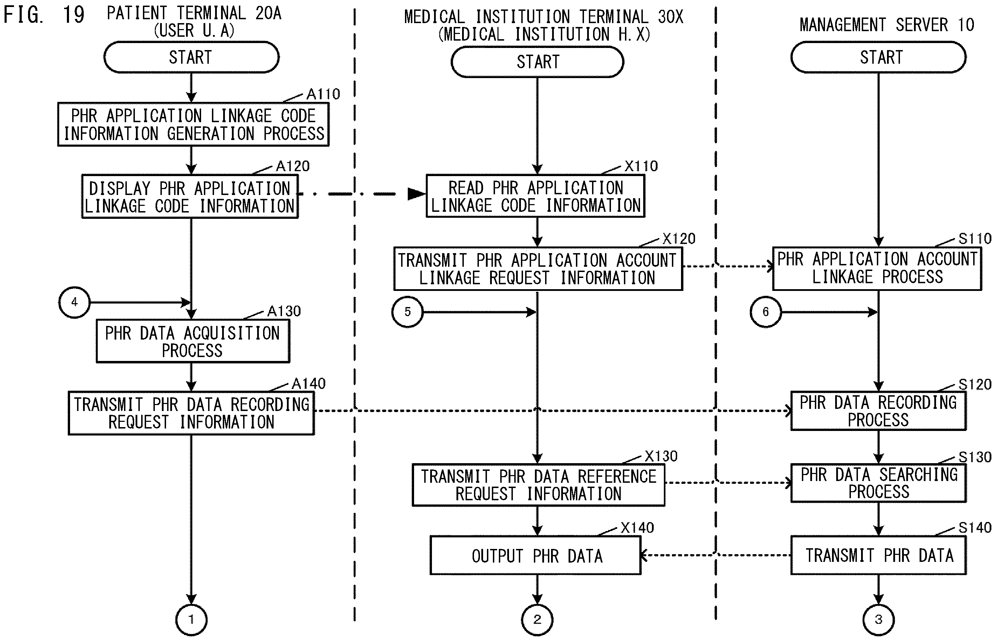

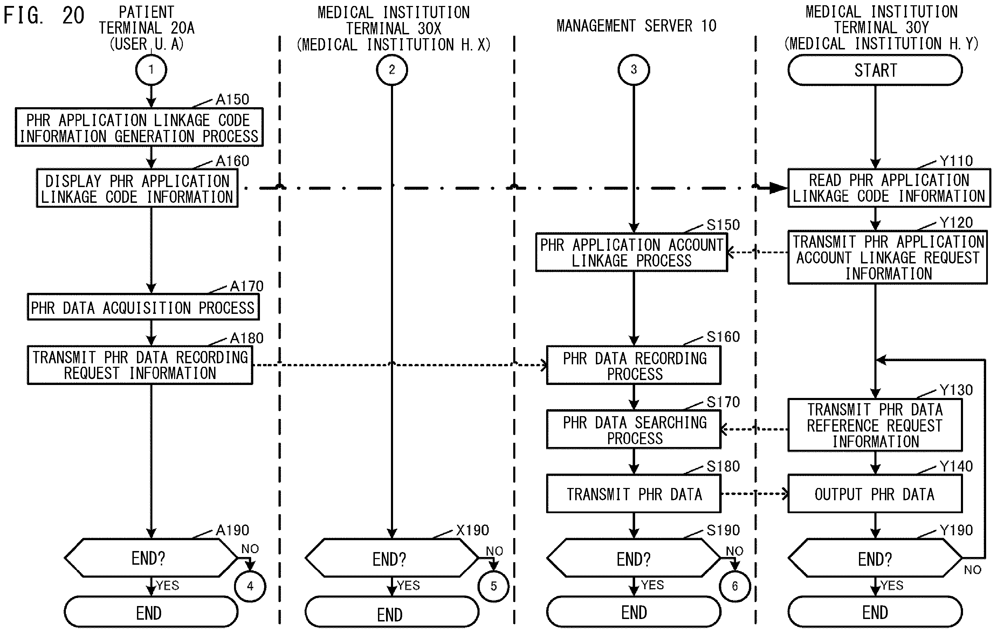

FIG. 19 and FIG. 20 illustrate flowcharts showing an example of the flow of processes to be executed by respective devices according to the present implementation example. FIG. 19 shows an example of a process to be executed by the controller 21 of the patient terminal 20A of the user U.A, an example of a process to be executed by a controller 31 of a medical institution terminal 30X in a medical institution H.X, and an example of a process to be executed by the controller 11 of the management server 10 in this order from the left side. FIG. 20 further shows an example of a process to be executed by a controller 31 of a medical institution terminal 30Y in a medical institution H.Y in a case where a linkage with the medical institution terminal 30Y is performed.

The processes described below are merely examples of processes for implementing the method of the present disclosure, and no limitation to these processes is made. Also, another step may be added to the processes described below, or any of the steps may be omitted (deleted) from the processes described below.

It is assumed that, before the processes in the present flowcharts are started, accounts of the user U.A and the medical institution H.X have already been registered (created) in the PHR platform, and PHR applications (e.g., ASD diagnostic applications) identified by the same PHR application ID have been installed on the patient terminal 20A and the medical institution terminal 30X.

Hereinafter, an example of the flow of the process to be executed by the controller of each of the patient terminal 20A, the medical institution terminal 30X, and the management server 10 will be described with reference to FIG. 19.

First, the controller 21 of the patient terminal 20A executes a PHR application linkage code information generation process based on input performed through the input/output unit 23 (e.g., user input (operation input, voice input, or the like performed by the user); the same may apply below), for example (A110). In the PHR application linkage code information generation process, the controller 21 of the patient terminal 20A generates PHR application linkage code information which is a one-dimensional code or a two-dimensional code based on PHR application linkage information including the user account ID of the patient terminal 20A and the PHR application ID of the PHR application through which a PHR data linkage is to be performed, for example. A code image such as a one-dimensional code or a two-dimensional code may be considered as one type of code information. Information encoded into the code image may also be considered as one type of code information. The PHR application linkage information and the PHR application linkage code information may include information (e.g., a patient name, a birthday, a sex, and the like) for identifying the patient.

Then, the controller 21 of the patient terminal 20A causes the display 24 to display the generated PHR application linkage code information, for example (A120).

The controller 31 of the medical institution terminal 30X causes, based on user input, the imaging unit 37 to read the PHR application linkage code information displayed on the patient terminal 20A, for example (X110). Then, the controller 31 of the medical institution terminal 30X decodes the read PHR application linkage code information so as to acquire the PHR application linkage information.

The method for relaying the PHR application linkage information is not limited to the method performed with such a code image. For example, the controller 21 of the patient terminal 20A may achieve the relaying by transmitting the PHR application linkage information to the medical institution terminal 30X through P2P communication by utilizing short-range wireless communication according to Bluetooth, NFC, or the like, for example.

Alternatively, for example, the controller 31 of the medical institution terminal 30X may receive the PHR application linkage information by receiving, through user input, the user account ID, the PHR application ID, or the like displayed on the patient terminal 20A.

The controller 21 of the patient terminal 20A may cause displaying of the PHR application linkage code information or transmit the PHR application linkage information when determining, based on user input, that the user has consented to the PHR data linkage.

Then, the controller 31 of the medical institution terminal 30X transmits, to the management server 10, PHR application account linkage request information including the user account ID and the PHR application ID which are based on the PHR application linkage information, and the medical institution account ID of the medical institution terminal 30X (X120).

In response to the PHR application account linkage request information from the medical institution terminal 30X, the controller 11 of the management server 10 executes a PHR application account linkage process (S110). In the PHR application account linkage process, the controller 11 of the management server 10 determines the user account ID of the patient as a linkage source based on the received PHR application account linkage request information. The controller 11 stores, in association with the corresponding user account ID in the account ID-PHR application linkage management data 159, the medical institution account ID as a linked pair and the PHR application ID regarding which the patient has permitted a linkage, for example.

For example, in a case where a PHR data linkage is performed without limitation to any PHR application by the patient, the PHR application ID in the PHR application linkage information may include all-applications permitting flag information (e.g., "*") or a flag indicating that all PHR applications are permitted to be linked, for example. In this case, the management server 10 may save the all-applications permitting flag information as a PHR application ID in the account ID-PHR application linkage management data 159 and may store the all-applications permitting flag information in association with the medical institution account ID.

In addition, upon completion of the PHR application account linkage process, the controller 11 of the management server 10 may transmit PHR linkage notification information to the patient terminal 20A and/or the medical institution terminal 30X. The notification may indicates that PHR data linkage has been completed. When receiving the PHR linkage notification information from the management server 10, the patient terminal 20A and/or the medical institution terminal 30X may output (e.g., display and output) the received PHR linkage notification information.

Following A120, the controller 21 of the patient terminal 20A executes a PHR data acquisition process based on user input, for example (A130). In the PHR data acquisition process, the controller 21 of the patient terminal 20A acquires PHR data through the input/output unit 23 of the patient terminal 20A based on the PHR application process program 281, for example.

In the PHR data acquisition process, PHR data may be automatically recorded without user input or may be provided from another person. For example, the patient terminal 20A may automatically measure the number of steps that the patient has taken in walking, and may acquire data of the measured number of steps as PHR data. Alternatively, the patient terminal 20A may receive prescription information for the patient from a doctor or a pharmacist and acquire the received prescription information as PHR data.

The controller 21 of the patient terminal 20A transmits, to the management server 10, PHR data recording request information. The PHR data recording request information includes: (i) the acquired PHR data, (ii) the PHR application ID of the PHR application, and (iii) the user account ID of the patient terminal 20A (A140).

When receiving the PHR data recording request information from the patient terminal 20A, the controller 11 of the management server 10 executes, for example, a PHR data recording process (S120). In the PHR data recording process, the controller 11 of the management server 10 refers to the account ID-PHR application linkage management data 159, for example. Then, the controller 11 stores, based on the PHR data recording request information, the PHR application ID and the PHR data in association with the PHR data management data (e.g., the data shown in FIG. 9) for the corresponding user account ID, for example. Information about the location of the PHR data may be acquired and stored instead of acquiring and storing the PHR data itself, and these processes may be considered as an example of processes for registering the health data of the patient to the sharing system.

In the PHR data recording process, the management server 10 may cause pieces of PHR data to be stored without being divided by PHR application IDs. That is, in the PHR data management data, pieces of PHR data from various types of PHR applications may be stored such that the applications from which the pieces of PHR data have been acquired cannot be identified.

After X120, the controller 31 of the medical institution terminal 30X transmits PHR data reference request information to the management server 10 based on user input, for example (X130). Here, the PHR data reference request information may include:
(A1) the user account ID of a patient regarding whom PHR data is to be referred to, the medical institution account ID, and the PHR application ID corresponding to the PHR data to be referred to;
(A2) user information (e.g., a name, a birth date, and the like) about a patient regarding whom PHR data is to be referred to, the medical institution account ID, and the PHR application ID corresponding to the PHR data to be referred to;
(A3) the user account ID of a patient regarding whom PHR data is to be referred to and the medical institution account ID; or
(A4) user information about a patient regarding whom PHR data is to be referred to and the medical institution account ID.

Upon receiving the PHR data reference request information from the medical institution terminal 30X, the controller 11 of the management server 10 executes a PHR data searching process (which may be referred to as a PHR data identification process) to identify PHR data based on the PHR data reference request information, for example (S 130). In the PHR data searching process, the controller 11 of the management server 10 may refer to the account ID-PHR application linkage management data 159 and determines whether the user account ID, the medical institution account ID, and the PHR application ID included in the PHR data reference request information are associated, for example. Upon determining that these IDs are associated with one another, the controller 11 of the management server 10 may refer to the corresponding PHR data management data to identify the requested PHR data associated with the PHR application ID and read the identified PHR data, for example.

In the PHR data searching process, in a case where the above information "(A2)" or "(A4)" is included in the PHR data reference request information, the controller 11 of the management server 10 may refer to the PHR user account registered data 153 and search for a user account ID that matches the user information about the patient, for example.

Meanwhile, in the PHR data searching process, in a case where the above information "(A3)" or "(A4)" is included in the PHR data reference request information, the controller 11 of the management server 10 may refer to the account ID-PHR application linkage management data 159 and read, from the PHR data management data corresponding to the user account ID of the patient, pieces of PHR data corresponding to respective PHR application IDs associated with the medical institution account ID included in the PHR data reference request information, for example. That is, pieces of PHR data may be able to be collectively transmitted in all PHR applications or selected PHR applications through which a linkage with a certain medical institution has been performed in the PHR platform.

After reading the PHR data, the controller 11 of the management server 10 transmits the PHR data to the medical institution terminal 30X, for example (S140).

Upon receiving the PHR data from the management server 10, the controller 31 of the medical institution terminal 30X outputs the received PHR data (X140). For example, the controller 31 causes the display 34 to display and output the PHR data.

Hereinafter, an example of performing a linkage with the medical institution terminal 30Y will be described with reference to FIG. 20.

The controller 21 of the patient terminal 20A generates the PHR application linkage code information (A150) and causes the PHR application linkage code information to be displayed (A160) in the same manner as in steps A110 and A120, for example.

A controller 31 of the medical institution terminal 30Y reads the PHR application linkage code information in the same manner as in step X110, for example (Y110). Then, the controller 31 of the medical institution terminal 30Y transmits PHR application account linkage request information to the management server 10 in the same manner as in step X120, for example (Y120).

Meanwhile, the controller 21 of the patient terminal 20A acquires PHR data (A170) and transmits PHR data recording request information to the management server 10 (A180) in the same manner as in steps A130 and A140, for example.

Then, the controller 11 of the management server 10 executes the PHR data recording process in the same manner as in step S120, for example (S160).

The controller 31 of the medical institution terminal 30Y transmits PHR data reference request information to the management server 10 in the same manner as in step X130, for example (Y130).

Upon receiving the PHR data reference request information from the medical institution terminal 30Y, the controller 11 of the management server 10 executes PHR data searching process to identify the requested PHTR data in the same manner as in step S130 (S170) and transmits the identified PHR data to the medical institution terminal 30Y in the same manner as in step S140 (S180), for example.

Upon receiving the PHR data from the management server 10, the controller 31 of the medical institution terminal 30Y causes outputting of the PHR data in the same manner as in step X140, for example (Y140).

That is, the patient can provide PHR data acquired by one or more selected PHR applications to one or more selected medical institutions.

The controller 21 of the patient terminal 20A may transmit PHR data reference request information to the management server 10 based on user input, for example. Then, the controller 11 of the management server 10 may transmit the requested PHR data to the patient terminal 20A based on the received PHR data reference request information. Upon receiving the PHR data from the management server 10, the controller 21 of the patient terminal 20A may cause the display 24 to output the received PHR data.

In the first implementation example, PHR data related to health of a patient is shared among the management server 10 which manages the PHR data, a plurality of types of PHR applications which acquire the PHR data and register the PHR data to the management server 10, and a medical institution terminal 30 which allows viewing of the PHR data registered to the management server 10. As mainly shown in FIG. 9, a linkage between (A) a user account ID which allows access to the management server 10 through applications corresponding to the patient and (B) a medical institution account ID which allows the medical institution terminal 30 to access the management server 10 is managed in association with at least one of the applications. The PHR data is registered to the management server 10 in association with the user account ID and the application through which the PHR data has been acquired. Then, as mainly shown in FIG. 19, FIG. 20, a reference request for PHR data associated with a medical institution account ID corresponding to a medical institution terminal 30 is received from the medical institution terminal 30, and the data acquired by an application associated with the user account ID and the medical institution account ID is provided to the medical institution terminal 30 which is a request source.

### <Advantageous Effects of First Implementation Example>

In the first implementation example, the data sharing system (e.g., the management server 10) makes it possible to share health data (e.g., PHR data) of a patient between (i) a plurality of types of applications (e.g., a plurality of types of PHR applications) which acquire the health data of the patient and (ii) a medical institution terminal. In addition, a linkage between a first account ID which allows the patient to access the data sharing system and a second account ID which allows a user of the medical institution terminal to access the data sharing system is stored in association with at least one of the applications. Consequently, the health data related to the specific application can be identified in response to a request from the medical institution based on the managed linkage between the account IDs.

In addition, in the first implementation example, as shown in FIG. 7, FIG. 9, and the like, a plurality of pieces of PHR data corresponding to the plurality of types of respective applications can be registered in the data storage 15 (an example of the data storage of the data sharing system) of the management server 10. Consequently, various types of PHR data can be provided to the medical institution.

In addition, in the first implementation example, as shown in FIG. 9 and the like, in a case where a plurality of applications are operated in the patient terminal as shown in FIG. 11 and the like, an account in the management server 10 does not need to be set for each of the PHR applications. Consequently, usability for the patient is improved.

In addition, in the first implementation example, as shown in FIG. 9 and the like, the plurality of types of PHR applications can be associated with the linkage between the user account ID and the medical institution account ID. Consequently, pieces of health data acquired by the plurality of types of respective PHR applications can be collectively managed.

In addition, in the first implementation example, as shown in FIG. 9 and the like, each of the plurality of types of PHR applications corresponding to the user account ID can be associated with a plurality of the medical institution account IDs. Consequently, the PHR data can be shared through linkages with the plurality of the medical institutions (terminals) for each of the plurality of PHR applications.

In addition, in the first implementation example, as shown in FIG. 19, FIG. 20, and the like, a linkage request which includes (i) the user account ID, (ii) the medical institution account ID, and (iii) an application ID of the at least one PHR application is received, and the linkage between the user account ID and the medical institution account ID is managed in association with the at least one PHR application in response to the linkage request. Consequently, the linkage can be managed and the PHR data can be provided, by using the PHR application through which the PHR data can be acquired.

In addition, in the first implementation example, as shown in FIG. 19, FIG. 20, and the like, in response to a reference request which includes information (which is not limited to the user account ID itself and the medical institution account ID itself and which only has to be information that enables identification of the user account ID and the medical institution account ID) that enables identification of the user account ID and the medical institution account ID, the PHR data acquired by the PHR application associated with the user account ID and the medical institution account ID identified by the information can be provided.

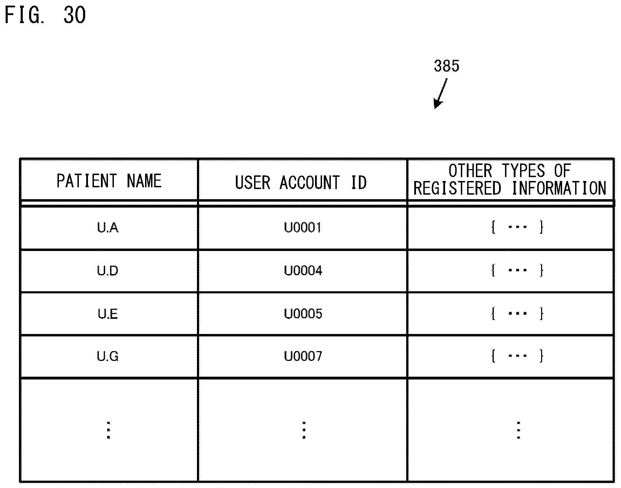

In addition, in the first implementation example, as shown in FIG. 30, FIG. 16, FIG. 19, and the like, the PHR data corresponding to the PHR application of the type associated with the user account ID and the medical institution account ID is provided in response to a reference request which includes specification of the PHR data by personal information (e.g., a name) about the patient. Consequently, provision of necessary PHR data can be requested without acquiring or inputting account information or the like (e.g., the user account ID of the patient or the medical institution account ID) on the medical institution side, whereby usability for the medical institution is improved.

In addition, in the first implementation example, as shown in FIG. 30, FIG. 16, FIG. 19, and the like, a reference request which includes specification of the PHR data by personal information (e.g., a name) about the patient is received, and the PHR data corresponding to the reference request is identified based on: (i) the user account ID corresponding to the personal information about the patient; (ii) the medical institution account ID corresponding to the medical institution terminal 30; and (iii) the PHR application associated with the user account ID and the medical institution account ID. Consequently, the PHR data to be provided can be identified and provided to the medical institution without acquiring or inputting the user account ID of the patient on the medical institution side.

In the first implementation example, as shown in FIG. 16, FIG. 19 and FIG. 20, and the like, in response to a reference request which includes information (which is not limited to the user account ID itself and the medical institution account ID itself and which only has to be information that enables identification of the user account ID and the medical institution account ID) that enables identification of the user account ID and the medical institution account ID, the PHR application associated with the user account ID and the medical institution account ID identified by the information can be identified, and the PHR data acquired by the identified PHR application can be provided.

In addition, in the first implementation example, as shown in FIG. 14 and FIG. 15, FIG. 19 and FIG. 20, and the like, a linkage request which includes information of (i) the user account ID and an application ID, and (ii) the medical institution account ID can be received, the information (i) is provided to the medical institution terminal 30 from a patient terminal 20 with the PHR application installed, and the linkage between the user account ID and the medical institution account ID can be managed in association with the application ID of the PHR application.

In addition, in the first implementation example, as shown in FIG. 14 and FIG. 15, FIG. 19 and FIG. 20, and the like, a linkage request which includes information of (i) the user account ID and an application ID, and (ii) the medical institution account ID can be received, the information (i) is provided to the medical institution terminal 30, based on a consent of the patient or a related person of the patient, from a patient terminal 20 with the PHR application installed, and the linkage between the user account ID and the medical institution account ID can be managed in association with the application ID of the PHR application.

In addition, in the first implementation example, as shown in FIG. 17 and the like, the medical institution terminal 30 runs a medical institution application to view the PHR data, and the medical institution application has compatibility with the plurality of types of PHR applications. Consequently, a person in charge in the medical institution does not need to use different PHR applications correspondingly to respective PHR applications for the patient, whereby usability for the person in charge in the medical institution is improved.

### <Modification (1) of First Implementation Example>

In the above implementation example, the PHR data is registered to the management server 10, and the PHR data is provided from the management server 10 to the medical institution terminal 30. However, no limitation thereto is made.

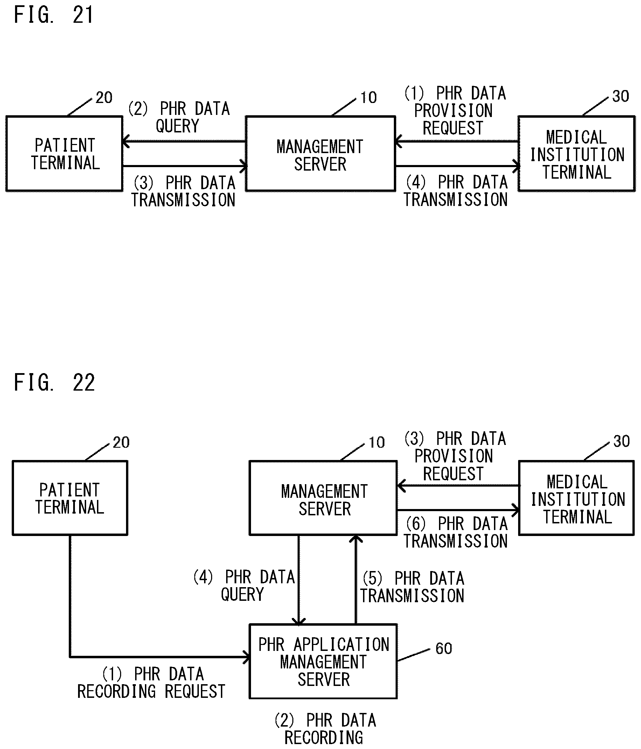

For example, the management server 10 may query (ask) the patient terminal 20 for PHR data acquired by the patient terminal 20 and may present the PHR data acquired from the patient terminal 20 to the medical institution terminal 30. As shown in, for example, FIG. 21, (1) a medical institution server 50 transmits, for example, the aforementioned PHR data reference request information to the management server 10 so as to make a reference request for PHR data of a target patient. (2) The management server 10 queries the patient terminal 20 of the target patient for the PHR data in response to the reference request. (3) The patient terminal 20 transmits the PHR data to the management server 10 in response to the query. (4) The management server 10 transmits the received PHR data to the medical institution terminal 30. For example, "(2)" may be considered as an example of an instance in which the controller of the data sharing system requests, for identification of the health data of the patient, the health data corresponding to each of the plurality of types of applications from the patient terminal. The management server 10 may register the PHR data received from the patient terminal 20 in "(3)". For example, the management server 10 may temporarily store, in the data storage 15, the PHR data received from the patient terminal 20 but may delete the PHR data after transmitting the PHR data to the medical institution terminal 30.

Alternatively, for example, the PHR data may be registered from the patient terminal 20 to a PHR application management server 60 which is a management server provided correspondingly to the PHR application. Then, the management server 10 may ask for the PHR data registered to the PHR application management server 60, thereby presenting the PHR data to the medical institution terminal 30.

The PHR application management server 60 saves (which may be rephrased as "registers") the PHR data acquired by the corresponding PHR application. Meanwhile, the management server 10 has a function of enabling collection and recording of pieces of PHR data in the plurality of types of PHR applications and linkages of the pieces of PHR data (however, the pieces of PHR data do not have to be registered in the present modification). The business entities running the PHR application management server 60 and the management server 10 may be different from or identical to each other.

As shown in, for example, FIG. 22, (1) the patient terminal 20 transmits, for example, the aforementioned PHR data recording request information to the PHR application management server 60 corresponding to the PHR application through which the PHR data has been acquired so as to make a recording request (registration request) for the PHR data. (2) The PHR application management server 60 registers the received PHR data in response to the recording request. (3) The medical institution server 50 transmits, for example, the aforementioned PHR data reference request information to the management server 10 so as to make a reference request for the PHR data of the target patient. (4) The management server 10 queries the PHR application management server 60 for the PHR data in response to the reference request. (5) The PHR application management server 60 transmits the PHR data to the management server 10 in response to the query. (6) The management server 10 transmits the received PHR data to the medical institution terminal 30. For example, "(4)" may be considered as an example of an instance in which the controller of the data sharing system requests, for identification of the health data, the health data corresponding to each of the plurality of types of applications from one or more corresponding application management servers. The management server 10 may, or does not have to, register the PHR data received from the PHR application management server 60 in "(5)". For example, the management server 10 may temporarily store, in the data storage 15, the PHR data received from the PHR application management server 60 but may delete the PHR data after transmitting the PHR data to the medical institution terminal 30. The transmission of the PHR data to the medical institution terminal 30 does not have to be performed via the management server 10 as in "(5)" and "(6)". That is, the management server 10 may request the PHR application management server 60 to directly transmit the PHR data from the PHR application management server 60 to the medical institution terminal 30. This type of request may be considered as an example of an instance in which the controller of the data sharing system makes, for identification of the health data, a request to transmit the health data corresponding to each of the plurality of types of applications to the medical institution terminal, the request being made to the one or more corresponding application management servers.

In the present modification, the controller of the data sharing system (e.g., the controller 11 of the management server 10) can request, for identification of the health data (e.g., the PHR data) of the patient, the health data corresponding to each of the plurality of types of applications (e.g., the plurality of types of PHR applications) from the patient terminal. Also, in this case, the plurality of types of PHR data (an example of the health data corresponding to each of the plurality of types of applications) may be able to be acquired by the patient terminal 20 corresponding to the patient, and the data sharing system may be able to request the PHR data corresponding to each of the plurality of types of PHR applications (an example of the plurality of types of applications) from the patient terminal 20. Consequently, pieces of health data that are related to the health of the patient and that correspond to the plurality of types of respective applications do not need to be registered to the sharing system, and the pieces of health data can be provided to the medical institution terminal by querying the patient terminal for the pieces of health data from the sharing system.

In addition, in the present modification, the controller of the data sharing system (e.g., the controller 11 of the management server 10) can request, for identification of the health data (e.g., the PHR data) of the patient, the health data corresponding to each of the plurality of types of applications (e.g., the plurality of types of PHR applications) from the one or more corresponding application management servers 60. In this case, a plurality of types of PHR data (an example of the health data corresponding to each of the plurality of types of applications) may be able to be registered to a plurality of types of the PHR application management servers 60 (an example of a plurality of types of application management servers corresponding to the plurality of types of applications), and the data sharing system may be able to request the PHR data corresponding to each of the plurality of types of the PHR application management servers 60 from the PHR application management server 60. Consequently, pieces of health data that are related to the health of the patient and that correspond to the plurality of types of respective applications do not need to be registered to the sharing system, and the pieces of health data can be provided to the medical institution terminal by querying the plurality of types of application management servers for the pieces of health data from the sharing system.

### <Modification (2) of First Implementation Example>

In the above implementation example, PHR application linkage code information is transmitted from the patient terminal 20 to the medical institution terminal 30. However, no limitation thereto is made. For example, PHR application linkage code information presented on the medical institution terminal 30 may be read by the patient terminal 20.

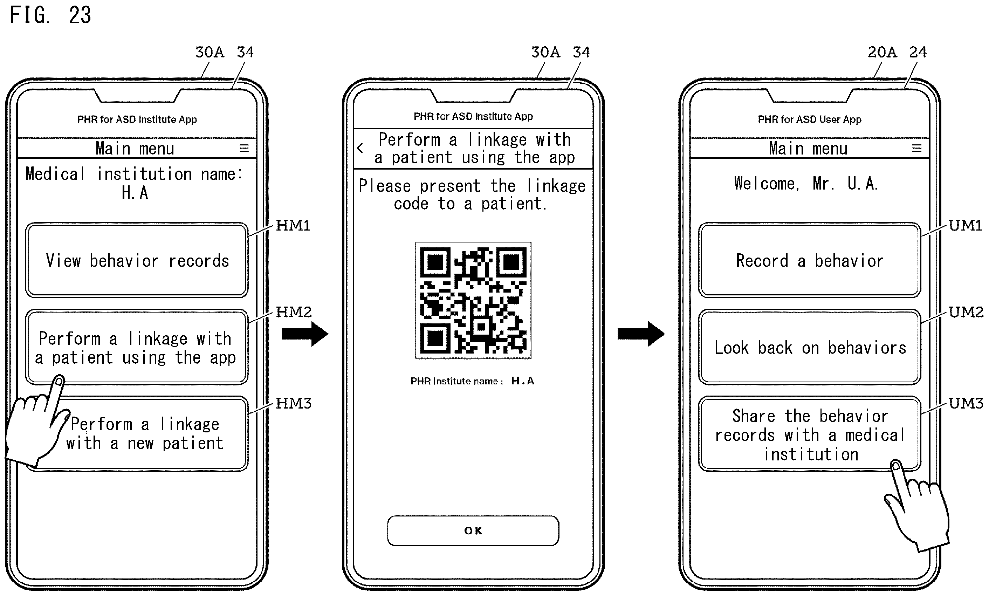

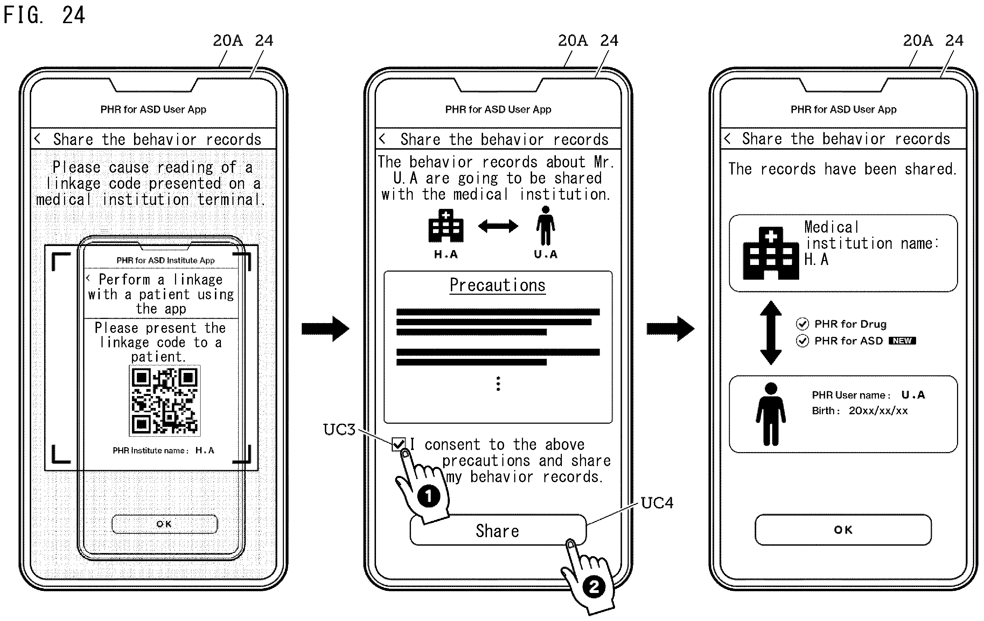

FIG. 23 and FIG. 24 show examples of screens displayed on the display 24 of the patient terminal 20A and the display 34 of the medical institution terminal 30A according to the present modification.

Upon the function button HM2 being tapped in the main menu screen of the medical-institution-side ASD diagnostic application on the medical institution terminal 30A on the left side of FIG. 23, the screen displayed on the medical institution terminal 30A is changed to a screen to display PHR application linkage code information at the center of FIG. 23. This screen is configured to display, for example, PHR application linkage code information expressed by a two-dimensional code and the medical institution name in the PHR application. The user of the patient terminal 20 can ascertain a PHR data sharing destination by checking the medical institution name, for example.

When the function button UM3 is tapped in the main menu screen of the patientside ASD diagnostic application on the patient terminal 20A on the right side of FIG. 23, the screen displayed on the patient terminal 20A is changed to a PHR application linkage code information reading screen on the left side of FIG. 24. The user moves the patient terminal 20A according to guidance information for prompting placement, within a preview screen, of the PHR application linkage code information displayed on the medical institution terminal 30, and the patient terminal 20A reads the PHR application linkage code information. Consequently, the screen displayed on the patient terminal 20A is changed to a PHR data sharing confirmation screen at the center of FIG. 24. When, for example, the user taps a checkbox UC3 for consenting to sharing of the PHR data with the medical institution H.A, the display mode of a PHR data sharing function button UC4 is changed from a grayed-out display mode to an ordinary display mode so that operation is enabled. Then, the PHR data sharing function button UC4 is tapped, whereby the management server 10 executes the PHR application account linkage process, and the screen displayed on the patient terminal 20A is changed to a screen to display PHR linkage notification information on the right side of FIG. 24.

PHR linkage notification information may be displayed on the medical institution terminal 30A upon execution of the PHR application account linkage process.

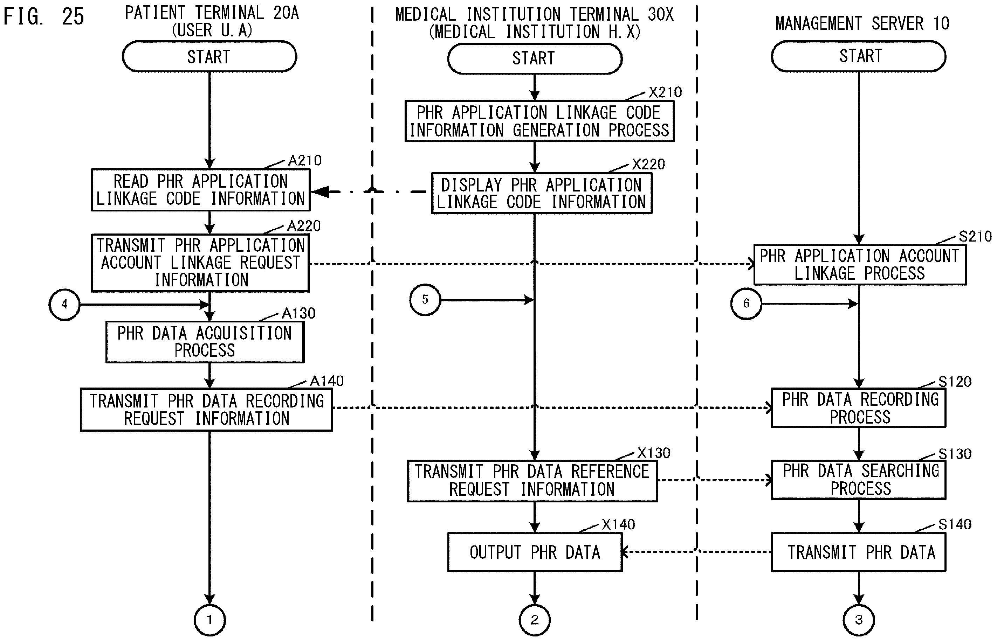

FIG. 25 is a flowchart showing an example of the flow of processes to be executed by the respective devices according to the present modification. This drawing shows an example of a process to be executed by the controller 21 of the patient terminal 20A of the user U.A, an example of a process to be executed by the controller 31 of the medical institution terminal 30X in the medical institution H.X, and an example of a process to be executed by the controller 11 of the management server 10 in this order from the left side.

First, the controller 31 of the medical institution terminal 30X executes a PHR application linkage code information generation process based on user input, for example (X210). In the PHR application linkage code information generation process, the controller 31 of the medical institution terminal 30X generates PHR application linkage code information which is a one-dimensional code or a two-dimensional code based on PHR application linkage information including the medical institution account ID of the medical institution terminal 30X and the PHR application ID of the PHR application through which a PHR data linkage is to be performed, for example. The PHR application linkage information and the PHR application linkage code information may include information (e.g., a medical institution name or the like) for identifying the medical institution.

Then, the controller 31 of the medical institution terminal 30X causes the display 34 to display and output the generated PHR application linkage code information, for example (X220).

The controller 21 of the patient terminal 20A causes, based on user input, the imaging unit 27 to read the PHR application linkage code information displayed on the medical institution terminal 30X, for example (A210). Then, the controller 21 of the patient terminal 20A decodes the read PHR application linkage code information so as to acquire the PHR application linkage information.

Alternatively, the controller 31 of the medical institution terminal 30X may achieve the relaying by transmitting the PHR application linkage information to the patient terminal 20A through P2P communication by utilizing short-range wireless communication according to Bluetooth, NFC, or the like, for example.

Alternatively, for example, the controller 21 of the patient terminal 20A may receive the PHR application linkage information by receiving, through user input, the medical institution account ID, the PHR application ID, or the like displayed on the medical institution terminal 30X.

Then, the controller 21 of the patient terminal 20A transmits, to the management server 10, PHR application account linkage request information including the medical institution account ID and the PHR application ID which are based on the PHR application linkage information, and the user account ID of the patient terminal 20A (A220).

The controller 21 of the patient terminal 20A may transmit the PHR application account linkage request information to the management server 10 when determining, based on user input, that the user has consented to the PHR data linkage.

Upon receiving the PHR application account linkage request information from the patient terminal 20A, the controller 11 of the management server 10 executes the PHR application account linkage process (S210). The PHR application account linkage process may be performed in the same manner as in S110 in FIG. 19, for example.

In the present modification, as shown in FIG. 23 and FIG. 24, FIG. 25, and the like, a linkage request which includes information of (i) the medical institution account ID provided from the medical institution terminal 30 to a patient terminal 20 with the PHR application installed, and (ii) the user account ID and an application ID, can be received, and the linkage between the user account ID and the medical institution account ID can be managed in association with the application ID of the PHR application.

In addition, in the present modification, as shown in FIG. 23 and FIG. 24, FIG. 25, and the like, a linkage request which includes information of (i) the medical institution account ID provided from the medical institution terminal 30 to a patient terminal 20 with the PHR application installed, and (ii) the user account ID and an application ID, can be received in response to a consent of the patient or a related person of the patient, and the linkage between the user account ID and the medical institution account ID can be managed in association with the application ID of the PHR application.

### <Modification (3) of First Implementation Example>

In the above implementation example, the PHR application linkage code information is transmitted from the patient terminal 20 to the medical institution terminal 30. However, no limitation thereto is made. For example, the PHR application account linkage process may be executed by selecting, on the patient terminal 20, a medical institution with which PHR data sharing is to be performed.

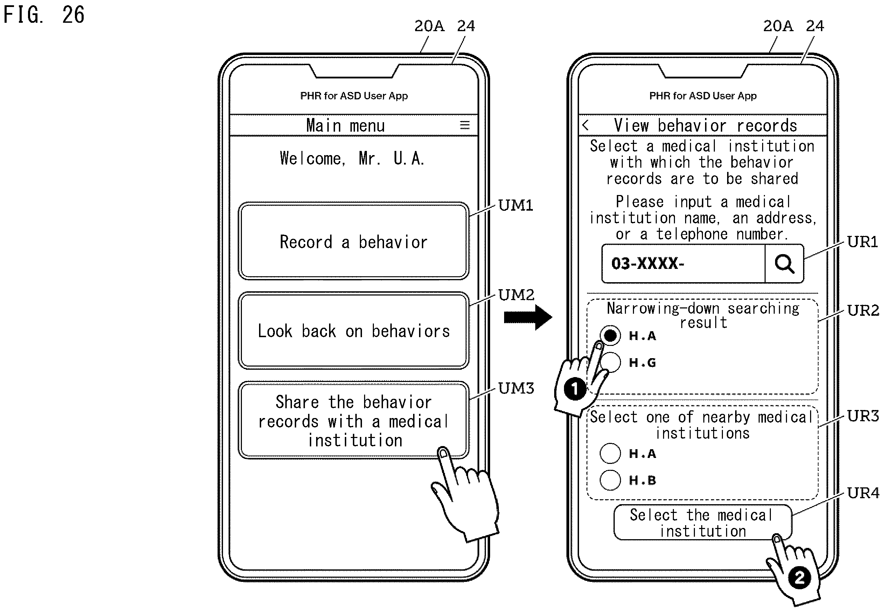

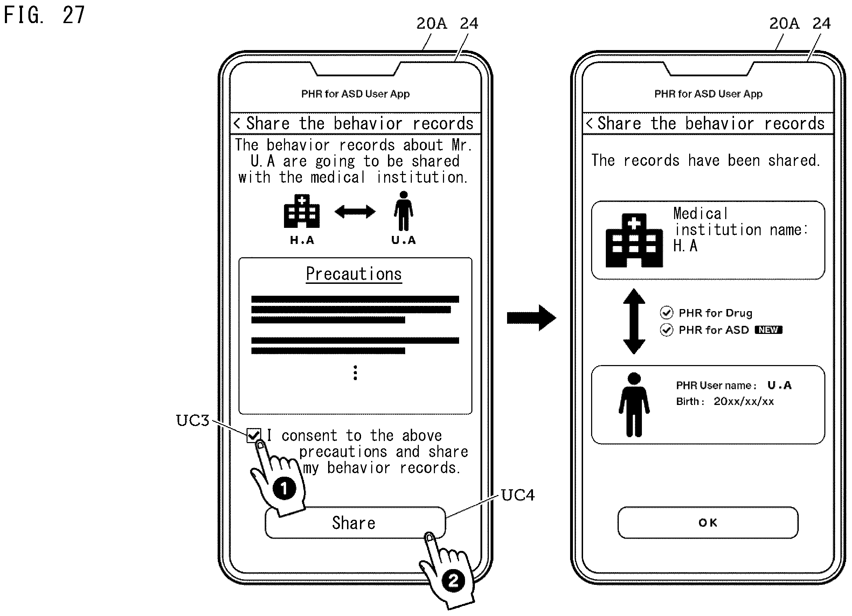

FIG. 26 and FIG. 27 show examples of screens displayed on the display 24 of the patient terminal 20A and the display 34 of the medical institution terminal 30A according to the present modification.

When the function button UM3 is tapped in the main menu screen of the patientside ASD diagnostic application on the left side of FIG. 26 on the patient terminal 20A, the screen displayed on the patient terminal 20A is changed to a PHR application linkage medical institution selection screen on the right side of FIG. 26. This screen is configured such that a medical institution information input region UR1 for narrowing down medical institutions with which the PHR data is to be shared is displayed. In addition, this screen is configured such that a narrowed-down medical institution selection region UR2 for displaying a list of medical institutions obtained by the narrowing-down performed based on the content inputted in the medical institution information input region UR1 is displayed beneath the medical institution information input region UR1. This screen is configured such that, for example, a nearby medical institution selection region UR3 for displaying a list of medical institutions existing within a predetermined distance (e.g., 1 km) from the patient terminal 20A is displayed beneath the narrowed-down medical institution selection region UR2 based on the positional information about the patient terminal 20A.

For example, in a case where the patient terminal 20A exists inside a medical institution (e.g., H.A), the medical institution H.A may be selectable in the nearby medical institution selection region UR3. The medical institution terminal 30A with which a linkage is to be performed may be set in the medical institution H.A.

When, for example, H.A is selected from among the medical institutions displayed in the narrowed-down medical institution selection region UR2, and a PHR data sharing destination medical institution selection button UR4 is tapped, the screen displayed on the patient terminal 20A is changed to a PHR data sharing confirmation screen on the left side of FIG. 27.

Then, when the PHR data sharing function button UC4 is tapped, the management server 10 executes the PHR application account linkage process, and the screen displayed on the patient terminal 20A is changed to a screen to display PHR linkage notification information on the right side of FIG. 27.

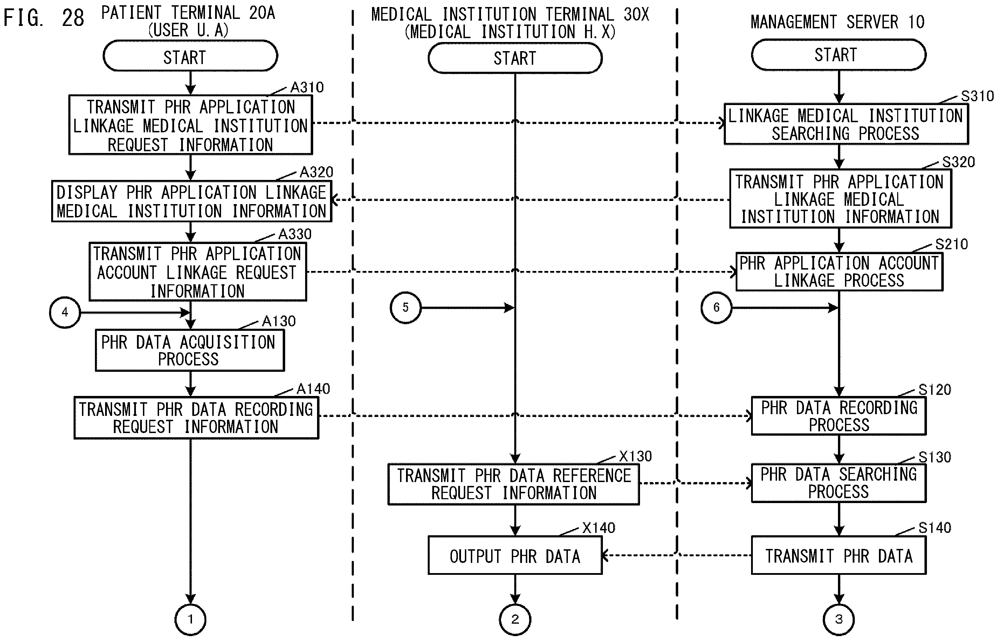

FIG. 28 is a flowchart showing an example of the flow of processes to be executed by the respective devices according to the present modification. This drawing shows an example of a process to be executed by the controller 21 of the patient terminal 20A of the user U.A, an example of a process to be executed by the controller 31 of the medical institution terminal 30X in the medical institution H.X, and an example of a process to be executed by the controller 11 of the management server 10 in this order from the left side.

First, the controller 21 of the patient terminal 20A transmits, to the management server 10, PHR application linkage medical institution request information for requesting a list of medical institutions as linkage destinations (PHR data sharing destinations) based on user input, for example (A310). The PHR application linkage medical institution request information may include, for example, the following information:
(B 1) the user account ID of the patient terminal 20A;
(B2) the positional information about the patient terminal 20A;
(B3) a part or the entirety of the name of a medical institution received through the user input;
(B4) a part or the entirety of the telephone number of a medical institution received through the user input; or
(B5) a part or the entirety of the address of a medical institution received through the user input.

When receiving the PHR application linkage medical institution request information from the patient terminal 20A, the controller 11 of the management server 10 executes a linkage medical institution searching process (S310). In the linkage medical institution searching process, the controller 11 of the management server 10 refers to the PHR medical institution account registered data 155 and searches for matching medical institution account IDs based on the received PHR application linkage medical institution request information, for example.

In the linkage medical institution searching process, in a case where, for example, the above information of "(B2)" is included in the PHR application linkage medical institution request information, the controller 11 of the management server 10 may search for medical institution account IDs of medical institutions existing within a predetermined range (e.g., having a radius of 1 km) from the position at which the patient terminal 20A exists. In this case, for example, the controller 11 may request positional information from the patient terminal 20A so as to acquire the positional information from the patient terminal 20A, or pieces of positional information may be transmitted from the patient terminal 20A to the management server 10 at regular timings so as to, for example, perform searching by using the latest one of the pieces of positional information about the patient terminal 20A. Alternatively, searching may be performed by using pieces of positional information obtained in the past over a predetermined period from the patient terminal 20A (as in a case where, for example, information about a position resulting from averaging the positions obtained in the past over the predetermined period is used).

Meanwhile, in the linkage medical institution searching process, in a case where, for example, the above information of any of "(B3)", "(B4)", and "(B5)" is included in the PHR application linkage medical institution request information, the controller 11 of the management server 10 may search for medical institution account IDs of medical institutions that match the name, the telephone number, or the address of a medical institution.

Then, the controller 11 of the management server 10 transmits, to the patient terminal 20A, PHR application linkage medical institution information which is a list of pieces of information about the medical institution account IDs searched for in the linkage medical institution searching process (S320).

The PHR application linkage medical institution information may include information such as the names or the locations of the medical institutions in association with the medical institution account IDs.

When receiving the PHR application linkage medical institution information from the management server 10, the controller 21 of the patient terminal 20A causes the received PHR application linkage medical institution information to be displayed and outputted, for example (A320). The controller 21 of the patient terminal 20A selects a desired medical institution from the PHR application linkage medical institution information based on user input, for example. Then, the controller 21 of the patient terminal 20A transmits, to the management server 10, PHR application account linkage request information including the selected medical institution ID, the PHR application ID, and the user account ID of the patient terminal 20A (A330).

Consequently, even in a case where the patient terminal and an arbitrarily-selected medical institution terminal are not close to each other, the patient can cause a PHR data linkage with the medical institution.

### <Modification (4) of First Implementation Example>

In the above implementation example, in a case where a patient regarding whom PHR data is to be referred to is specified on the medical institution terminal 30, the management server 10 identifies a user account ID of the patient. However, no limitation thereto is made. For example, the user account ID may be searched for on the medical institution terminal 30 based on the name, the birth date, or the like of the patient.

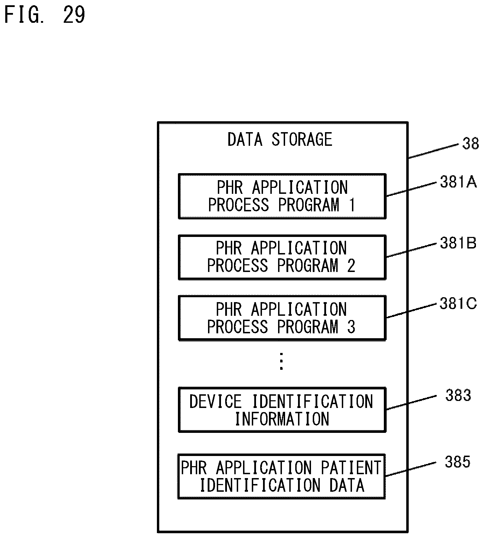

FIG. 29 shows an example of data and the like stored in a data storage 38 of the medical institution terminal 30 according to the present modification. The data storage 38 stores therein, for example, various types of PHR application process programs 381A, 381B, 381C, ··· to be executed as PHR application processes, the device identification information 383 (e.g., a telephone number, an IMEI, or the like), and PHR application patient identification data 385.

The PHR application patient identification data 385 is data for managing (caching) association between patient information such as the name or the birth date of a patient and a user account ID of the patient in the PHR platform, and an example of a data configuration thereof is shown in FIG. 30. In the PHR application patient identification data 385, patient names, user account IDs, and other types of registered information are stored in association with one another, for example.

Each of the patient names may be set to be, for example, the name of the corresponding patient registered in the PHR platform. Alternatively, the patient name and the name of the patient registered in the PHR platform may differ from each other. The patient name is an example of the identification information for the patient.

Each of the user account IDs is, for example, a user account ID of the corresponding patient associated with (subjected to a PHR data linkage with) the medical institution account ID of this medical institution terminal 30 in the PHR application account linkage process.

In the other types of registered information, various types of information may be stored, for example. The various types of information include: PHR application IDs of PHR applications through which the respective patients have caused PHR data linkages; and the birth dates of the patients.

When, for example, receiving PHR linkage notification information from the management server 10 after the management server 10 executes the PHR application account linkage process, the controller 31 of the medical institution terminal 30 may update the PHR application patient identification data 385 by storing the linkage therein.

By thus storing the linkage in the PHR application patient identification data 385 on the medical institution terminal 30, the user of the medical institution terminal can identify a user account ID of a patient by using information different from the identification information for the patient registered to the PHR platform.

In a case where PHR data reference request information is transmitted, the medical institution terminal 30 may refer to the PHR application patient identification data 385 and identify a user account ID from a patient name or the like, for example.

### <Second Implementation Example>

A second implementation example is an implementation example related to a linkage between the PHR platform service provided via a global network such as the Internet and an electronic medical record service provided via a private network such as an intranet.

The features described in the second implementation example are applicable to the other implementation examples and modifications in the same manner.

### <System Configuration>

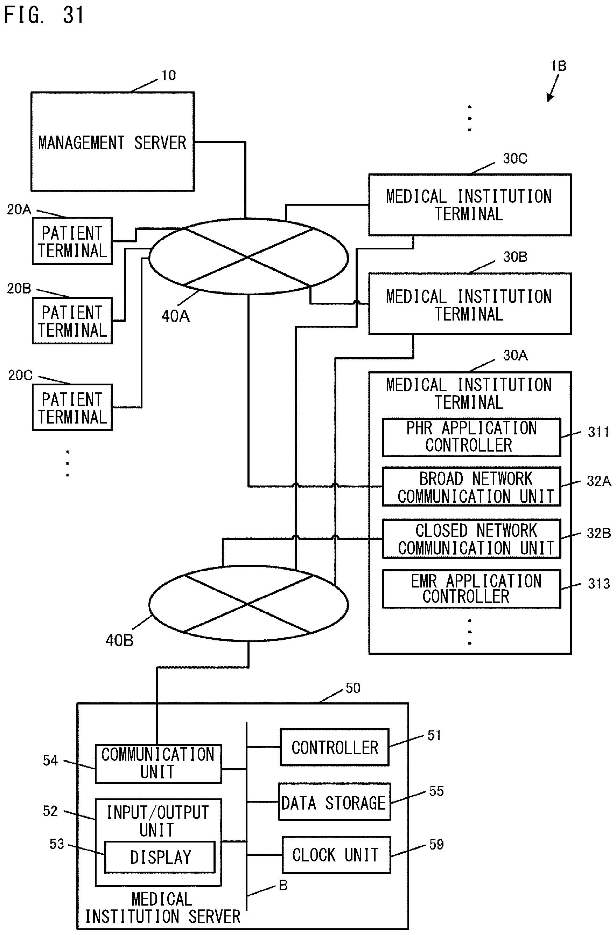

FIG. 31 shows an example of a system configuration of an information system 1B according to the present implementation example. In the information system 1B, the network 40 is separated into a broad communication network 40A such as the Internet and a closed communication network 40B such as an intranet. The broad communication network 40A may be referred to as an open network or a global network. The closed communication network 40B may be referred to as a closed network or a private network.

The closed communication network 40B is, for example, a network implemented by a VPN, a dedicated line, or the like and is a network, other than the Internet, that is usable only by a specific person or in a specific site. A user (e.g., a medical institution) can prevent an attack from a third party outside the closed network and can securely use the system in the closed communication network 40B, by using the closed network.

In the information system 1B, the management server 10, the one or more patient terminals 20, and the one or more medical institution terminals 30 are connected via the broad communication network 40A, for example. In addition, in the information system 1B, the medical institution server 50 and the one or more medical institution terminals 30 are connected via the closed communication network 40B, for example.

The medical institution server 50 is, for example, an information processing device possessed by a business entity running an EMR application and has a function of providing, for example, an electronic medical record service to the medical institution terminals 30 and the like via the closed communication network 40B. The medical institution server 50 may be a single server device or a plurality of server devices. The hardware configuration of the medical institution server 50 may be the same as that of the management server 10, for example.

Each of the medical institution terminals 30 includes, as controllers 31, a PHR application controller 311 and an EMR application controller 313, for example. In addition, the medical institution terminal 30 includes, as communication units 32, a broad network communication unit 32A for performing communication via the broad communication network 40A and a closed network communication unit 32B for performing communication via the closed communication network 40B, for example.

The PHR application controller 311 can use, for example, the broad network communication unit 32A. Meanwhile, the EMR application controller 313 can use, for example, the closed network communication unit 32B. Alternatively, the PHR application controller 311 may be able to use the closed network communication unit 32B.

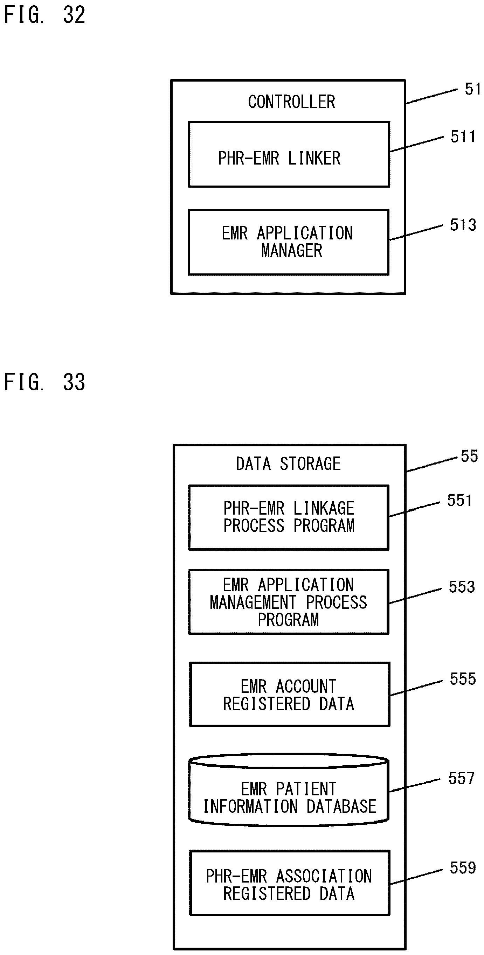

FIG. 32 shows an example of functions realized by a controller 51 of the medical institution server 50 according to the present implementation example. The controller 51 includes, as a functional unit, a PHR-EMR linker 511 which executes a PHR-EMR account linkage process (which may be referred to as a PHR application management auxiliary process) according to a PHR-EMR linkage process program 551 stored in a data storage 55, for example. In addition, the controller 51 includes an EMR application manager 513 which executes an EMR application management process according to an EMR application management process program 553 stored in the data storage 55, for example.

FIG. 33 shows an example of information stored in the data storage 55 of the medical institution server 50 according to the present implementation example. The data storage 55 stores therein, for example, the PHR-EMR linkage process program 551, the EMR application management process program 553, EMR account registered data 555, an EMR patient information database 557, and PHR-EMR association registered data 559.

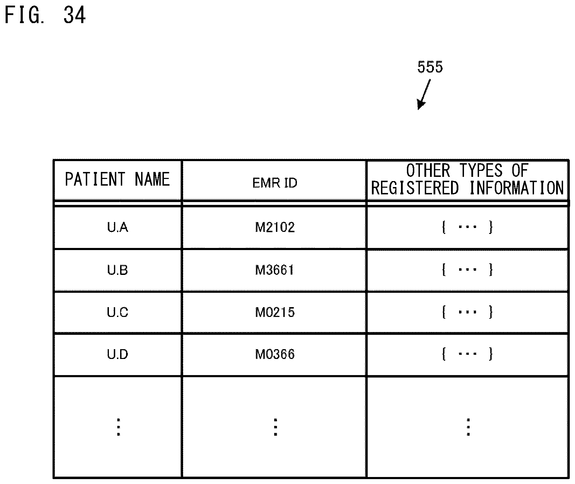

The EMR account registered data 555 is registered data related to patient accounts for a medical institution in which the EMR application is used, and an example of a data configuration thereof is shown in FIG. 34. In the EMR account registered data 555, patient names, EMR IDs, and other types of registered information are stored in association with one another, for example.

Each of the patient names is, for example, a patient name of the corresponding patient who undergoes clinical treatment in the medical institution in which the EMR application is used. The patient name and the corresponding user name in the PHR application which are set for the same patient may be identical to or different from each other.

Each of the EMR IDs is information to be used for identifying an account of the corresponding patient in the EMR application. Each of the EMR IDs is preferably a value unique to the corresponding account, and, for example, a unique value (distinct value) is set and stored for each of the accounts by the medical institution server 50. The EMR ID may be information associated with the medical institution terminal 30 or the medical institution in which the medical institution terminal 30 is used. Alternatively, the EMR ID may be a third account ID.

In the other types of registered information, various types of basic information such as the addresses, the birth dates, or the sexes of the respective patients may be stored, for example.

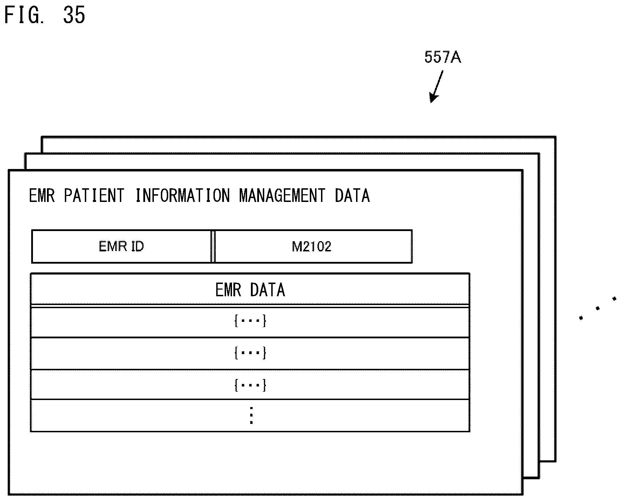

The EMR patient information database 557 is, for example, a database for managing pieces of medical information about certain patients in the medical institution, and an EMR patient information database 557A having a data configuration which is an example of the data configuration of the EMR patient information database 557 is shown in FIG. 35. In the EMR patient information database 557A, pieces of EMR patient information management data are stored as pieces of management data for the respective EMR IDs, for example. In each of the pieces of EMR patient information management data, the corresponding EMR ID and EMR data are stored in association with each other, for example. As the EMR data, various medical information and the like about the patient identified with the EMR ID may be stored.

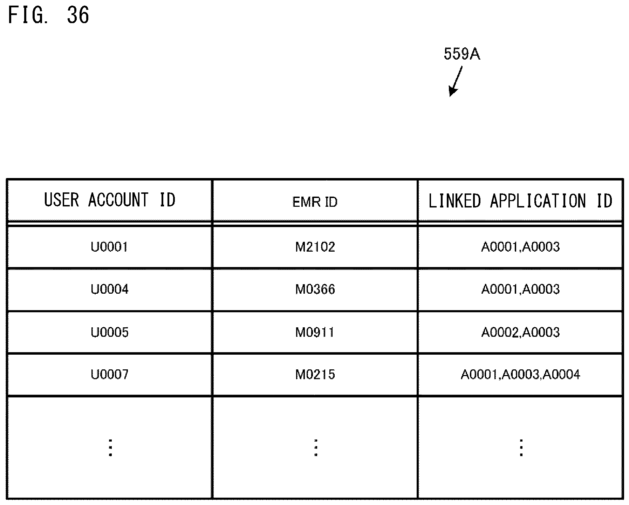

The PHR-EMR association registered data 559 is data for managing the correspondence relationship between each of the user account IDs in the PHR platform and a corresponding EMR ID in the EMR service, and PHR-EMR association registered data 559A having a data configuration which is an example of the data configuration of the PHR-EMR association registered data 559 is shown in FIG. 36. In the PHR-EMR association registered data 559A, user account IDs, EMR IDs, and linked application IDs are stored in association with one another, for example. As each of the linked application IDs, a PHR application ID of a PHR application through which the corresponding patient has caused a linkage with the medical institution may be stored.

The medical institution terminal 30 or the medical institution server 50 may ask the management server 10 for any of the linked application IDs based on a user account ID and a medical institution account ID, thereby acquiring the linked application ID, for example. In a case where the patient causes collective linkages through applications in the PHR platform, the all-applications permitting flag information may be stored for each of the linked application IDs, or the storing item "LINKED APPLICATION ID" does not have to be provided.

### <Displayed Screen>

In this example, the name of the EMR application used in the medical institution is exemplified by being written as "EHR/EMR App".

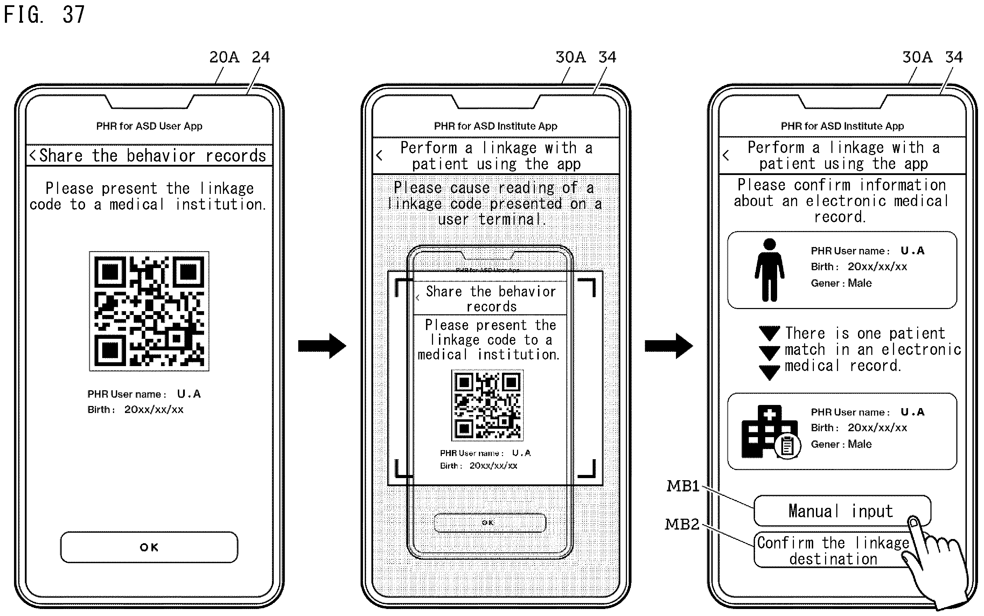

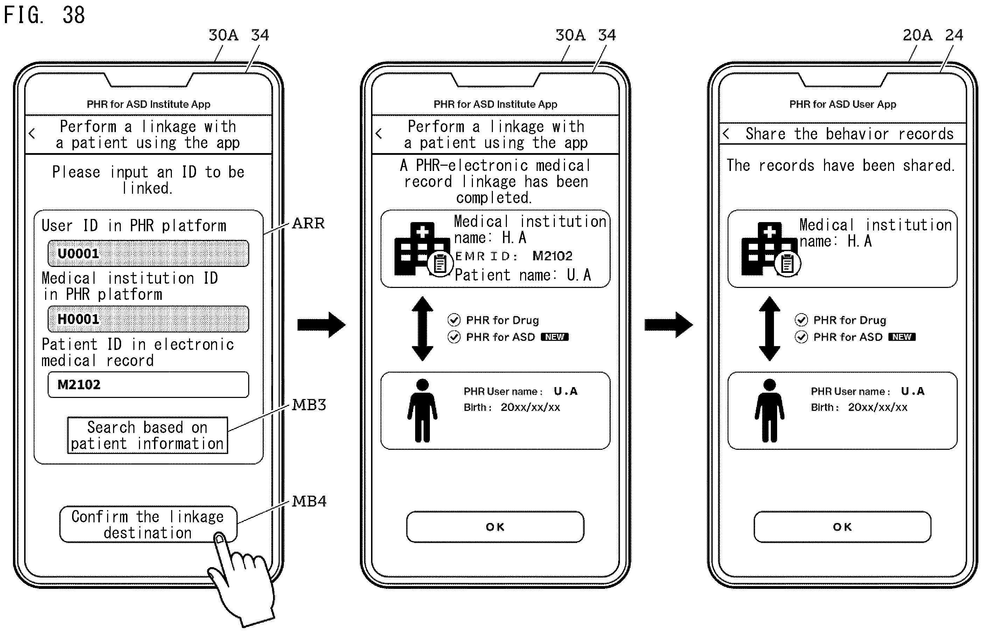

FIG. 37 and FIG. 38 show examples of screens displayed on the display 24 of the patient terminal 20A and the display 34 of the medical institution terminal 30A according to the present implementation example.

The screen on the left side of FIG. 37 is, for example, a PHR application linkage code information display screen displayed on the patient terminal 20A by consenting to sharing of PHR data in the PHR data sharing confirmation screen. For example, the medical institution terminal 30A reads the PHR application linkage code information in a PHR application linkage code information reading screen at the center of FIG. 37, and the management server 10 executes the PHR application account linkage process, whereby the screen displayed on the medical institution terminal 30A is changed to an electronic medical record linkage confirmation screen on the right side of FIG. 37.

This screen is configured as follows, for example. That is, information is displayed in this screen as a result of transmission, of information about the patient regarding whom a PHR application linkage has been performed, by the medical institution terminal 30X to the medical institution server 50. The information displayed in this screen is for asking whether or not to link the user account ID with an EMR ID of a patient who has the same surname and given name and the same birth date, the EMR ID having been selected from the registered EMR account registered data 555 by the medical institution server 50. In addition, this screen is configured such that, for example, a function button MB1 for manually specifying an EMR ID as a linkage destination and a function button MB2 for performing a linkage with the EMR ID automatically selected from the EMR account registered data 555 are displayed beneath the information asking whether or not to perform the linkage.

When, for example, the user taps the function button MB1, the screen displayed on the medical institution terminal 30A is changed to a linkage destination EMR ID input screen in FIG. 38.

This screen has a linkage destination EMR ID input region ARR in which the user account ID and the medical institution account ID linked with each other in the PHR application account linkage process are automatically inputted and are set to be unchangeable in a grayed-out display mode. In addition, this screen is configured such that a text box for manually inputting an EMR ID and a function button MB3 for inputting an EMR ID by asking the medical institution server 50 for an EMR ID based on the patient information such as the patient name are displayed beneath the user account ID and the medical institution account ID.

When an EMR ID is inputted in the text box, and a function button MB4 for associating the inputted EMR ID with the user account ID displayed in the linkage destination EMR ID input region ARR is tapped, the medical institution server 50 executes a PHR-EMR account linkage process described later. Consequently, for example, the screen displayed on the medical institution terminal 30A is changed to an EMR linkage notification information display screen at the center of FIG. 38.

This screen is configured such that, for example, paired information is displayed as EMR linkage notification information. The paired information is composed of: information about the patient regarding whom PHR data has been shared and regarding whom a linkage with an electronic medical record has been performed; and patient information in the electronic medical record with which the linkage has been performed.

In addition, when the medical institution server 50 executes the PHR-EMR account linkage process described later, the screen displayed on the patient terminal 20A is changed to an EMR linkage notification information display screen on the right side of FIG. 38, for example. This screen is configured such that a medical record icon indicating that a linkage with the electronic medical record has been performed in the medical institution H.A is displayed. However, this screen is configured such that the EMR ID and the patient name in the medical record are not displayed. Consequently, in a case where the name, the sex, or the like in the electronic medical record differs from the name, the sex, or the like that the patient himself/herself has confirmed and registered to the PHR application, unnecessary confusion by the patient can be avoided by hiding the registered information in the electronic medical record from the patient.

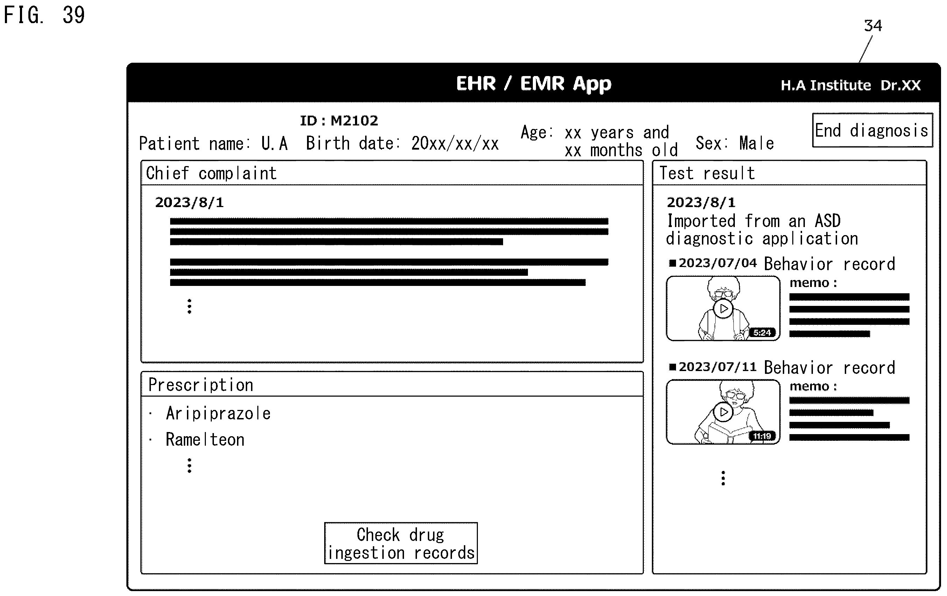

FIG. 39 is an example of an EMR data display screen in a case of referring to PHR data linked by using the EMR application. This screen is configured such that information (e.g., a patient name, an EMR ID, and the like) about a patient regarding whom EMR data is being viewed is displayed in an upper part of the display 34, for example. This screen is configured such that, beneath the information, a chief complaint record region and a prescription record region are displayed on a left pane, and a test result record region is displayed on a right pane, for example.

Since, for example, the ASD diagnostic application and the drug ingestion record application have been linked between the user U.A and the medical institution H.A, this screen is configured such that a "Check drug ingestion records" function button for referring to pieces of PHR data in the drug ingestion record application is displayed in the prescription record region. In addition, pieces of PHR data in the ASD diagnostic application are displayed in synchronization in the test result record region.

Alternatively, for example, a URI for referring to the PHR data may be displayed without displaying the PHR data on EMR data. In this case, for example, the medical institution terminal 30X may be able to refer to the PHR data based on the URI from the management server 10 when the URI is tapped.

### <Processes>

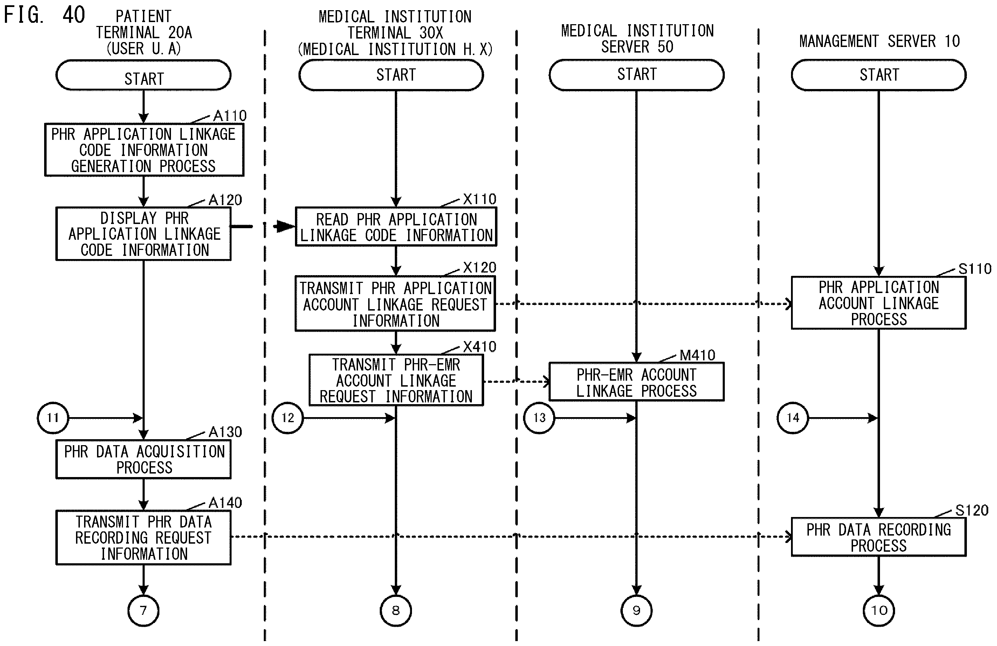

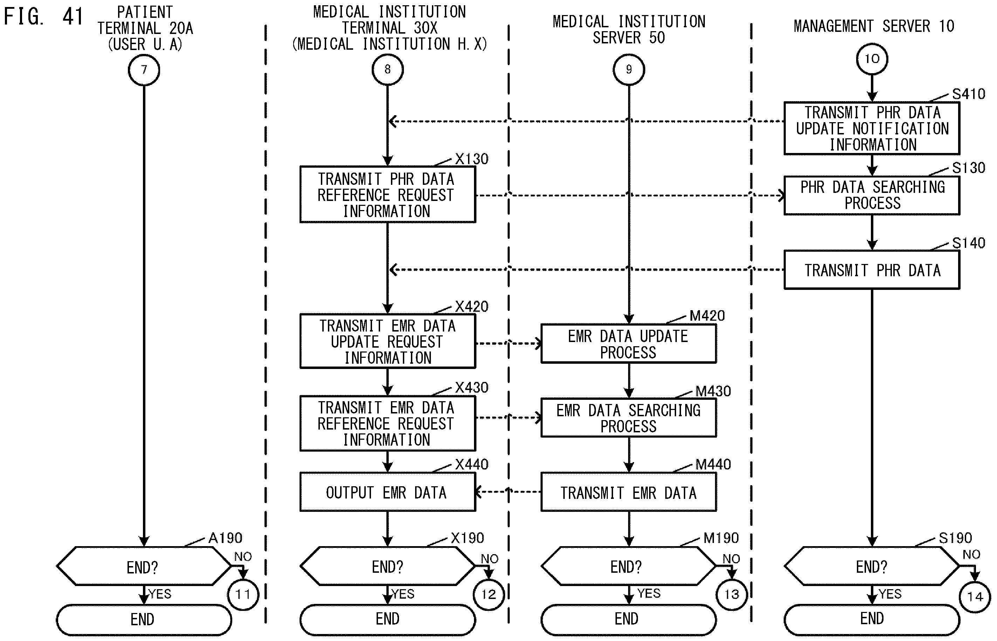

FIG. 40 and FIG. 41 are each a flowchart showing an example of the flow of processes to be executed by respective devices according to the present implementation example. Each of these drawings shows an example of a process to be executed by the controller 21 of the patient terminal 20A of the user U.A, an example of a process to be executed by the controller 31 of the medical institution terminal 30X in the medical institution H.X, an example of a process to be executed by the controller 51 of the medical institution server 50, and an example of a process to be executed by the controller 11 of the management server 10 in this order from the left side.

It is assumed that, before the processes in the present flowcharts are started, accounts of the user U.A and the medical institution H.X have already been registered (created) in the PHR platform, and PHR applications (e.g., ASD diagnostic applications) identified by the same PHR application ID have been installed on the patient terminal 20A and the medical institution terminal 30X. In addition, it is assumed that an EMR ID for storing patient information about the user U.A has been assigned in the medical institution server 50.

Hereinafter, description will be given with reference to FIG. 40.

When, for example, transmitting PHR application account linkage request information to the management server 10 (X120), the controller 31 of the medical institution terminal 30X transmits, to the medical institution server 50, PHR-EMR account linkage request information including the user account ID and the PHR application ID which are based on the PHR application linkage information, and an EMR ID corresponding to the user of the patient terminal 20A (X410). The transmission of the PHR-EMR account linkage request information may be executed by the PHR application controller 311. Alternatively, the transmission of the PHR-EMR account linkage request information may be executed by the EMR application controller 313 by using a plug-in for the EMR application, or the like.

The transmission of the PHR-EMR account linkage request information by the controller 31 of the medical institution terminal 30X may be performed after the controller 31 receives PHR linkage notification information from the management server 10. In addition, after transmitting the PHR-EMR account linkage request information, the controller 31 of the medical institution terminal 30X may transmit, to the management server 10, EMR linkage notification information indicating that the PHR data has been linked with the EMR application. When receiving the EMR linkage notification information, the controller 11 of the management server 10 may additionally store, based on the medical institution account ID, the user account ID, and the PHR application ID which are included in the EMR linkage notification information, EMR linkage flag information with respect to the corresponding medical institution account ID in the account ID-PHR application linkage management data 159, for example.

Alternatively, the PHR-EMR account linkage request information may include, instead of the EMR ID itself, information (e.g., a patient name, a birth date, or the like) that enables identification of the EMR ID. The PHR-EMR account linkage request information does not have to include the PHR application ID.

When receiving the PHR-EMR account linkage request information from the medical institution terminal 30X, the controller 51 of the medical institution server 50 executes a PHR-EMR account linkage process (M410). In the PHR-EMR account linkage process, the PHR-EMR linker 511 adds a record to the PHR-EMR association registered data 559 based on the PHR-EMR account linkage request information and stores the user account ID and the EMR ID in association with each other, for example. The PHR-EMR linker 511 may store, as a linked application ID, the linked PHR application ID in an associated manner.

When receiving the PHR-EMR account linkage request information, the PHR-EMR linker 511 may identify, based on the information that enables identification of the EMR ID, the EMR ID before executing the PHR-EMR account linkage process.

In addition, when executing the PHR-EMR account linkage process, the controller 51 of the medical institution server 50 may transmit, to the medical institution terminal 30X, EMR linkage notification information indicating that a linkage between the PHR data and EMR data has been performed, for example. When receiving the EMR linkage notification information from the medical institution server 50, the medical institution terminal 30X may output (e.g., display and output) the received EMR linkage notification information (see the center of FIG. 38). Then, the controller 31 of the medical institution terminal 30X may transmit the EMR linkage notification information to the patient terminal 20A via the management server 10. When receiving the EMR linkage notification information from the management server 10, the controller 21 of the patient terminal 20A may cause the received EMR linkage notification information to be outputted (e.g., displayed and outputted) (see the right side of FIG. 38).

Hereinafter, description will be given with reference to FIG. 41.

After executing the PHR data recording process (S120), the controller 11 of the management server 10 refers to the account ID-PHR application linkage management data 159 and transmits, based on the user account ID regarding which the PHR data has been updated, PHR data update notification information to each of medical institution terminals 30 having respective medical institution account IDs associated with the PHR application ID corresponding to the updated PHR data, for example (S410). Alternatively, the controller 11 of the management server 10 may transmit the PHR data update notification information to a medical institution terminal 30 having a medical institution account ID to which the EMR linkage flag information has been added in the account ID-PHR application linkage management data 159. The PHR data update notification information may include the PHR application ID and the user account ID regarding which the PHR data has been updated, and may further include the updated PHR data. Alternatively, the PHR data update notification information may include a URI for referring to the updated PHR data. The URI may include an access token that enables reference to the updated PHR data within a predetermined period in the PHR platform.

When receiving the PHR data update notification information from the management server 10, the controller 31 of the medical institution terminal 30X transmits PHR data reference request information to the management server 10, for example (X130). Then, the controller 31 of the medical institution terminal 30X receives the PHR data from the management server 10.

Here, when receiving the PHR data update notification information, the controller 31 of the medical institution terminal 30X may cause the display 34 to display and output the PHR data update notification information, for example. Then, the controller 31 of the medical institution terminal 30X may transmit the PHR data reference request information to the management server 10 based on user input. Alternatively, when receiving the PHR data update notification information, the controller 31 of the medical institution terminal 30X may automatically transmit the PHR data reference request information to the management server 10 based on the received PHR data update notification information.

When receiving the PHR data from the management server 10, the controller 31 of the medical institution terminal 30X transmits EMR data update request information to the medical institution server 50, for example (X420). The EMR data update request information may include the PHR data and the user account ID. The EMR data update request information may include the PHR application ID.

Alternatively, the EMR data update request information may include a URI for referring to the updated PHR data.

When receiving the EMR data update request information from the medical institution terminal 30X, the controller 51 of the medical institution server 50 executes an EMR data update process, for example (M420). The EMR data update process may be considered as a process for registering the PHR data to corresponding EMR data. In the EMR data update process, the controller 51 of the medical institution server 50 refers to the PHR-EMR association registered data 559 and searches for an EMR ID corresponding to the user account ID included in the EMR data update request information, for example. Then, the controller 51 of the medical institution server 50 additionally stores, based on the EMR ID having been searched for, the PHR data in the corresponding EMR data of the EMR patient information database 557, for example. Alternatively, the controller 51 of the medical institution server 50 may additionally store, in the EMR data, the URI for referring to the updated PHR data.

The controller 31 of the medical institution terminal 30X transmits EMR data reference request information to the medical institution server 50 based on user input, for example (X430). The EMR data reference request information may include, for example, an EMR ID of a patient regarding whom EMR data is to be referred to. Alternatively, the EMR data reference request information may include information (e.g., a patient name, a birth date, a user account ID, or the like) that enables identification of the EMR ID of the patient regarding whom EMR data is to be referred to.

When receiving the EMR data reference request information from the medical institution terminal 30X, the controller 51 of the medical institution server 50 executes an EMR data searching process (M430). In the EMR data searching process, the controller 51 of the medical institution server 50 refers to, based on the EMR ID included in the EMR data reference request information, the EMR patient information database 557 so as to read EMR data from EMR patient information management data identified with the EMR ID, for example. Alternatively, in the EMR data searching process, the controller 51 of the medical institution server 50 may refer to the EMR account registered data 555 or the PHR-EMR association registered data 559 so as to identify the EMR ID based on the information that enables identification of the EMR ID.

Then, the controller 51 of the medical institution server 50 transmits the read EHR data to the medical institution terminal 30X (M440).

When receiving the EMR data from the medical institution server 50, the controller 31 of the medical institution terminal 30X causes the display 34 to display and output the received EMR data, for example (X440).

In a case where the received EMR data includes a URI for referring to the PHR data, the controller 31 of the medical institution terminal 30X may receive the PHR data from the management server 10 based on the URI and cause the received PHR data to be displayed and outputted, for example.

### <Advantageous Effects of Second Implementation Example>

In the second implementation example, as shown in FIG. 36, FIG. 40 and FIG. 41, FIG. 37 and FIG. 38, and the like, an EMR ID can be managed in association with the user account ID, and the EMR ID is assigned to the patient in a medical institution server 50 that provides electronic medical record information.

In addition, in the second implementation example, as shown in FIG. 36, FIG. 40 and FIG. 41, FIG. 37 and FIG. 38, and the like, an EMR ID can be managed in association with the user account ID, and the EMR ID is assigned to the patient in a medical institution server 50 that provides electronic medical record information, whereby PHR data related to health of the patient can be shared also with the medical institution server 50 in addition to the management server 10, the PHR application corresponding to the patient, and the medical institution terminal 30.

In addition, in the second implementation example, as shown in FIG. 36, FIG. 40 and FIG. 41, FIG. 37 to FIG. 39, and the like, an EMR ID can be managed in association with the user account ID, the EMR ID is assigned to the patient in a medical institution server 50 that provides electronic medical record information, the PHR data and/or information related to the PHR data corresponding to the user account ID can be registered in association with the EMR ID to the medical institution server 50, and, in response to a request for providing the EMR data corresponding to the EMR ID, (i) the EMR data and (ii) the PHR data and/or the information related to the PHR data registered in association with the EMR ID can be provided to the medical institution terminal 30.

In the above implementation example, the PHR data is registered to the management server 10, the PHR data is provided from the management server 10 to the medical institution terminal 30, and the EMR data is updated. However, no limitation thereto is made. For example, the PHR data may be registered from the patient terminal 20 to a PHR application management server 60 which is a management server corresponding to the PHR application. Then, the management server 10 may ask the PHR application management server 60 for the PHR data registered to the PHR application management server 60, so that the management server 10 acquires the PHR data. Then, the management server 10 may transmit the acquired PHR data to the medical institution server 50 and the medical institution terminal 30. In this case, the medical institution terminal 30 may receive the PHR data via the medical institution server 50, and the medical institution server 50 may update the EMR data based on the received PHR data.

Alternatively, the transmission of the PHR data to the medical institution terminal 30 does not have to be performed via the management server 10 or the medical institution server 50. That is, the management server 10 may request the PHR application management server 60 to directly transmit the PHR data from the PHR application management server 60 to the medical institution terminal 30. The PHR application management server 60 having received the request may directly transmit the PHR data to the medical institution terminal 30. Also, the PHR application management server 60 may transmit the PHR data to the medical institution server 50, and the medical institution server 50 may update the EMR data based on the received PHR data.

The management server 10 may ask the patient terminal 20 for PHR data acquired by the patient terminal 20 and transmit the PHR data acquired from the patient terminal 20 to the medical institution terminal 30, for example. In addition, the management server 10 may transmit the PHR data to the medical institution server 50, and the medical institution server 50 may update the EMR data based on the received PHR data.

### <Modification (1) of Second Implementation Example>

In the above implementation example, PHR application linkage code information is transmitted from the patient terminal 20 to the medical institution terminal 30. However, no limitation thereto is made. For example, PHR application linkage code information presented on the medical institution terminal 30 may be read by the patient terminal 20.

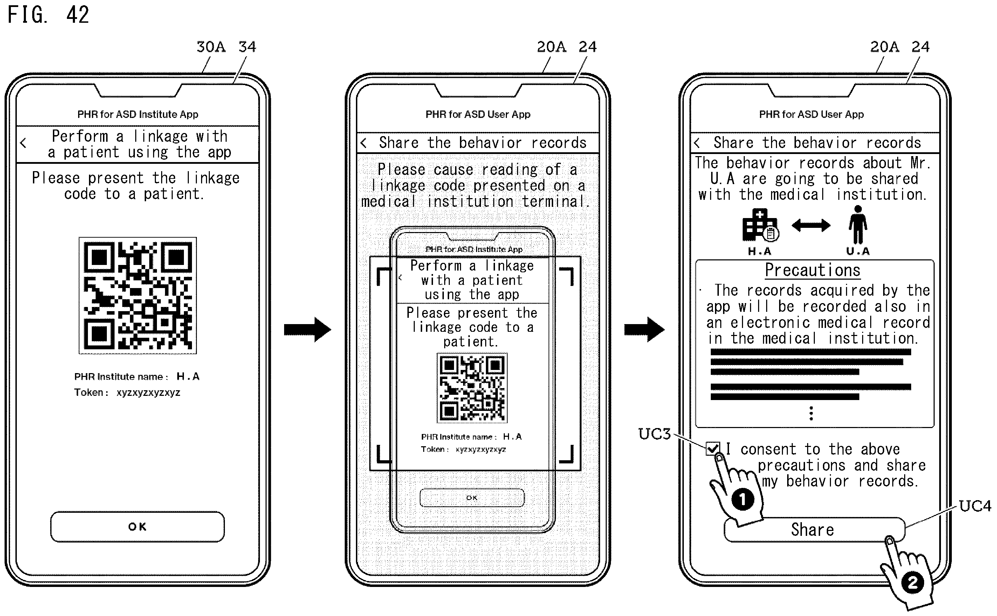

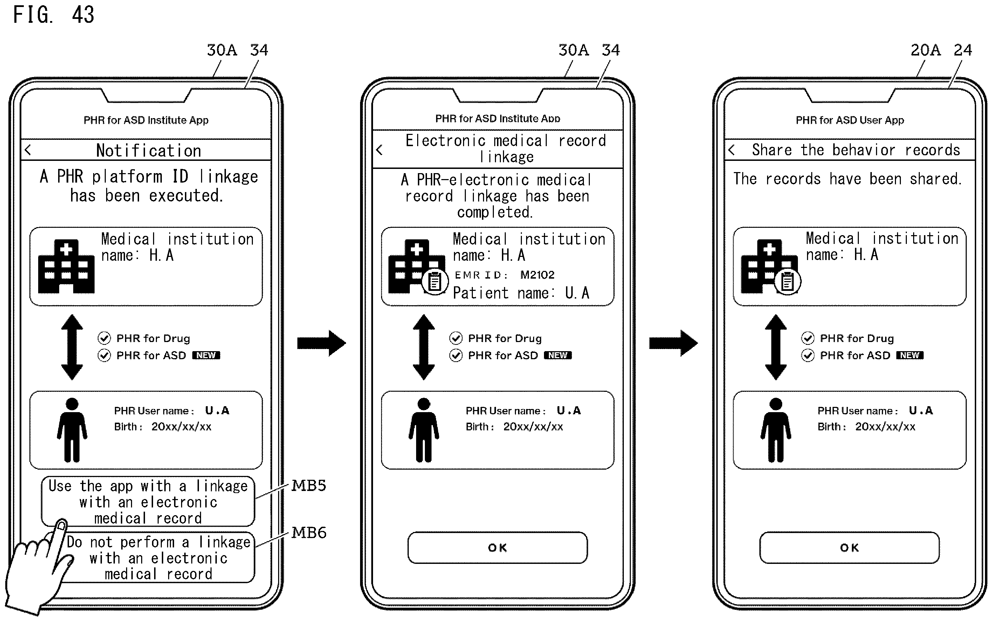

FIG. 42 and FIG. 43 show examples of screens displayed on the display 24 of the patient terminal 20A and the display 34 of the medical institution terminal 30A according to the present modification.

When, for example, the function button HM2 is tapped in the main menu screen of the medical-institution-side ASD diagnostic application on the left side of FIG. 23 on the medical institution terminal 30A, the screen displayed on the medical institution terminal 30A is changed to a PHR application linkage code information display screen on the left side of FIG. 42. This screen is configured such that, for example, an EMR ID token is displayed in addition to the PHR application linkage code information expressed by a two-dimensional code and the medical institution name in the PHR application. The EMR ID token is, for example, a token (e.g., character string information) related to an EMR ID in an electronic medical record corresponding to the patient having the patient terminal 20A, included in the PHR application linkage code information.

By using the EMR ID token, the user of the medical institution terminal 30A can assuredly relay the EMR ID that matches the user of the patient terminal 20A.

In a case where a validity period or an expiration date is set for the EMR ID token, the validity period or the expiration date of the EMR ID token may be displayed in the PHR application linkage code information display screen.

When, for example, the function button UM3 is tapped in the main menu screen of the patient-side ASD diagnostic application on the right side of FIG. 23 on the patient terminal 20A, the screen displayed on the patient terminal 20A is changed to a PHR application linkage code information reading screen at the center of FIG. 42. When the PHR application linkage code information is read from the medical institution terminal 30A, the screen displayed on the patient terminal 20A is changed to a PHR data sharing confirmation screen on the right side of FIG. 42.

In this screen, a notification indicating that the PHR data is to be linked with EMR data is displayed in a precaution field based on the fact that the EMR ID token is displayed in addition to the PHR application linkage code information, for example.

When, for example, the user taps the PHR data sharing function button UC4, the management server 10 executes the PHR application account linkage process. Consequently, the screen displayed on the medical institution terminal 30A is changed to a PHR linkage notification information display screen on the left side of FIG. 43. This screen is configured such that a function button MB5 for performing a linkage with the EMR ID based on the EMR ID token and a function button MB6 for performing only a PHR data linkage without performing any linkage with the electronic medical record are displayed.

When, for example, the user taps the function button MB5, the medical institution server 50 executes the PHR-EMR account linkage process, and the screen displayed on the medical institution terminal 30A is changed to an EMR linkage notification information display screen at the center of FIG. 43. In this screen, a notification indicating that an account linkage between the user account ID and the EMR ID has been automatically performed based on the EMR ID token is displayed.

In addition, when the medical institution server 50 executes the PHR-EMR account linkage process, the screen displayed on the patient terminal 20A is changed to an EMR linkage notification information display screen on the right side of FIG. 43, for example.

Alternatively, the PHR-EMR account linkage process may be executed by manually inputting the EMR ID on the medical institution terminal 30A without using the EMR ID token, unlike in this example.

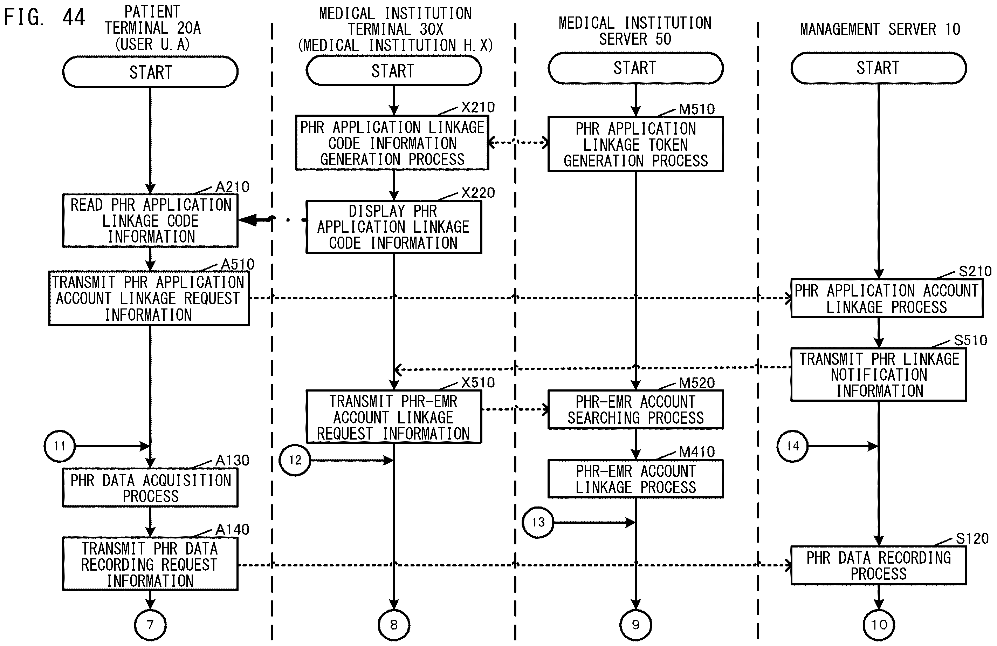

FIG. 44 is a flowchart showing an example of the flow of processes to be executed by the respective devices according to the present modification. This drawing shows an example of a process to be executed by the controller 21 of the patient terminal 20A of the user U.A, an example of a process to be executed by the controller 31 of the medical institution terminal 30X in the medical institution H.X, an example of a process to be executed by the controller 51 of the medical institution server 50, and an example of a process to be executed by the controller 11 of the management server 10 in this order from the left side.

First, the controller 31 of the medical institution terminal 30X executes a PHR application linkage code information generation process based on user input, for example (X210).

In the PHR application linkage code information generation process, the controller 31 of the medical institution terminal 30X transmits, to the medical institution server 50, EMR ID token request information including an EMR ID of a patient corresponding to the user of the patient terminal 20 to which the PHR application linkage code information is to be presented, for example.

When receiving the EMR ID token request information from the medical institution terminal 30X, the controller 51 of the medical institution server 50 executes a PHR application linkage token generation process, for example (M510). In the PHR application linkage token generation process, the controller 51 of the medical institution server 50 generates a token based on the received EMR ID, for example. Then, the controller 51 of the medical institution server 50 stores, in the data storage 55, the generated token and the EMR ID in association with each other. An expiration date or a validity period may be able to be set for the token. Then, the controller 51 of the medical institution server 50 transmits EMR ID token information including the generated token to the medical institution terminal 30X.

When receiving the EMR ID token information from the medical institution server 50, the controller 31 of the medical institution terminal 30X generates PHR application linkage code information based on PHR application linkage information including the token, and causes the PHR application linkage code information to be displayed and outputted, for example (X220).

The PHR application linkage information and the PHR application linkage code information may include the EMR ID instead of the token. Meanwhile, the PHR application linkage information and the PHR application linkage code information does not have to include the EMR ID or the token related to the EMR ID.

The controller 21 of the patient terminal 20A causes the imaging unit 27 to read the PHR application linkage code information displayed on the medical institution terminal 30X, and acquires the PHR application linkage information, for example (A210). Then, the controller 21 of the patient terminal 20A transmits PHR application account linkage request information including the medical institution account ID, the PHR application ID, the user account ID of the patient terminal 20A, and the token or the EMR ID to the management server 10 (A510).

When receiving the PHR application account linkage request information from the patient terminal 20A, the controller 11 of the management server 10 executes the PHR application account linkage process (S210). Then, the controller 11 of the management server 10 transmits PHR linkage notification information to the medical institution terminal 30X (S510). Here, in a case where the PHR application account linkage request information includes the token or the EMR ID, the controller 11 of the management server 10 transmits PHR linkage notification information including the token or the EMR ID to the medical institution terminal 30X. Since the management server 10 cannot decode the token, usage of the token makes it possible to prevent the EMR ID of the patient from leaking to the outside.

Then, the controller 31 of the medical institution terminal 30X transmits, to the medical institution server 50, PHR-EMR account linkage request information including the user account ID and the PHR application ID which are based on the PHR linkage notification information, and the EMR ID or the token corresponding to the user of the patient terminal 20A, for example (X510). In a case where the PHR linkage notification information does not include the EMR ID or the token, the controller 31 of the medical institution terminal 30X may acquire, based on user input, an EMR ID corresponding to the user of the patient terminal 20A or information that enables identification of the EMR ID, for example.

When receiving the PHR-EMR account linkage request information from the medical institution terminal 30X, the controller 51 of the medical institution server 50 executes a PHR-EMR account searching process, for example (M520). In the PHR-EMR account searching process, the controller 51 of the medical institution server 50 refers to tokens and EMR IDs stored during the PHR application linkage token generation process and searches for an EMR ID corresponding to the token included in the PHR-EMR account linkage request information, for example. After searching for the EMR ID, the controller 51 of the medical institution server 50 may delete the EMR ID having been searched for and the token from the data storage 55.

In a case where the PHR-EMR account linkage request information includes the EMR ID, the controller 51 of the medical institution server 50 does not have to execute the PHR-EMR account searching process.

### <Modification (2) of Second Implementation Example>

In the above implementation example, the PHR application linkage code information is transmitted from the patient terminal 20 to the medical institution terminal 30. However, no limitation thereto is made. For example, the PHR application account linkage process may be executed by selecting, on the patient terminal 20, a medical institution with which PHR data sharing is to be performed.

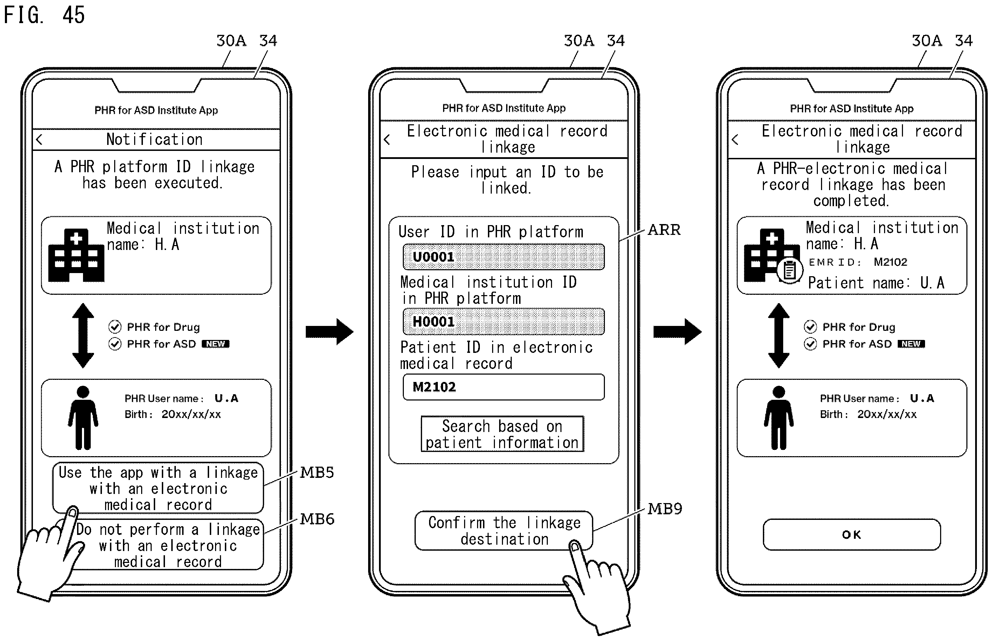

FIG. 45 shows an example of screens displayed on the display 34 of the medical institution terminal 30A according to the present modification. When, in the same manner as in FIG. 26 and FIG. 27, a medical institution with which a linkage is to be performed is selected on the patient terminal 20, and the management server 10 executes the PHR application account linkage process, a PHR linkage notification information display screen on the left side of FIG. 45 is displayed on the medical institution terminal 30A, for example. When the user taps the function button MB5 in this screen, the screen displayed on the medical institution terminal 30A is changed to a linkage destination EMR ID input screen at the center of FIG. 45, for example. Then, when a function button MB9 for associating an inputted EMR ID with the user account ID displayed in the linkage destination EMR ID input region ARR is tapped, the medical institution server 50 executes the PHR-EMR account linkage process. Consequently, the screen displayed on the medical institution terminal 30A is changed to an EMR linkage notification information display screen on the right side of FIG. 45, for example.

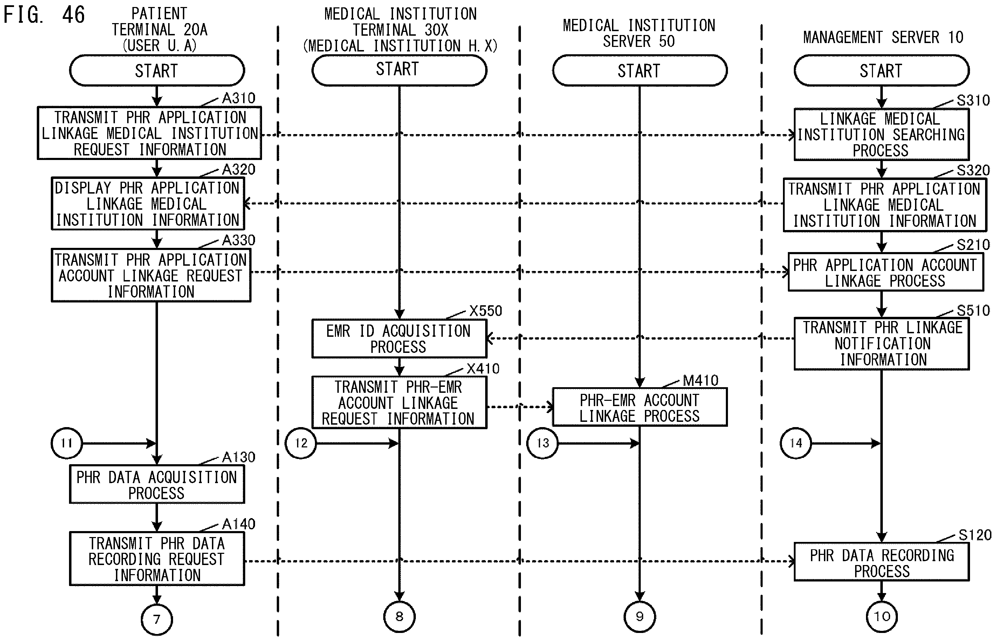

FIG. 46 is a flowchart showing an example of the flow of processes to be executed by the respective devices according to the present modification. This drawing shows an example of a process to be executed by the controller 21 of the patient terminal 20A of the user U.A, an example of a process to be executed by the controller 31 of the medical institution terminal 30X in the medical institution H.X, an example of a process to be executed by the controller 51 of the medical institution server 50, and an example of a process to be executed by the controller 11 of the management server 10 in this order from the left side.

When executing the PHR application account linkage process (S210), the controller 11 of the management server 10 transmits PHR linkage notification information to the medical institution terminal 30X, for example (S510). The PHR linkage notification information may include, for example, the PHR application ID and the user account ID of the patient terminal 20A with which a PHR data linkage has been performed.

When receiving the PHR linkage notification information from the management server 10, the controller 31 of the medical institution terminal 30X causes the display 34 to display and output the received PHR linkage notification information, for example. Then, the controller 31 of the medical institution terminal 30X executes an EMR ID acquisition process (X550). In the EMR ID acquisition process, the controller 31 of the medical institution terminal 30X receives, based on user input, an EMR ID corresponding to the user of the patient terminal 20A or information that enables identification of the EMR ID, for example.

Then, the controller 31 of the medical institution terminal 30X transmits, to the management server 10, PHR-EMR account linkage request information including the acquired EMR ID or the acquired information that enables identification of the EMR ID (X410).

### <Modification (3) of Second Implementation Example>

In the above implementation example, a linkage with the PHR platform is performed without extending data related to the EMR service. However, no limitation thereto is made. For example, a linkage with the PHR platform may be performed with an EMR patient information management database 557B obtained by extension.

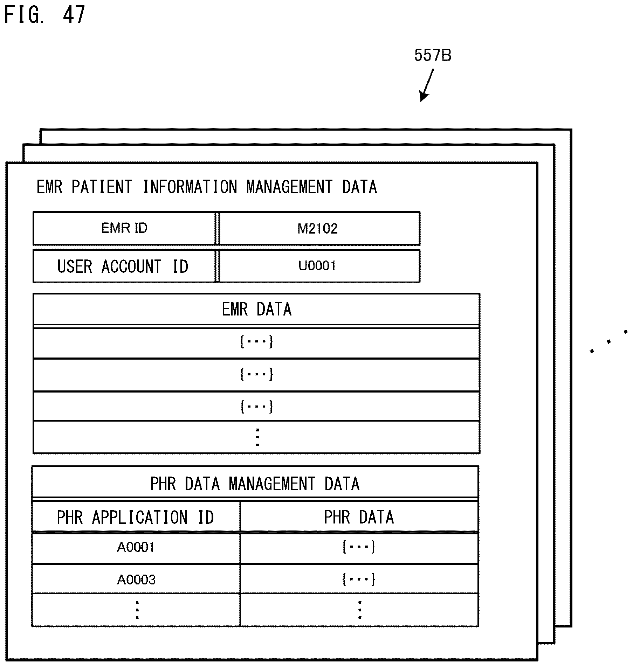

FIG. 47 shows the EMR patient information management database 557B having a data configuration which is another example of the data configuration of the EMR patient information database 557.

In the EMR patient information management database 557B, pieces of EMR patient information management data are stored as pieces of management data for the respective EMR IDs, for example. In each of the pieces of EMR patient information management data, the corresponding EMR ID, the corresponding user account ID, EMR data, and PHR data management data are stored in association with one another, for example.

In this case, the PHR-EMR association registered data 559 does not have to be used. Also, for example, in a case where an API for each PHR application is incorporated in the EMR application management process program 553, the PHR-EMR linkage process program 551 does not have to be used.

In the PHR-EMR account linkage process, the controller 51 of the medical institution server 50 may store an EMR ID and a user account ID in association with a corresponding piece of the EMR patient information management data, for example.

Also, in the EMR data update process, the controller 51 of the medical institution server 50 may cause PHR data in the EMR data update request information to be stored in association with the PHR data management data in a corresponding piece of the EMR patient information management data, for example.

### <Third Implementation Example>

A third implementation example is, for example, an implementation example related to a case where a patient is registered as a patient in an electronic medical record system of a medical institution but is not registered as a user in the PHR platform. The present implementation example may be considered as, for example, an implementation example in which a health professional in the medical institution provides a prescription to the patient through a PHR application.

The features described in the third implementation example are applicable to the other implementation examples and modifications in the same manner.

### <Display Screen>

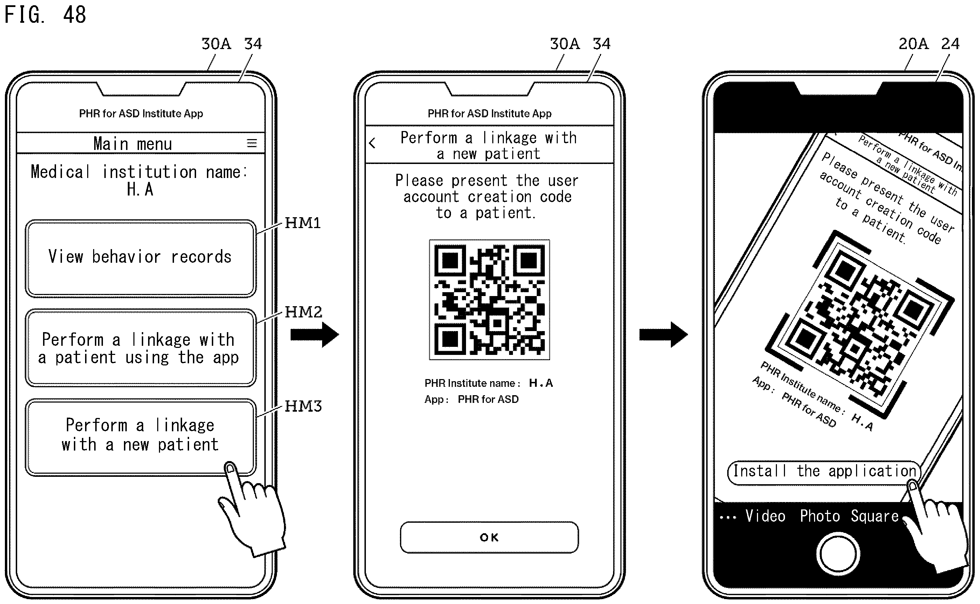

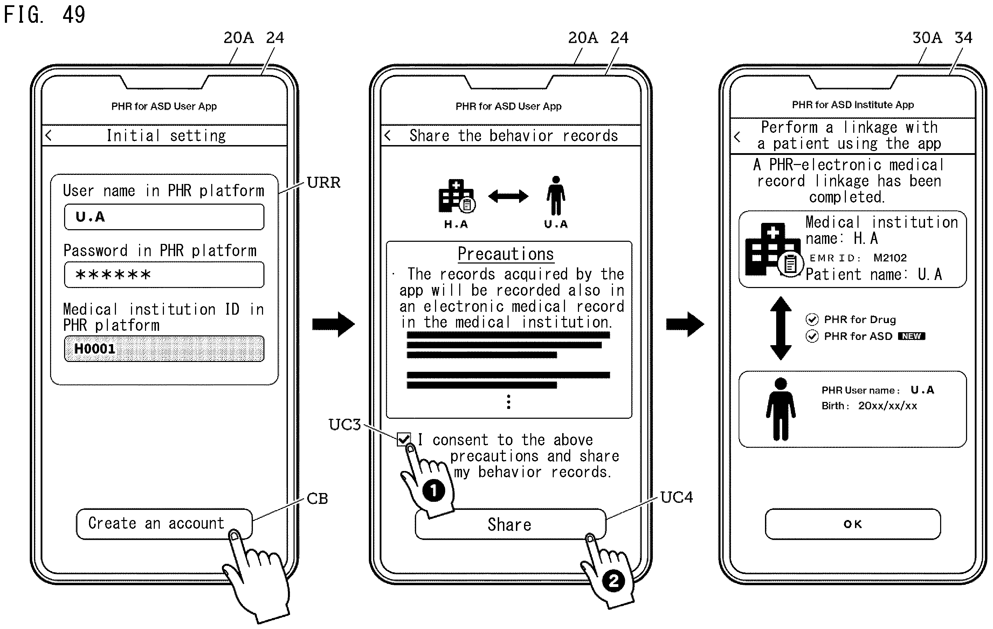

FIG. 48 and FIG. 49 show examples of screens displayed on the display 24 of the patient terminal 20A and the display 34 of the medical institution terminal 30A according to the present implementation example.

When, for example, the user taps the function button HM3 in the main menu screen of the medical-institution-side ASD diagnostic application on the left side of FIG. 48 on the medical institution terminal 30A, the screen displayed on the medical institution terminal 30A is changed to a PHR application user account creation asking information display screen at the center of FIG. 48.

This screen is configured such that, for example, PHR application user account creation asking information expressed by a two-dimensional code, the name of a PHR application that the user of the patient terminal 20A is prompted to install, and the name of the medical institution in which the medical institution terminal 30A is used are displayed, the names being included in PHR application user account creation asking information having yet to be encoded.

When, for example, a camera application is activated based on user input performed on the patient terminal 20A, the screen displayed on the patient terminal 20A is changed to a camera preview screen on the right side of FIG. 48. In this screen, for example, the two-dimensional code indicating the PHR application user account creation asking information is shown within a preview screen for an image to be acquired by the imaging unit 27, whereby the two-dimensional code is decoded, and an "Install the application" button which is a link to a URI included in the information obtained by the decoding is displayed. When the user taps the "Install the application" button, the application (in this example, the ASD diagnostic application) specified in the PHR application user account creation asking information is installed on the patient terminal 20A, for example.

The screen on the left side of FIG. 49 is an example of a PHR platform user registration screen in the ASD diagnostic application displayed on the patient terminal 20A after the application is installed. This screen is configured such that, for example, text boxes for inputting a user name and a password are displayed in a PHR platform user registration information input region URR. In addition, beneath the text boxes, a medical institution account ID regarding which a linkage is to be performed with a user account ID to be created based on the information inputted to the text boxes is automatically inputted based on the PHR application user account creation asking information and is set in a grayed-out display mode to be unchangeable. In the PHR platform user registration information input region URR, various types of user information such as a birthday and a sex may be able to be inputted.

When a user name and a password are inputted to the text boxes, and an account creation function button CB for creating a PHR platform user account is tapped, the management server 10 assigns a user account ID to the user of the patient terminal 20A and updates the PHR medical institution account registered data 155.

Consequently, the screen displayed on the patient terminal 20A is changed to a PHR data sharing confirmation screen at the center of FIG. 49, for example.

In this screen, a notification indicating that the PHR data is to be linked with EMR data is displayed in a precaution field based on the fact that the EMR ID token is displayed in addition to the PHR application user account creation asking information, for example.

When, for example, the user taps the PHR data sharing function button UC4, the management server 10 executes the PHR application account linkage process, and the medical institution server 50 executes the PHR-EMR account linkage process. Consequently, an EMR linkage notification information display screen on the right side of FIG. 49 is displayed on the medical institution terminal 30A.

Alternatively, registration of a user account in the PHR platform may be performed through the PHR integration application or a web app in the PHR platform service provided as SaaS, unlike in this example.

### <Processes>

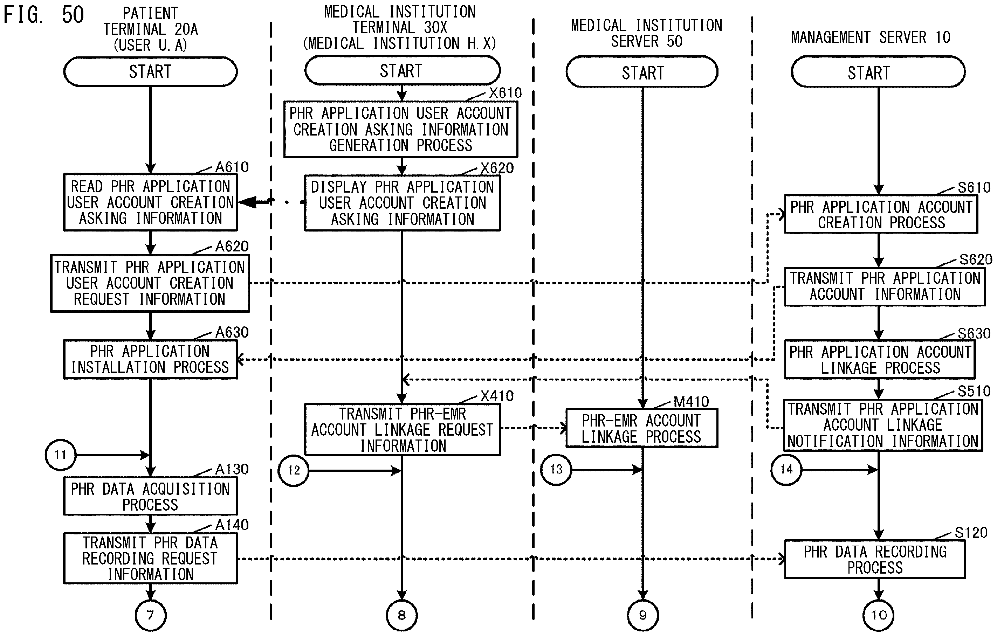

FIG. 50 is a flowchart showing an example of the flow of processes to be executed by the respective devices according to the present implementation example. This drawing shows an example of a process to be executed by the controller 21 of the patient terminal 20A of the user U.A, an example of a process to be executed by the controller 31 of the medical institution terminal 30X in the medical institution H.X, an example of a process to be executed by the controller 51 of the medical institution server 50, and an example of a process to be executed by the controller 11 of the management server 10 in this order from the left side.

It is assumed that, before the processes in the present flowchart are started, an account of the medical institution H.X has been registered (created) in the PHR platform, and a PHR application (e.g., the ASD diagnostic application) identified with a predetermined PHR application ID has been installed on the medical institution terminal 30X. In addition, it is assumed that an EMR ID for storing patient information about the user U.A has been assigned in the medical institution server 50.

First, the controller 31 of the medical institution terminal 30X executes a PHR application user account creation asking information generation process based on user input, for example (X610). In the PHR application user account creation asking information generation process, the controller 31 of the medical institution terminal 30X generates PHR application user account creation asking information including the medical institution account ID of the medical institution terminal 30X and the PHR application ID of the PHR application that the patient is to be requested to install, for example. The PHR application user account creation asking information may include, for example, installation guidance information (e.g., a URI to an app store or a download site) for installing the predetermined PHR application and account registration guidance information (e.g., a URI to an account creation page that is managed by the management server 10) for registering the user to the PHR platform. The PHR application user account creation asking information may be encoded as, for example, one-dimensional code or two-dimensional code information. The PHR application user account creation asking information may include information (e.g., a medical institution name or the like) for identifying the medical institution and an EMR ID corresponding to the user of the patient terminal 20A.

As the PHR application that the patient is to be requested to install, any application registered to the PHR application registered data 157 may be able to be selected based on user input performed on the medical institution terminal 30X, for example.

The medical institution terminal 30X and the medical institution server 50 may, in the same manner as in FIG. 44, generate an EMR ID token and cause the EMR ID token to be included in the PHR application user account creation asking information, for example.

When generating the PHR application user account creation asking information which is, for example, two-dimensional code information, the controller 31 of the medical institution terminal 30X causes the display 34 to display and output the PHR application user account creation asking information (X620). Alternatively, the controller 31 of the medical institution terminal 30X may achieve the relaying by transmitting the PHR application user account creation asking information to the patient terminal 20A through P2P communication by utilizing short-range wireless communication according to Bluetooth, NFC, or the like, for example.

The controller 21 of the patient terminal 20A causes, based on user input, the imaging unit 27 to read the PHR application user account creation asking information displayed on the medical institution terminal 30X, for example (A610). Consequently, the controller 21 of the patient terminal 20A may receive, based on the account registration guidance information, information (e.g., a user name, a password, and the like) necessary for registering a user account in the PHR platform, for example.

Alternatively, for example, the controller 21 of the patient terminal 20A may receive the PHR application user account creation asking information by receiving, through user input, the medical institution account ID, the PHR application ID, or the like displayed on the medical institution terminal 30X.

Then, the controller 21 of the patient terminal 20A transmits, to the management server 10, PHR application user account creation request information including the information necessary for registering a user account, the medical institution account ID of the medical institution terminal 30X, and the PHR application ID, for example (A620). The PHR application user account creation request information may include an EMR ID token or an EMR ID.

When receiving the PHR application user account creation request information from the patient terminal 20A, the controller 11 of the management server 10 executes a PHR application account creation process, for example (S610). In the PHR application account creation process, the controller 11 of the management server 10 additionally stores, based on the received PHR application user account creation request information, a new user-related record in the PHR user account registered data 153, for example.

Then, the controller 11 of the management server 10 transmits, to the patient terminal 20A, PHR application account information including a user account ID assigned to the user of the patient terminal 20A, for example (S620). The PHR application account information may include installation guidance information.

Then, the controller 11 of the management server 10 executes a PHR application account linkage process, for example (S630). In the PHR application account linkage process, the controller 11 of the management server 10 associates the user account ID with the medical institution account ID and the PHR application ID in the same manner as in step S210 in FIG. 44, for example. The user account ID has been assigned to the user of the patient terminal 20A, and the medical institution account ID and the PHR application ID are based on the PHR application user account creation request information.

When receiving the PHR application account information from the management server 10, the controller 21 of the patient terminal 20A executes a PHR application installation process, for example (A630). In the PHR application installation process, the controller 21 of the patient terminal 20A installs the predetermined PHR application based on the installation guidance information and executes the PHR application by using the user account ID received in a state of being included in the PHR application account information, for example.

Alternatively, when receiving the PHR application user account creation asking information, the controller 21 of the patient terminal 20A may execute the PHR application installation process and acquire, in the PHR application activated in a state where user registration has not been performed, the information necessary for registering a user account in the PHR platform, for example. Thereafter, the controller 21 of the patient terminal 20A may transmit the PHR application user account creation request information to the management server 10, for example.

### <Advantageous Effects of Third Implementation Example>

In the third implementation example, as shown in FIG. 48, FIG. 50, and the like, the user account ID is created in response to a patient terminal 20 obtaining the medical institution account ID from the medical institution terminal 30, and the PHR application associated with the medical institution account ID is installed on the patient terminal 20. Thus, in the medical institution, the patient uses his/her own patient terminal 20 to acquire the medical institution account ID from the medical institution terminal 30, whereby the patient can easily use a PHR application that the medical institution wants the patient to use.

In addition, in the third implementation example, as shown in FIG. 49, FIG. 50, and the like, the user account ID is managed in association with the EMR ID based on a consent of the patient. Thus, after a PHR application is installed on the patient terminal 20 in the medical institution, a medical worker in the medical institution can check, through the electronic medical record system, PHR data acquired by the PHR application.

### <Modification (1) of Third Implementation Example>

In the above implementation example, the PHR application to be installed on the patient terminal 20 (the PHR application through which a prescription is to be provided to the patient from the medical institution) is specified on the medical institution terminal 30. However, no limitation thereto is made. For example, the PHR application user account creation asking information does not have to include any PHR application ID.

In this case, the controller 11 of the management server 10 may cause information about a list of PHR applications to be included in the PHR application account information, for example. Then, the controller 21 of the patient terminal 20A may collectively install, based on user input, one or more desired PHR applications out of the information about the list of PHR applications on the patient terminal 20A, for example.

In addition, the controller 21 of the patient terminal 20A may specify a PHR application ID of a PHR application through which a linkage with the medical institution terminal 30 is to be performed among the installed PHR applications. Then, the controller 21 may transmit PHR application account linkage request information including the specified PHR application ID to the management server 10. When receiving the PHR application account linkage request information from the patient terminal 20A, the controller 11 of the management server 10 may execute the PHR application account linkage process according to the specified PHR application ID.

Alternatively, the PHR application user account creation asking information may include a plurality of PHR application IDs of PHR applications that can be installed on the patient terminal 20. In this case, the PHR application user account creation request information may include PHR application IDs arbitrarily selected by the user of the patient terminal 20. In this case, the controller 11 of the management server 10 may cause installation guidance information based on the arbitrarily-selected PHR application IDs to be included in the PHR application account information, for example. The controller 11 of the management server 10 may execute the PHR application account linkage process on each of the PHR application IDs selected by the user of the patient terminal 20, for example.

### <Modification (2) of Third Implementation Example>

In the above implementation example, a case where the user of the patient terminal 20A has not registered any account in the PHR platform has been presented as an example. However, no limitation thereto is made. For example, there may be a case where: the user of the patient terminal 20A has been registered as a user in the PHR platform (possesses a user account ID); but the user of the medical institution terminal 30 has not yet installed the PHR application that the patient is to be requested to install.

In this case, the controller 21 of the patient terminal 20A may execute the PHR application installation process upon reading of the PHR application user account creation asking information, for example. Then, the controller 21 of the patient terminal 20A may transmit, to the management server 10, PHR application account linkage request information including the medical institution account ID and the PHR application ID which are based on the PHR application user account creation asking information, and the user account ID, for example.

### <Fourth Implementation Example>

A fourth implementation example is, for example, an implementation example in which the linkage between the user account ID corresponding to the patient and the medical institution account ID is canceled.

The features described in the fourth implementation example are applicable to the other implementation examples and modifications in the same manner.

### <Display Screen>

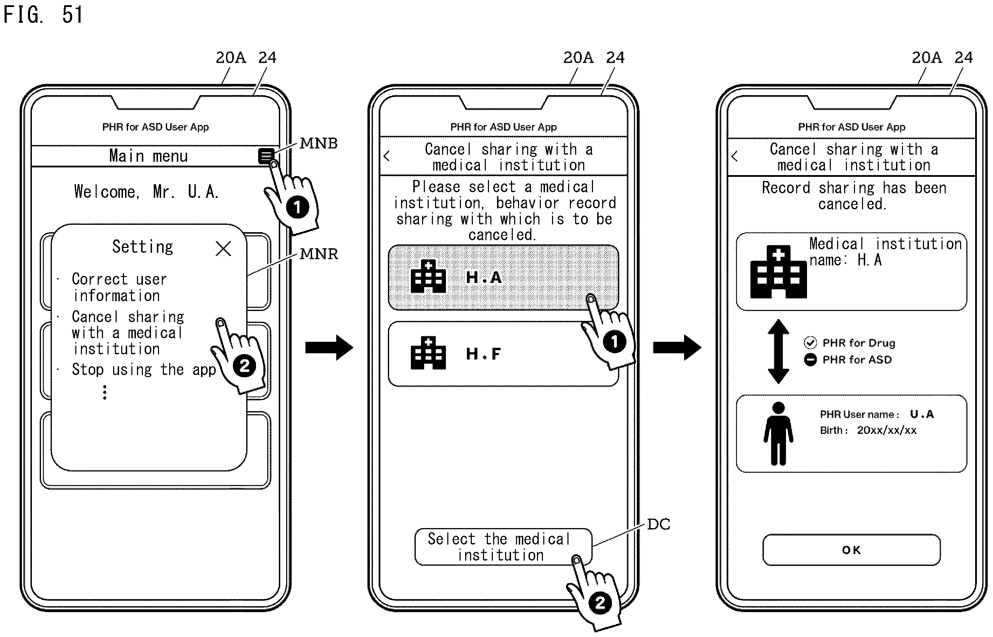

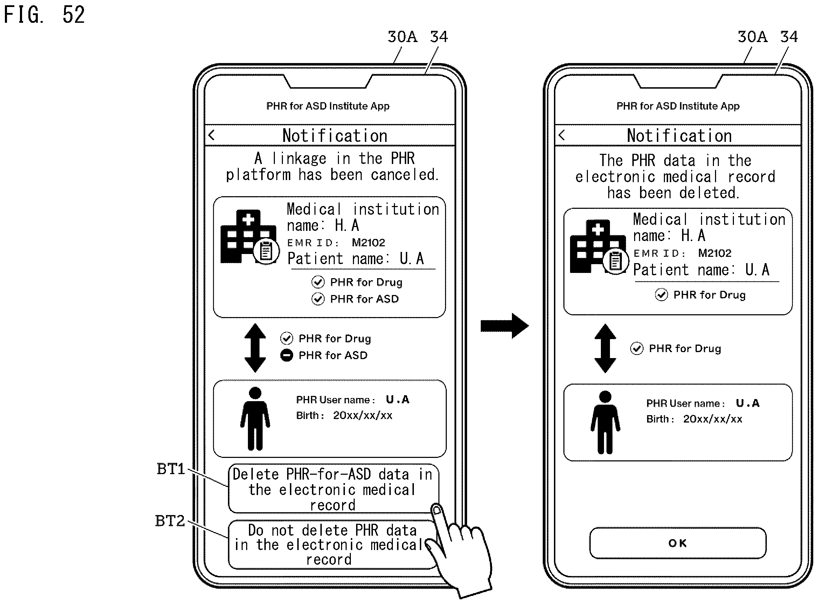

FIG. 51 and FIG. 52 show examples of screens displayed on the display 24 of the patient terminal 20A and the display 34 of the medical institution terminal 30A according to the present implementation example.

A configuration is employed in which a PHR application setting menu region MNR is displayed when the user taps a setting function button MNB in the main menu screen of the patient-side ASD diagnostic application on the left side of FIG. 51 on the patient terminal 20A, for example. When, for example, the user taps an item for canceling a PHR data linkage with a medical institution in the PHR application setting menu region MNR, the screen displayed on the patient terminal 20A is changed to a screen to display PHR application-linked medical institution list information at the center of FIG. 51.

This screen is configured such that, for example, medical institutions in which PHR data in the ASD diagnostic application is shared with the user U.A are listed. When, for example, the medical institution H.A is tapped, the medical institution H.A is highlighted so that the user U.A ascertains that the selection has been made. When, for example, the medical institution H.A is selected and the user taps a function button DC for canceling the corresponding PHR application linkage, the management server 10 executes a PHR application linkage cancellation process described later. Consequently, the screen displayed on the patient terminal 20A is changed to a PHR application account linkage cancellation notification information display screen on the right side of FIG. 51, for example.

In this screen, it is indicated that the linkage in the ASD diagnostic application out of the PHR applications through which linkages have been performed between the medical institution H.A and the patient U.A has been canceled. This indication is made by an icon on the left side of the name of the application. In addition, it is indicated that the linkage in the drug ingestion record application is maintained.

In addition, when, for example, the management server 10 executes the PHR application linkage cancellation process, a PHR application account linkage cancellation notification information display screen on the left side of FIG. 52 is displayed on the medical institution terminal 30A. In this screen, it is indicated that the linkage in the ASD diagnostic application with the user U.A has been canceled, but each of PHR data obtained through the ASD diagnostic application and PHR data obtained through the drug ingestion management application is recorded in the corresponding EMR data in the medical institution server 50.

The screen is configured such that, for example, a function button BT1 and a function button BT2 are displayed on the lower side of the screen. The function button BT1 is a button with which the PHR data that has been obtained through the ASD diagnostic application and that has been recorded in the corresponding EMR data is deleted from said EMR data. The function button BT2 is for canceling said linkage without deleting the PHR data that has been obtained through the ASD diagnostic application and that has been recorded in the corresponding EMR data.

When, for example, the user taps the function button BT1, the medical institution server 50 executes a PHR-EMR account linkage cancellation process described later. Consequently, for example, a PHR-EMR account linkage cancellation notification information display screen on the right side of FIG. 52 showing the medical institution terminal 30A is displayed on the medical institution terminal 30A. In this screen, it is indicated that the PHR data obtained through the ASD diagnostic application has been deleted from the EMR data. In addition, in this screen, it is indicated that: the linkage in the drug ingestion record application is maintained between the medical institution H.A and the user U.A; and the PHR data obtained through the drug ingestion record application is recorded in the corresponding EMR data.

### <Processes>

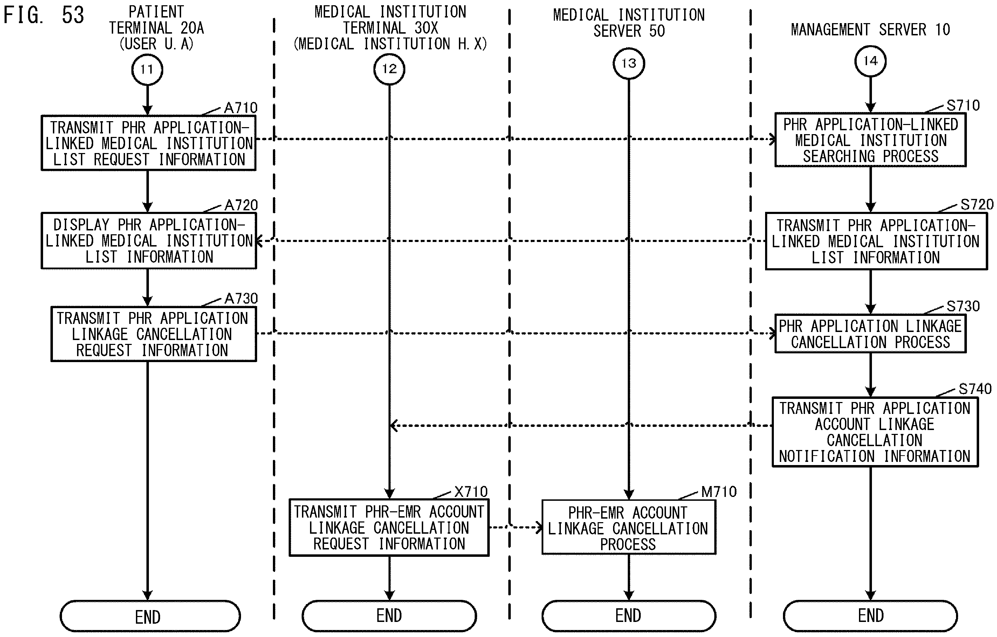

FIG. 53 is a flowchart showing an example of the flow of processes to be executed by the respective devices according to the present implementation example. This drawing shows an example of a process to be executed by the controller 21 of the patient terminal 20A of the user U.A, an example of a process to be executed by the controller 31 of the medical institution terminal 30X in the medical institution H.X, an example of a process to be executed by the controller 51 of the medical institution server 50, and an example of a process to be executed by the controller 11 of the management server 10 in this order from the left side.

It is assumed that, before the processes in the present flowchart are started, a linkage between the user U.A and the medical institution H.X has been performed in the PHR platform according to the processes in FIG. 40 and FIG. 41 so that data sharing is performed between PHR data and the electronic medical record service, for example.

The controller 21 of the patient terminal 20A transmits, based on user input, PHR application-linked medical institution list request information to the management server 10, for example (A710). The PHR application-linked medical institution list request information is for requesting a list of medical institutions in which linkages with the user account ID of the patient terminal 20A have been performed. The PHR application-linked medical institution list request information may include, for example, a PHR application ID specified by the user of the patient terminal 20A.

When receiving the PHR application-linked medical institution list request information from the patient terminal 20A, the controller 11 of the management server 10 executes a PHR application-linked medical institution searching process, for example (S710). In the PHR application-linked medical institution searching process, the controller 11 of the management server 10 refers to the account ID-PHR application linkage management data 159 and searches for linked medical institution account IDs for each of the PHR application IDs associated with the user account ID of the patient terminal 20A, for example.

Then, the controller 11 of the management server 10 transmits, to the patient terminal 20A, PHR application-linked medical institution list information including a list of the medical institution account IDs having been searched for. The PHR application-linked medical institution list information may include a medical institution account ID linked with the PHR application ID specified in the PHR application-linked medical institution list request information.

When receiving the PHR application-linked medical institution list information from the management server 10, the controller 21 of the patient terminal 20A causes the display 24 to display and output the received PHR application-linked medical institution list information, for example (A720).

When, for example, a medical institution the linkage with which is to be canceled is selected based on user input performed on the displayed PHR application-linked medical institution list information, the controller 21 of the patient terminal 20A transmits, to the management server 10, PHR application linkage cancellation request information including the user account ID of the patient terminal 20A and the selected medical institution account ID (A730).

The PHR application linkage cancellation request information may include, for example, the PHR application ID of the PHR application specified by the user of the patient terminal 20 such that the linkage in the PHR application is to be canceled. Alternatively, linkages with a certain medical institution account ID may be able to be collectively canceled without specifying any PHR application ID.

When receiving the PHR application linkage cancellation request information from the patient terminal 20A, the controller 11 of the management server 10 executes a PHR application linkage cancellation process, for example (S730). In the PHR application linkage cancellation process, the controller 11 of the management server 10 deletes the medical institution account ID in the account ID-PHR application linkage management data 159 based on the received PHR application linkage cancellation request information, for example.

Then, the controller 11 of the management server 10 transmits, to the medical institution terminal 30X having the deleted medical institution account ID, PHR application account linkage cancellation notification information indicating that the linkage in a PHR application has been canceled, for example (S740). The PHR application account linkage cancellation notification information may include the PHR application ID of the PHR application the linkage in which has been canceled. In addition, the controller 11 of the management server 10 may transmit the PHR application account linkage cancellation notification information to the patient terminal 20A.

When receiving the PHR application account linkage cancellation notification information from the management server 10, the controller 31 of the medical institution terminal 30X transmits, to the medical institution server 50, PHR-EMR account linkage cancellation request information including the user account ID and the PHR application ID which are included in the PHR application account linkage cancellation notification information, for example (X710). The PHR-EMR account linkage cancellation request information may include, for example, PHR-data-in-EMR-data deletion request information for deleting the relevant PHR data stored in the corresponding EMR data. Here, the transmission of the PHR-EMR account linkage cancellation request information by the controller 31 of the medical institution terminal 30X may be automatically performed upon receiving the PHR application account linkage cancellation notification information from the management server 10. Alternatively, the transmission of the PHR-EMR account linkage cancellation request information by the controller 31 of the medical institution terminal 30X may be performed based on, for example, user input performed with the PHR application account linkage cancellation notification information being outputted.

When receiving the PHR-EMR account linkage cancellation request information from the medical institution terminal 30X, the controller 51 of the medical institution server 50 executes a PHR-EMR account linkage cancellation process, for example (M710). In the PHR-EMR account linkage cancellation process, the controller 51 of the medical institution server 50 refers to the PHR-EMR association registered data 559 and deletes the unlinked PHR application ID from the linked application IDs corresponding to the user account ID specified in the PHR-EMR account linkage cancellation request information, for example. When, for example, determining that all of linked application IDs corresponding to a certain user account ID have been deleted or linkages for a certain user account ID have been collectively canceled, the controller 51 of the medical institution server 50 may delete a record in which the user account ID and an EMR ID have been associated with each other.

When determining that the PHR-EMR account linkage cancellation request information includes the PHR-data-in-EMR-data deletion request information, the controller 51 of the medical institution server 50 may refer to the EMR patient information database 557 and delete the PHR data from the EMR data corresponding to the EMR ID associated with the user account ID specified in the PHR-EMR account linkage cancellation request information, for example.

In addition, after executing the PHR-EMR account linkage cancellation process, the controller 51 of the medical institution server 50 may transmit PHR-EMR account linkage cancellation notification information to the medical institution terminal 30X.

Although the electronic medical record service in the medical institution server 50 is assumed to be provided in the present flowchart, no limitation thereto is made. In consideration of the first implementation example, the PHR account linkage cancellation process can be executed in the same manner in the case of absence of the medical institution server 50 as well.

### <Advantageous Effects of Fourth Implementation Example>

In the fourth implementation example, as shown in FIG. 51, FIG. 53, and the like, the linkage between the user account ID and the medical institution account ID is canceled in response to an operation of the patient terminal 20 by the patient. Consequently, in a case where, for example, the patient desires to stop sharing PHR data with the medical institution or change the medical institution with which the patient thinks that PHR data may be shared, the patient can stop such sharing by operating his/her own terminal.

In addition, in the fourth implementation example, as shown in FIG. 52, FIG. 53, and the like, the PHR data is deleted in response to an operation of the medical institution terminal 30 by a medical worker, said PHR data having been acquired by the PHR application and having been managed in association with an EMR ID which is assigned to the patient in a medical institution server 50 that provides electronic medical record information. Consequently, in the medical institution, data management can be performed while the will of the patient not to share PHR data is respected.

### <Modification (1) of Fourth Implementation Example>

In the above implementation example, a PHR application linkage is canceled by the patient terminal 20. However, no limitation thereto is made. For example, a PHR application linkage may be canceled by the medical institution terminal 30.

In a case where, for example, a PHR application linkage is canceled based on user input performed on the medical institution terminal 30, the controller 31 of the medical institution terminal 30X may transmit, to the management server 10, PHR application linkage cancellation request information including a PHR application ID and a user account ID regarding which a linkage is to be canceled.

Alternatively, the transmission of the PHR application linkage cancellation request information to the management server 10 by the controller 31 of the medical institution terminal 30X may be automatically performed when a predetermined condition is satisfied. Here, the predetermined condition may be:
(C1) a condition that
   the patient has not seen a doctor for at least a predetermined period (e.g., one year) in the medical institution in which the medical institution terminal 30X is used, or
   an electronic medical record based on a certain EMR ID has not been edited or viewed for at least the predetermined period; or
(C2) a condition that PHR data has not been updated for at least a predetermined period (e.g., half a year) in a PHR application through which the PHR data has been linked.

In one example, in the case of the above condition (C1), the PHR application linkage cancellation request information may include request information for canceling linkages in all the PHR applications.

In another example, in the case of the above condition (C2), the PHR application linkage cancellation request information may include a PHR application ID related to the PHR data that has not been updated.

In this manner, the linkage between the user account ID and the medical institution account ID is automatically canceled according to a predetermined condition, whereby data management can be made efficient.

### <Fifth Implementation Example>

A fifth implementation example is, for example, an implementation example related to storing, in the management server 10, PHR data as privacy-related information requiring attention in handling in a state where the PHR data is encrypted.

The features described in the fifth implementation example are applicable to the other implementation examples and modifications in the same manner.

### <System Configuration>

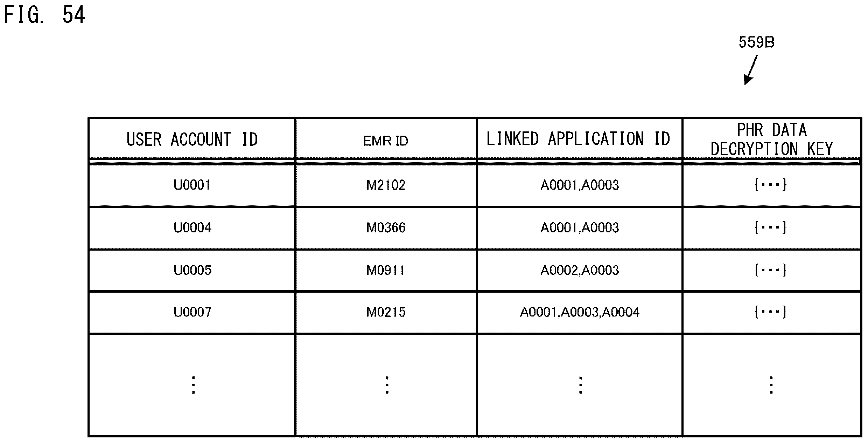

FIG. 54 shows PHR-EMR association registered data 559B which is another example of the PHR-EMR association registered data 559, according to the present implementation example. In the PHR-EMR association registered data 559B, PHR data decryption keys are stored in an associated manner in addition to the user account IDs, the EMR IDs, and the linked application IDs, for example.

Each of the PHR data decryption keys is a decryption key for decrypting the corresponding PHR data having been encrypted (referred to as "encrypted PHR data"). An encryption scheme for the PHR data may be, for example, a symmetric-key encryption scheme or a public-key encryption scheme. In the case of the symmetric-key encryption scheme, the PHR data decryption key may be considered as a secret key associated with the corresponding user account ID. Meanwhile, in the case of the public-key encryption scheme, the PHR data decryption key may be considered as a public key associated with the corresponding user account ID. The decryption key may differ among the PHR application IDs.

### <Processes>

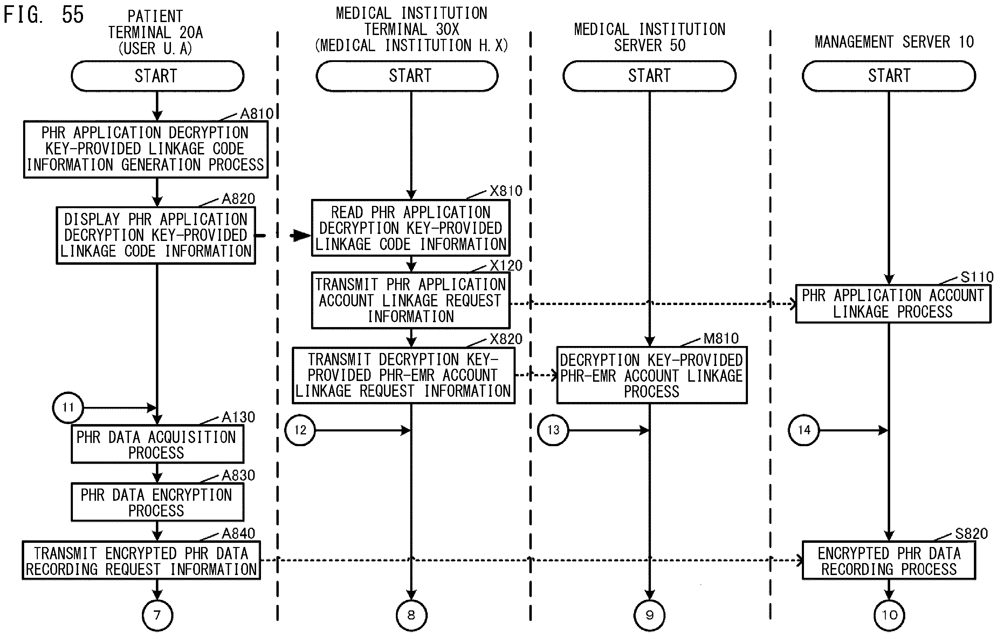

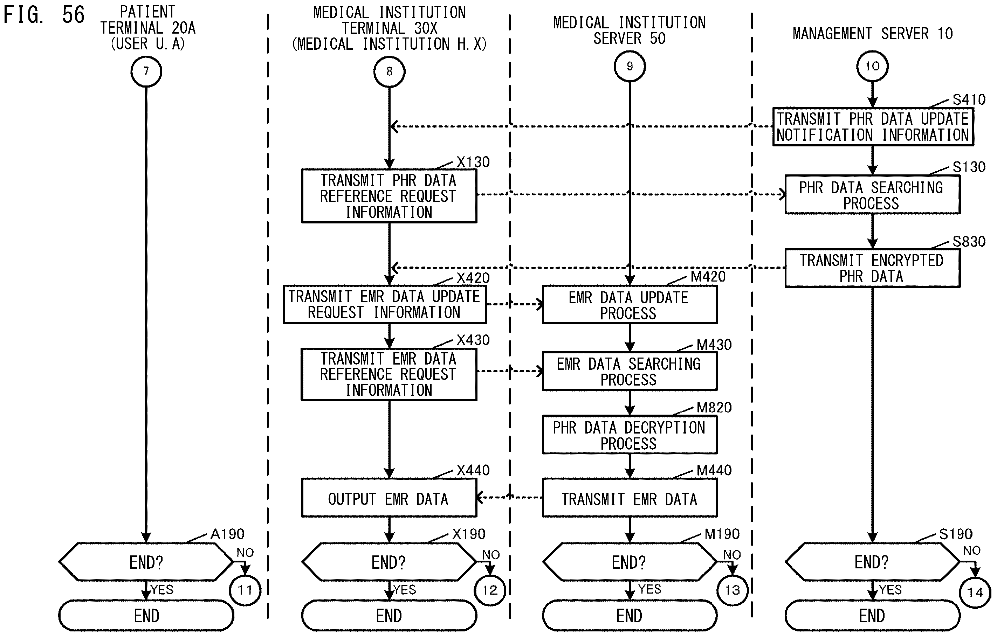

FIG. 55 and FIG. 56 are each a flowchart showing an example of the flow of processes to be executed by the respective devices according to the present implementation example. Each of these drawings shows an example of a process to be executed by the controller 21 of the patient terminal 20A of the user U.A, an example of a process to be executed by the controller 31 of the medical institution terminal 30X in the medical institution H.X, an example of a process to be executed by the controller 51 of the medical institution server 50, and an example of a process to be executed by the controller 11 of the management server 10 in this order from the left side.

In the present flowcharts, the case of the symmetric-key encryption scheme is presented as an example, and it is assumed that a secret key has been generated on the patient terminal 20A and stored in the data storage 28 before the processes.

Hereinafter, description will be given with reference to FIG. 55.

The controller 21 of the patient terminal 20A executes a PHR application decryption key-provided linkage code information generation process based on user input, for example (A810). In the PHR application decryption key-provided linkage code information generation process, the controller 21 of the patient terminal 20A generates PHR application decryption key-provided linkage code information as a two-dimensional code based on PHR application decryption key-provided linkage information including the PHR application linkage information and the secret key stored in the data storage 28, for example.

Then, the controller 21 of the patient terminal 20A causes the display 24 to display and output the generated PHR application decryption key-provided linkage code information, for example (A820).

The controller 31 of the medical institution terminal 30X causes, based on user input, the imaging unit 37 to read the PHR application decryption key-provided linkage code information displayed on the patient terminal 20A, for example (X810). Then, the controller 31 of the medical institution terminal 30X decodes the read PHR application decryption key-provided linkage code information so as to acquire the PHR application decryption key-provided linkage information.

The method for relaying the PHR application decryption key-provided linkage information is not limited to the method performed with such a code image. For example, the controller 21 of the patient terminal 20A may achieve the relaying by transmitting the PHR application decryption key-provided linkage information to the medical institution terminal 30X through P2P communication by utilizing short-range wireless communication according to Bluetooth, NFC, or the like, for example.

The PHR application account linkage request information does not have to include the decryption key, for example. That is, the decryption key does not have to be stored in the management server 10.

After transmitting the PHR application account linkage request information to the management server 10, the controller 31 of the medical institution terminal 30X transmits, to the medical institution server 50, decryption key-provided PHR-EMR account linkage request information including the user account ID, the PHR application ID, the corresponding EMR ID, and the decryption key, for example (X820).

When receiving the decryption key-provided PHR-EMR account linkage request information from the medical institution terminal 30X, the controller 51 of the medical institution server 50 executes a decryption key-provided PHR-EMR account linkage process (M810). In the decryption key-provided PHR-EMR account linkage process, the PHR-EMR linker 511 adds a record to the PHR-EMR association registered data 559 based on the decryption key-provided PHR-EMR account linkage request information and stores the user account ID, the EMR ID, and the decryption key in association with one another, for example.

The controller 21 of the patient terminal 20A executes the PHR data acquisition process (A130) and then executes a PHR data encryption process (A830), for example. In the PHR data encryption process, the controller 21 of the patient terminal 20A encrypts the acquired PHR data by using the secret key stored in the data storage 28 so as to generate encrypted PHR data, for example.

Then, the controller 21 of the patient terminal 20A transmits encrypted PHR data recording request information including the encrypted PHR data to the management server 10 (A840).

When receiving the encrypted PHR data recording request information from the patient terminal 20A, the controller 11 of the management server 10 executes an encrypted PHR data recording process (S820). In the encrypted PHR data recording process, the controller 11 of the management server 10 causes the encrypted PHR data to be stored in the PHR data management data in association with the corresponding PHR application ID, for example.

In the management server 10, the decryption key is not acquired, and thus the PHR data cannot be taken out from the encrypted PHR data.

When executing the PHR data searching process (S130), the controller 11 of the management server 10 transmits the encrypted PHR data to the medical institution terminal 30X, for example (S830).

In the medical institution terminal 30X, the decryption key is not stored, and thus the PHR data cannot be taken out from the encrypted PHR data, for example.

Hereinafter, description will be given with reference to FIG. 56.

When executing the EMR data searching process (M430) after receiving the EMR data reference request information from the medical institution terminal 30X, the controller 51 of the medical institution server 50 executes a PHR data decryption process, for example (M820). In the PHR data decryption process, the controller 51 of the medical institution server 50 refers to the PHR-EMR association registered data 559 and acquires the PHR data by using the PHR data decryption key to decrypt the encrypted PHR data saved in the EMR data of the corresponding piece of EMR patient information management data, for example. Then, the controller 51 of the medical institution server 50 transmits EMR data including the decrypted PHR data to the medical institution terminal 30X, for example (M440).

The controller 51 of the medical institution server 50 may, when executing the EMR data update process, execute the PHR data decryption process and save the decrypted PHR data into the EMR data of the corresponding piece of EMR patient information management data, for example.

Alternatively, the controller 31 of the medical institution terminal 30X may store, in the data storage 38, the decryption key and the user account ID in association with each other. The decryption key and the user account ID are based on the PHR application decryption key-provided linkage information. In this case, when receiving the encrypted PHR data from the management server 10, the controller 31 of the medical institution terminal 30X may execute the PHR data decryption process and acquire the PHR data. The EMR data update request information may include the decrypted PHR data.

### <Advantageous Effects of Fifth Implementation Example>

In the fifth implementation example, as shown in FIG. 54, FIG. 55, FIG. 56, and the like, the PHR data encrypted with an encryption key corresponding to the PHR application is registered to the management server 10, and the encrypted PHR data is provided to the medical institution terminal 30 that has a decryption key corresponding to the encryption key. Since the PHR data registered to the management server 10 is encrypted, a security-related risk such as a data leak is decreased.

In addition, in the fifth implementation example, as shown in FIG. 54, FIG. 55, FIG. 56, and the like, the PHR data encrypted with an encryption key corresponding to the PHR application is registered to the management server 10, the encrypted PHR data is provided to the medical institution terminal 30 that has a decryption key corresponding to the encryption key, and the decryption key is provided only to the medical institution terminal 30 with an ID linkage. Since the decryption key is provided only to the medical institution terminal 30 with the ID linkage, the PHR data can be prevented from being provided to an unintended medical institution.

### <Modification (1) of Fifth Implementation Example>

In the above implementation example, an example in which the decryption key is a secret key based on the symmetric-key encryption scheme has been presented. However, no limitation thereto is made. For example, the decryption key may be a secret key based on the public-key encryption scheme in the medical institution server 50.

In this case, it is assumed that a secret key and a public key have been generated in each of the patient terminal 20A and the medical institution server 50 and stored in each of the data storage 28 and the data storage 55 before the processes. Hereinafter, the secret key and the public key in the patient terminal 20A are respectively referred to as "secret key A" and "public key A", and the secret key and the public key in the medical institution server 50 are respectively referred to as "secret key H" and "public key H".

For example, the controller 21 of the patient terminal 20A transmits PHR application decryption key-provided linkage information including the public key A based on the secret key A to the medical institution terminal 30X by means of code information, P2P communication, or the like. Then, the controller 31 of the medical institution terminal 30X transmits PHR application account linkage request information including the public key A as a decryption key to the management server 10.

In addition, the controller 31 of the medical institution terminal 30X receives the public key H based on the secret key H from the medical institution server 50 and transmits PHR application decryption key-provided linkage information including the public key H to the patient terminal 20A by means of code information, P2P communication, or the like. Then, the controller 21 of the patient terminal 20A stores, in the data storage 28, the received public key H in association with the medical institution account ID, for example.

In the PHR data encryption process, the controller 21 of the patient terminal 20A encrypts the PHR data based on the public key A so as to generate first encrypted PHR data, for example. In addition, the controller 21 of the patient terminal 20A encrypts the PHR data based on the public key H so as to generate second encrypted PHR data, for example. The first encrypted PHR data may be considered as encrypted PHR data that can be decrypted by the user of the patient terminal 20A. Meanwhile, the second encrypted PHR data may be considered as encrypted PHR data that can be decrypted by the user of the medical institution server 50. In a case where a plurality of linkage destinations for the PHR data exist, the controller 21 of the patient terminal 20A may further generate a plurality of pieces of encrypted PHR data based on public keys H corresponding to the respective medical institution accounts, for example.

In the PHR data decryption process, when acquiring the second encrypted PHR data, the controller 51 of the medical institution server 50 decrypts the second encrypted PHR data based on the secret key H so as to acquire the PHR data, for example.

Alternatively, the secret key and the public key in the medical institution server 50 may be a secret key and a public key generated by any of the medical institution terminals 30. That is, a key exchange with the patient terminal 20 may be performed for each of the medical institution terminals 30. In this case, when receiving the second encrypted PHR data from the management server 10, the controller 31 of the medical institution terminal 30 may execute the PHR data decryption process so as to acquire the PHR data. The EMR data update request information may include the decrypted PHR data.

In addition, when receiving PHR linkage notification information after the management server 10 executes the PHR application account linkage process, the controller 21 of the patient terminal 20A may transmit PHR data reference request information for requesting first encrypted PHR data that has already been recorded in an application linked with the management server 10, for example. Then, when using the secret key A to decrypt the first encrypted PHR data so as to acquire the PHR data, the controller 21 of the patient terminal 20A may encrypt the PHR data by using the public key H in a newly linked medical institution terminal 30 or the medical institution server 50 and cause the encrypted PHR data to be additionally stored in the management server 10.

### [Remarks]

The present disclosure includes following items 1-27.

Item 1: A data sharing system configured to share health data of a patient between (i) a plurality of types of applications configured to acquire the health data and (ii) a terminal of a medical institution, the data sharing system comprising:
a data storage configured to store, in association with at least one of the applications, a linkage between a first account ID which allows the patient to access the data sharing system and a second account ID which allows a user of the terminal of the medical institution to access the data sharing system; and
a controller programmed to identify, in response to a request from the terminal of the medical institution, the health data acquired by the at least one application associated with the linkage between the first account ID and the second account ID.

Item 2: The data sharing system according to item 1,
wherein the controller is programmed to register, in the data storage, the health data corresponding to each of the plurality of types of applications.

Item 3: The data sharing system according to item 1,
wherein the controller is programmed to request, for identification of the health data, the health data corresponding to each of the plurality of types of applications from a terminal of the patient.

Item 4: The data sharing system according to item 1,
wherein the controller is programmed to request, for identification of the health data, the health data corresponding to each of the plurality of types of applications from one or more corresponding application management servers.

Item 5: The data sharing system according to item 1,
wherein the data storage is configured to manage an account corresponding to the patient based on the first account ID shared by the plurality of types of applications operating on a terminal of the patient.

Item 6: The data sharing system according to item 1,
wherein the data storage is configured to manage the plurality of types of applications in association with the linkage between the first account ID and the second account ID.

Item 7: The data sharing system according to item 1,
wherein the data storage is configured to manage each of the plurality of types of applications corresponding to the first account ID in association with a plurality of the second account IDs.

Item 8: The data sharing system according to item 1,
wherein the controller is further programmed to receive an ID linkage request which includes (i) the first account ID, (ii) the second account ID, and (iii) an application ID of the at least one application, and
wherein the data storage is configured to manage the linkage between the first account ID and the second account ID in association with the at least one application in response to the ID linkage request.

Item 9: The data sharing system according to item 1,
wherein the request from the terminal of the medical institution includes information that enables identification of the first account ID and the second account ID, and
wherein the controller is programmed to identify the health data acquired by the application associated with the first account ID and the second account ID identified by the information.

Item 10: The data sharing system according to item 1,
wherein the request from the terminal includes specification of the health data by personal information about the patient, and
wherein the controller is programmed to identify the health data corresponding to the request from the terminal based on:
   (i) the first account ID corresponding to the personal information;
   (ii) the second account ID corresponding to the terminal; and
   (iii) the application associated with the first account ID and the second account ID.

Item 11: The data sharing system according to item 1,
wherein the request from the terminal includes information that enables identification of the first account ID and the second account ID, and
wherein the controller is programmed to identify the application associated with the first account ID and the second account ID identified by the information, and to identify the health data acquired by the identified application.

Item 12: The data sharing system according to item 1,
wherein the controller is further programmed to receive an ID linkage request which includes information of (i) the first account ID and an application ID of the at least one application, and (ii) the second account ID,
wherein the information (i) is provided to the terminal of the medical institution from a terminal with the application installed, and
wherein the data storage is configured to manage the linkage between the first account ID and the second account ID in association with the application ID.

Item 13: The data sharing system according to item 1,
wherein the controller is further programmed to receive an ID linkage request which includes information of (i) the first account ID and an application ID of the at least one application, and (ii) the second account ID,
wherein the information (i) is provided to the terminal of the medical institution, based on a consent of the patient or a related person of the patient, from a terminal with the application installed, and
wherein the data storage is configured to manage the linkage between the first account ID and the second account ID in association with the application ID.

Item 14: The data sharing system according to item 1,
wherein the controller is further programmed to receive an ID linkage request which includes information of (i) the second account ID provided from the terminal of the medical institution to a terminal with the application installed, and (ii) the first account ID and an application ID of the application, and
wherein the data storage is configured to manage the linkage between the first account ID and the second account ID in association with the application ID.

Item 15: The data sharing system according to item 1,
wherein the controller is further programmed to receive, in response to a consent of the patient or a related person of the patient, an ID linkage request which includes information of (i) the second account ID provided from the terminal of the medical institution to a terminal with the application installed, and (ii) the first account ID and an application ID of the application, and
wherein the data storage is configured to manage the linkage between the first account ID and the second account ID in association with at least one of application IDs.

Item 16: The data sharing system according to item 1,
wherein the terminal of the medical institution runs a medical institution application to view the health data, and
wherein the medical institution application has compatibility with the plurality of types of applications.

Item 17: The data sharing system according to item 1,
wherein the data storage is configured to manage a third account ID in association with the first account ID, and
wherein the third account ID is assigned to the patient in an electronic medical record system that provides electronic medical record information.

Item 18: The data sharing system according to item 1,
wherein the data storage is configured to manage a third account ID in association with the first account ID,
wherein the third account ID is assigned to the patient in an electronic medical record system that provides electronic medical record information,
wherein the controller is programmed to register the health data and/or information related to the health data to the electronic medical record system in association with the third account ID, and
wherein the health data and/or the information related to the health data corresponds to the first account ID.

Item 19: The data sharing system according to item 1,
wherein the data storage is configured to manage a third account ID in association with the first account ID,
wherein the third account ID is assigned to the patient in an electronic medical record system that provides electronic medical record information,
wherein the controller is programmed to register, in association with the third account ID, the health data and/or information related to the health data corresponding to the first account ID, and
wherein the controller is programmed to provide, in response to a request for providing the electronic medical record information corresponding to the third account ID, (i) the electronic medical record information and (ii) the health data and/or the information related to the health data registered in association with the third account ID to the terminal of the medical institution.

Item 20: The data sharing system according to item 1,
wherein the controller is programmed to create the first account ID in response to a terminal of the patient obtaining the second account ID from the terminal of the medical institution, and to request to install the application associated with the second account ID on the terminal of the patient.

Item 21: The data sharing system according to item 20,
wherein the data storage is configured to manage, based on a consent of the patient or a related person of the patient, the first account ID corresponding to the patient in association with a third account ID, and
wherein the third account ID is assigned to the patient in an electronic medical record system that provides electronic medical record information.

Item 22: The data sharing system according to item 1,
wherein the data storage is configured to cancel the linkage between the first account ID and the second account ID in response to an operation of a terminal of the patient.

Item 23: The data sharing system according to item 1,
wherein the data storage is configured to cancel the linkage between the first account ID and the second account ID according to a predetermined condition.

Item 24: The data sharing system according to item 22,
wherein the data storage is configured to delete, in response to an operation by the user of the medical terminal, the health data acquired by the application and managed in association with a third account ID which is assigned to the patient in an electronic medical record system that provides electronic medical record information.

Item 25: The data sharing system according to item 1,
wherein the health data is encrypted with an encryption key corresponding to the application, and
wherein the controller is programmed to provide the encrypted data to the terminal of the medical institution that has a decryption key corresponding to the encryption key.

Item 26: The data sharing system according to item 25,
wherein the controller is programmed to provide the decryption key only to the terminal of the medical institution with an ID linkage.

Item 27: A method for sharing data in a data sharing system configured to share health data of a patient between (i) a plurality of types of applications configured to acquire the health data and (ii) a terminal of a medical institution, the data sharing method comprising:
managing, in association with at least one of the applications, a linkage between a first account ID which allows the patient to access the data sharing system and a second account ID which allows a user of the terminal of the medical institution to access the data sharing system; and
identifying, in response to a request from the terminal of the medical institution, the health data acquired by the at least one application associated with the linkage between the first account ID and the second account ID.

### DESCRIPTION OF THE REFERENCE CHARACTERS

- 1: information system
- 10: management server
- 20: patient terminal
- 30: medical institution terminal
- 40: network
- 50: medical institution server
- 60: PHR application management server

## Claims

1. A data sharing system configured to share health data of a patient between (i) a plurality of types of applications configured to acquire the health data and (ii) a terminal of a medical institution, the data sharing system comprising:
a data storage configured to store, in association with at least one of the applications, a linkage between a first account ID which allows the patient to access the data sharing system and a second account ID which allows a user of the terminal of the medical institution to access the data sharing system; and
a controller programmed to identify, in response to a request from the terminal of the medical institution, the health data acquired by the at least one application associated with the linkage between the first account ID and the second account ID.

2. The data sharing system of claim 1,
wherein the controller is programmed to register, in the data storage, the health data corresponding to each of the plurality of types of applications.

3. The data sharing system of any one of claims 1 to 2,
wherein the controller is programmed to request, for identification of the health data, the health data corresponding to each of the plurality of types of applications from a terminal of the patient.

4. The data sharing system of any one of claims 1 to 3
wherein the controller is programmed to request, for identification of the health data, the health data corresponding to each of the plurality of types of applications from one or more corresponding application management servers.

5. The data sharing system of any one of claims 1 to 4,
wherein the data storage is configured to manage an account corresponding to the patient based on the first account ID shared by the plurality of types of applications operating on a terminal of the patient.

6. The data sharing system of any one of claims 1 to 5,
wherein the data storage is configured to manage the plurality of types of applications in association with the linkage between the first account ID and the second account ID.

7. The data sharing system of any one of claims 1 to 6,
wherein the data storage is configured to manage each of the plurality of types of applications corresponding to the first account ID in association with a plurality of the second account IDs.

8. The data sharing system of any one of claims 1 to 7,
wherein the controller is further programmed to receive an ID linkage request which includes (i) the first account ID, (ii) the second account ID, and (iii) an application ID of the at least one application, and
wherein the data storage is configured to manage the linkage between the first account ID and the second account ID in association with the at least one application in response to the ID linkage request.

9. The data sharing system of any one of claims 1 to 8
wherein the request from the terminal of the medical institution includes information that enables identification of the first account ID and the second account ID, and
wherein the controller is programmed to identify the health data acquired by the application associated with the first account ID and the second account ID identified by the information.

10. The data sharing system of any one of claims 1 to 9,
wherein the request from the terminal includes specification of the health data by personal information about the patient, and
wherein the controller is programmed to identify the health data corresponding to the request from the terminal based on:
(i) the first account ID corresponding to the personal information;
(ii) the second account ID corresponding to the terminal; and
(iii) the application associated with the first account ID and the second account ID.

11. The data sharing system of any one of claims 1 to 10,
wherein the request from the terminal includes information that enables identification of the first account ID and the second account ID, and
wherein the controller is programmed to identify the application associated with the first account ID and the second account ID identified by the information, and to identify the health data acquired by the identified application.

12. The data sharing system of any one of claims 1 to 11,
wherein the controller is further programmed to receive an ID linkage request which includes information of (i) the first account ID and an application ID of the at least one application, and (ii) the second account ID,
wherein the information (i) is provided to the terminal of the medical institution from a terminal with the application installed, and
wherein the data storage is configured to manage the linkage between the first account ID and the second account ID in association with the application ID.

13. The data sharing system of any one of claims 1 to 12,
wherein the controller is further programmed to receive an ID linkage request which includes information of (i) the first account ID and an application ID of the at least one application, and (ii) the second account ID,
wherein the information (i) is provided to the terminal of the medical institution, based on a consent of the patient or a related person of the patient, from a terminal with the application installed, and
wherein the data storage is configured to manage the linkage between the first account ID and the second account ID in association with the application ID.

14. The data sharing system of any one of claims 1 to 13,
wherein the controller is further programmed to receive an ID linkage request which includes information of (i) the second account ID provided from the terminal of the medical institution to a terminal with the application installed, and (ii) the first account ID and an application ID of the application, and
wherein the data storage is configured to manage the linkage between the first account ID and the second account ID in association with the application ID.

15. The data sharing system of any one of claims 1 to 14,
wherein the data storage is configured to manage a third account ID in association with the first account ID, and
wherein the third account ID is assigned to the patient in an electronic medical record system that provides electronic medical record information.

16. The data sharing system of any one of claims 1 to 15,
wherein the health data is encrypted with an encryption key corresponding to the application, and
wherein the controller is programmed to provide the encrypted data to the terminal of the medical institution that has a decryption key corresponding to the encryption key.

17. The data sharing system of any one of claims 1 to 16,
wherein the controller is programmed to provide the decryption key only to the terminal of the medical institution with an ID linkage.

18. A method for sharing data in a data sharing system configured to share health data of a patient between (i) a plurality of types of applications configured to acquire the health data and (ii) a terminal of a medical institution, the data sharing method comprising:
managing, in association with at least one of the applications, a linkage between a first account ID which allows the patient to access the data sharing system and a second account ID which allows a user of the terminal of the medical institution to access the data sharing system; and
identifying, in response to a request from the terminal of the medical institution, the health data acquired by the at least one application associated with the linkage between the first account ID and the second account ID.
